# EUROPEAN PATENT APPLICATION

(11) **EP 4 796 125 A1**
(43) Date of publication of application: **26.08.2026**
(21) Application number: 24879124.6
(22) Date of filing: 18.10.2024
(51) Int. Cl.: A61K 45/00, A61P 35/00

(54) **TREATMENT OF PATIENT WITH NEGATIVE P53 GENE MUTATION, POSITIVE KRAS GENE MUTATION AND POSITIVE BRCA MUTATION**

(30) Priority: 18.10.2023 CN 202311350617; 03.04.2024 CN 202410407200
(71) Applicant: Ascentawits Pharmaceuticals, Ltd., Shenzhen, Guangdong 518122 (CN)
(72) Inventor: DUAN, Jianxin, Shenzhen, Guangdong 518000 (CN); QI, Tianyang, Shenzhen, Guangdong 518000 (CN); MENG, Fanying, San Francisco, California California 94121 (US); LIU, Xing, Shenzhen, Guangdong 518000 (CN); WANG, Yizhi, Shenzhen, Guangdong 518000 (CN); LI, Bing, Shenzhen, Guangdong 518000 (CN); WANG, Cheng, Shenzhen, Guangdong 518000 (CN)
(74) Representative: Haseltine Lake Kempner LLP
(86) International application number: PCT/CN2024/125790
(87) International publication number: WO 2025/082484

(57) **Abstract**

A treatment method, wherein a drug which contains an AKR1 C3 enzyme-activated or hypoxia-activated anti-cancer prodrug compound, or a salt, ester, solvate or isotopic isomer thereof, is used alone or in combination with other drugs to treat a cancer patient with a negative P53 gene mutation and a positive KRAS gene mutation, or to treat a cancer patient with a negative P53 gene mutation and a positive BRCA gene mutation, or to treat a cancer patient with a positive KRAS gene mutation and a positive BRCA gene mutation, or to treat a cancer patient with a negative P53 gene mutation, a positive KRAS gene mutation and a positive BRCA gene mutation.

## Description

### TECHNICAL FIELD

The present invention relates to a method of treating tumor or cancer, and in particular to treatment of cancer or tumor patients with particular gene profiles using an anti-cancer prodrug compound activated by AKR1C3 enzyme/hypoxia, belonging to the field of tumor therapy.

### BACKGROUND

The DNA alkylating agent prodrug AST-3424 (WO2016145092, WO2017087428) targeting overexpressed Aldo-Keto reductase 1C3 (AKR1C3) has CAS No. 2097713-69-2 and the following structure:

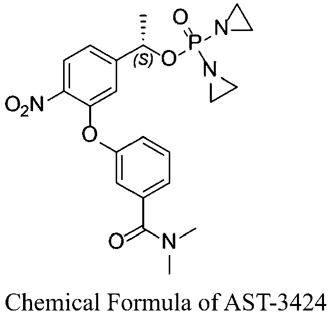

AST-3424 (also known as OBI-3424, TH-3424) enters cancer cells and is activated by AKR1C3 enzyme overexpressed by the cancer cell to release the metabolite AST-2660. AST-3424 itself has low toxicity to cancer cells and its pharmacological effects are correlated with the expression of AKR1C3 enzyme in animal models and *in vitro* pharmacological experiments: the prodrug AST-3424 is metabolized into AST-2660 under the action of AKR1C3 enzyme and NADPH, and the expression level of the enzyme is positively correlated with the drug efficacy (Meng F, Li WF, Jung D, et al. A novel selective AKR1C3-activated prodrug AST-3424/OBI-3424 exhibits broad anti-tumor activity. Am J Cancer Res. 2021;11(7):3645-3659; Evans K, Duan J, Pritchard T, et al. OBI-3424, a Novel AKR1C3-Activated Prodrug, Exhibits Potent Efficacy against Preclinical Models of T-ALL. Clin Cancer Res. 2019;25(14):4493-4503. doi:10.1158/1078-0432.CCR-19-0551; Wang Y, Liu Y, Zhou C, et al. An AKR1C3-specific prodrug with potent anti-tumor activities against T-ALL. Leuk Lymphoma. 2020;61(7):1660-1668. doi:10.1080/10428194.2020.1728746; He P, Wang C, Wang Y, et al. A Novel AKR1C3 Specific Prodrug TH3424 With Potent Antitumor Activity in Liver Cancer [retracted in: Clin Pharmacol Ther. 2021 Jul;110(1):262]. Clin Pharmacol Ther. 2021, 110(1):229-237. doi:10.1002/cpt.2171). 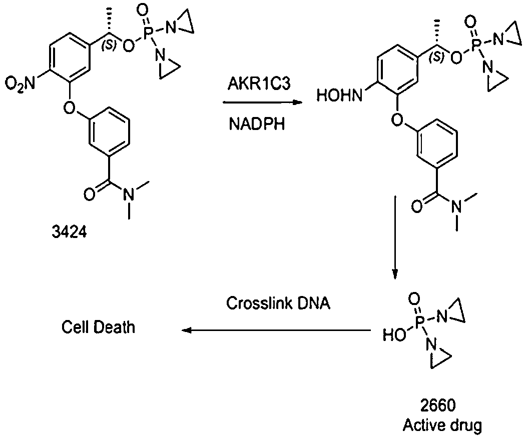 Chemical reaction equation of metabolizing AST-3424 into AST-2660 (2660 shown in the figure)

At present, the drug has entered Phase II clinical trials in China and the United States, respectively (NCT03592264 in the U.S. for castration-resistant prostate cancer and liver cancer; NCT04315324 in the U.S. for T-cell acute lymphoblastic leukemia (T-ALL); CTR20191399 in China for various solid tumors; and CTR20201915 for T-cell acute lymphocytic leukemia and B-cell acute lymphocytic leukemia).

### SUMMARY OF THE INVENTION

In the phase II clinical trial of AST-3424 in China, the applicant noted that the therapeutic effect on cancer and tumor patients negative for P53 gene mutation or deficiency is better than that of patients positive for P53 gene mutation or deficiency. Therefore, the applicant supposed that AST-3424 would have a better therapeutic effect in treating tumor or cancer patients with negative detection results for P53 gene mutation or deficiency; that is, tumor or cancer patients with negative detection results for P53 gene mutation or deficiency would have more obvious clinical benefits when receiving AST-3424 treatment.

Moreover, in further preclinical efficacy studies, the applicant noted that the above AKR1C3 enzyme-activated anti-cancer prodrug compounds with similar structure to AST-3424 and the hypoxia-activated anti-cancer prodrug compounds with similar structure to TH-302 have excellent therapeutic effect on BRCA-mutated tumors; that is, comparatively speaking, the above AKR1C3 enzyme-activated DNA alkylating agent prodrug compounds with similar structure to AST-3424 and hypoxia-activated DNA alkylating agent prodrug compounds with similar structure to TH-302 have better therapeutic effect on tumor models carrying pathogenic BRCA mutations as compared with wild-type tumor models (not having BRCA mutations or having non-pathogenic BRCA mutations), and the difference is very significant (please refer to PCT/CN2023/081542 with publication number WO2023174319A1).

Particularly, in the Phase II clinical trial of AST-3424 conducted in China, the patients having BRCA mutations showed certain advantages in therapeutic effects as compared with those without the mutations.

Particularly, in the preclinical studies, the applicant noted that the AKR1C3 enzyme-activated anti-cancer prodrug compounds AST-3424 and AST had significant inhibitory effects on PDX tumor models or cells with Kras-G12D/G12C mutations (please refer to Examples 1-9 of the application PCT/CN2022/120817 with publication number WO2023/046060).

Therefore, the above AKR1C3 enzyme-activated anti-cancer prodrug compounds with similar structure or similar mechanism to AST-3424 and the hypoxia-activated anti-cancer prodrug compounds with similar structure to TH-302 may have better therapeutic effects on patients negative for p53 gene mutation and positive for both BRCA mutation and KRAS mutation.

Therefore, the applicant proposes the following methods for treating cancers:
A treatment method, comprising using a drug containing a DNA alkylating agent prodrug compound, or a salt, ester, solvate, or isotopic isomer thereof, alone or in combination, for treating a cancer or tumor patient, wherein the cancer or tumor patient is selected from the group consisting of the patients that meet one of the following conditions:
cancer patients negative for P53 gene mutation and positive for KRAS gene mutation;
cancer patients negative for P53 gene mutation and positive for BRCA gene mutation;
cancer patients positive for both KRAS gene mutation and BRCA gene mutation; and
cancer patients that meet all three conditions of being negative for P53 gene mutation and positive for both KRAS gene mutation and BRCA gene mutation.

A treatment method, comprising using a drug containing a DNA alkylating agent prodrug compound, or a salt, ester, solvate, or isotopic isomer thereof, alone or in combination, for treating a cancer or tumor patient, wherein the DNA alkylating agent prodrug compound is activated by AKR1C3 enzyme, β-D-Glucosidase, Carboxylesterase, Esterase and Caspase-3, Cathepsin B, γ-Glutamyltranspeptidase, β-galactosidase, or hypoxia, and wherein the cancer or tumor patient is selected from the group consisting of the patients that meet one of the following conditions:
cancer patients negative for P53 gene mutation and positive for KRAS gene mutation;
cancer patients negative for P53 gene mutation and positive for BRCA gene mutation;
cancer patients positive for both KRAS gene mutation and BRCA gene mutation; and
cancer patients that meet all three conditions of being negative for P53 gene mutation and positive for both KRAS gene mutation and BRCA gene mutation.

A treatment method, comprising using a drug containing a DNA alkylating agent prodrug compound, or a salt, ester, solvate or isotopic isomer thereof, alone or in combination with other drugs, for treating a cancer patient negative for P53 gene mutation and positive for BRCA gene mutation, wherein the DNA alkylating agent prodrug compound is selected from the group consisting of the DNA alkylating agent prodrug compounds that are activated by AKR1C3 enzyme, β-D-Glucosidase, Carboxylesterase, Esterase and Caspase-3, Cathepsin B, γ-Glutamyltranspeptidase, β-galactosidase, or hypoxia.

A treatment method, comprising using a drug containing an AKR1C3 enzyme-activated anti-cancer prodrug compound, or a salt, ester, solvate, or isotopic isomer thereof, alone or in combination with other drugs, for treating a cancer patient negative for P53 gene mutation and positive for KRAS gene mutation.

A treatment method, comprising using a drug containing an AKR1C3 enzyme-activated anti-cancer prodrug compound, or a salt, ester, solvate, or isotopic isomer thereof, alone or in combination with other drugs, for treating a cancer patient positive for both KRAS gene mutation and BRCA gene mutation.

The DNA alkylating agent prodrug compound refers to a prodrug compound that can be metabolized and converted into a DNA alkylating agent which eventually alkylates DNA in cancer cells, thereby destroying the DNA structure of cancer cells and finally leading to cell death.

Most prodrug compounds can be metabolized and converted under physiological environments. For anti-tumor and anti-cancer drugs, these physiological environments typically refer to the unique microenvironments of tumor tissues or cancer cells, such as, high expression of certain transport proteins on cell membranes, high specific expression or high concentration due to enrichment of certain enzymes or proteins in intracellular or extracellular environments as compared with normal cells, or hypoxia or abnormal pH, etc. Therefore, the existence of these microenvironments is generally considered to be caused by some specific mechanisms of tumor tissues or cancer cells.

Targeting of tumor/cancer cells with the above microenvironments can be achieved by activating prodrugs to metabolize and convert into active drugs through these microenvironments. Currently, known targets of these microenvironments reported in the literatures include various enzyme activations, such as AKR1C3 enzyme activation, β-D-Glucosidase activation, Carboxylesterase activation, Esterase and Caspase-3 activation, Cathepsin B activation, γ-Glutamyltranspeptidase activation, β-galactosidase activation, or hypoxia activation.

The AKR1C3 enzyme-activated anti-cancer prodrug compound means that the compound is a prodrug that reacts with AKR1C3 enzyme to release a toxic anti-tumor compound. For detailed definitions and meanings, please refer to the application PCT/CN2021/114774 with publication number WO2022048492A1.

The AKR1C3 enzyme-activated anti-cancer prodrug compounds comprise AKR1C3 enzyme-activated DNA alkylating agent prodrug compounds and AKR1C3 enzyme-activated, non-DNA alkylating agent prodrug compounds;
the AKR1C3 enzyme-activated DNA alkylating agent prodrug compound is selected from the group consisting of the compounds of Formulae (4)-(11) and (15); and the AKR1C3 enzyme-activated, non-DNA alkylating agent prodrug compound is selected from the group consisting of the compounds of Formulae (12)-(14).

The DNA alkylating agent prodrug compound is selected from the group consisting of the DNA alkylating agent prodrug compounds that are activated by AKR1C3 enzyme, β-D-Glucosidase, Carboxylesterase, Esterase and caspase-3, Cathepsin B, γ-Glutamyltranspeptidase, β-galactosidase, or hypoxia, and preferably activated by AKR1C3 enzyme, β-D-Glucosidase, or hypoxia.

The hypoxia-activated DNA alkylating agent prodrug compound is selected from the group consisting of the compounds of Formulae (1)-(3); the AKR1C3-activated DNA alkylating agent prodrug compound is selected from the group consisting of the compounds of Formulae (4)-(11); and the β-D-Glucosidase-activated or β-galactosidase-activated DNA alkylating agent prodrug compound is selected from the group consisting of the compound of Formula (15). 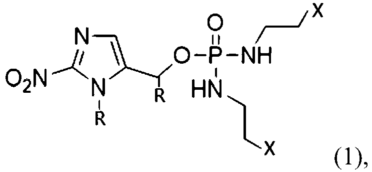 wherein R is each independently selected from the group consisting of H, -CH₃, - CH₂CH₃, and -CF₃, and X is each independently selected from the group consisting of leaving groups Cl, Br, MsO, TsO, and the like.

Formulations related to TH-302 or its analogous compounds include oral preparations, freeze-dried preparations and concentrated injection solutions, and the related formulations, preparation methods, clinical combinations, and administration methods are described and disclosed in detail in the related patents of Threshold Pharmaceuticals, Inc.: WO2010048330A1, WO2012142520A2, WO2008083101A1, WO2007002931A3, which are incorporated herein by reference in their entirety.

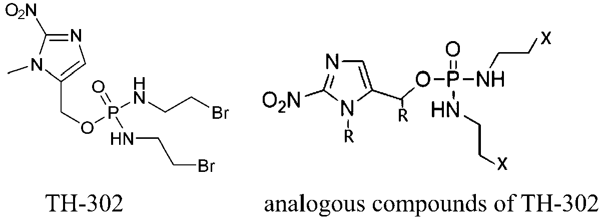

TH-302 or its analogous compound is a DNA alkylating agent anticancer drug with broad cancer therapeutic potential and the related cancer indication experiments and clinical trials are disclosed in the relevant patent applications of Threshold Pharmaceuticals, Inc. and other pharmaceutical companies (such as WO2016011195A2, WO2004087075A1, WO2007002931A1, WO2008151253A2, WO2009018163A1, WO2009033165A2, WO2010048330A2, WO2012142520A1, WO2008083101A2, WO2020007106A1, WO2020118251A1, WO2014169035A1, WO2013116385A1, WO2019173799A2, WO2016081547A1, WO2014062856A1, WO2015069489A1, WO2012006032A2, WO2018026606A2, WO2010048330A2, WO2015171647A1, WO2013096687A1, WO2013126539A2, WO2013096684A2, WO2012009288A2, WO2012145684A2, WO2016014390A2, WO2019055786A2, WO2012135757A2, WO2015013448A2, WO2016011328A2, WO2013177633A2, WO2016011195A2, WO2015051921A2) and in the clinical trials registered with FDA (NCT02402062, NCT02020226, NCT02076230, NCT01381822, NCT02093962, NCT01440088, NCT02255110, NCT02342379, NCT01864538, NCT01149915, NCT02433639, NCT00743379, NCT01485042, NCT01721941, NCT02047500, NCT00742963, NCT01497444, NCT00495144, NCT01746979, NCT01144455, NCT01403610, NCT01522872, NCT01833546, NCT02598687, NCT03098160, NCT02496832, NCT02712567). The above patent applications and clinical trial information are incorporated herein by reference in their entirety. 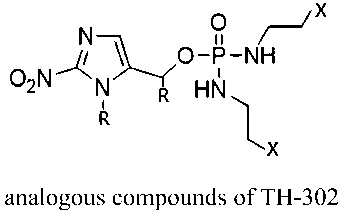 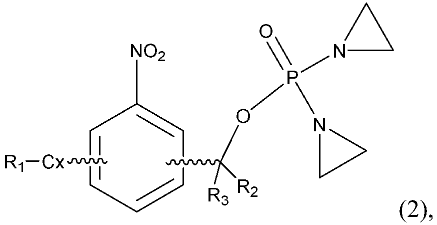 wherein the definitions of R₁, R₂, R₃, and Cx are as described in the claims of the patent application PCT/CN2020/114519 with publication number WO2021120717A1 (corresponding to the Chinese patent application No. 2020800673113 with publication number CN114466853A), and the synthesis and preparation methods of the specific compounds are also described in the above application, which is incorporated herein by reference in its entirety. The specific definitions are as follows:
Cx represents a 5- to 10-membered aromatic ring, an aromatic heterocycle, an aliphatic heterocyclic ring, or a cycloalkane group which shares two carbon atoms with the nitrobenzene ring to form a fused ring structure;
R₁ is linked to any ring atom of the Cx ring and is selected from the group consisting of hydrogen, a halogen atom, cyano or isocyano, hydroxyl, sulfhydryl, amino, OTs, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or
Z-substituted aryl, 4- to 15-membered heterocyclyl or Z-substituted heterocyclyl, 5- to 15-membered heteroaryl or Z-substituted heteroaryl, alkoxy of 1-6 carbon atoms or Z-substituted alkoxy of 1-6 carbon atoms, -CONR⁶R⁷, -SO₂NR⁶R⁷, -SO₂R⁶, -OCOO-R⁶, -COOR⁶, -NR⁶COR⁷, -OCOR⁶, -NR⁶SO₂R⁷, and -NR⁶SO₂NR⁶R⁷;
R₂ and R₃ are each independently hydrogen, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4- to 15-membered heterocyclyl or Z-substituted heterocyclyl, 5- to 15-membered heteroaryl or
Z-substituted heteroaryl, or R₂ and R₃ together with the benzylic carbon atom to which they are bonded form a 3- to 6-membered ring;
the group 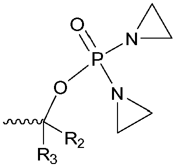 may substitute a hydrogen atom at any position on the carbon atom of the fused ring, and the number of substitution is 1;
the Z substituent is halogen, cyano or isocyano, hydroxyl, sulfhydryl, amino, C₁-C₃ alkyl or substituted alkyl, C₁-C₃ alkoxy or substituted alkoxy, C₂-C₃ alkenyl or substituted alkenyl group, C₂-C₃ alkynyl or substituted alkynyl group, C₃-C₈ cycloalkyl or substituted cycloalkyl group;
R⁶ and R⁷ are each independently hydrogen, C₁-C₆ alkyl or Z-substituted C₁-C₆ alkyl, C₂-C₆ alkenyl or Z-substituted C₂-C₆ alkenyl, C₂-C₆ alkynyl or Z-substituted C₂-C₆ alkynyl, C₃-C₈ cycloalkyl or Z-substituted C₃-C₈ cycloalkyl, C₆-C₁₀ aryl or Z-substituted C₆-C₁₀ aryl, 4- to 15-membered heterocyclyl or Z-substituted 4- to 15-membered heterocyclyl, 5- to 15-membered heteroaryl or Z-substituted 5- to 15-membered heteroaryl, or R⁶ and R⁷ together with the atom to which they are bonded form a 5- to 7-membered heterocyclyl or a Z-substituted 5- to 7-membered heterocyclyl. 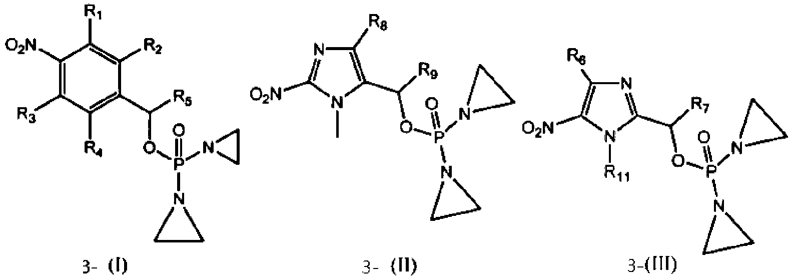 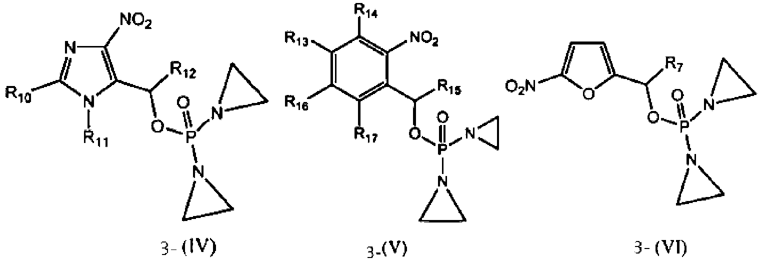
wherein the definitions of R₁, R₂, R₃, R₄, R₅, R₆, R₇, R₈, R₉, R₁₀, R₁₁, R₁₂, R₁₃, R₁₄, R₁₅, R₁₆, and R₁₇ are as described in the claims of the patent application PCT/US2016/039092 with publication number WO2016210175A1 (corresponding to the Chinese patent application No. 2016800368985 with publication number CN108024974A), and the synthesis and preparation methods of the specific compounds are also described in the above application, which is incorporated herein by reference in its entirety. The specific definitions are as follows:
   R₁ is hydrogen, -N₃, CN, halogeno, NR²¹R²², -OR²³, -SO₂ (C₁-C₆ alkyl), C₁-C₆ alkyl, C₂-C₆ alkenyl, C₂-C₆ alkynyl, C₃-C₈ cycloalkyl, C₆-C₁₀ aryl, 4- to 15-membered heterocyclyl, 5- to 15-membered heteroaryl, or ether;
   R²¹ and R²² are each independently hydrogen, hydroxyl, C₁-C₆ alkyl, C₂-C₆ alkenyl, C₂-C₆ alkynyl, C₃-C₈ cycloalkyl, C₆-C₁₀ aryl, 4- to 15-membered heterocyclyl, 5- to 15-membered heteroaryl, or -SO₂ (C₁-C₆ alkyl); or R²¹ and R²² together with the nitrogen atom to which they are bonded form 4- to 15-membered heterocyclyl or 5- to 15-membered heteroaryl;
   R²³ is hydrogen, C₁-C₆ alkyl or C₆-C₁₀ aryl;
   R₂ and R₃ are independently hydrogen or halogeno;
   R₄ is hydrogen, halogeno, C₁-C₆ alkoxy, C₁-C₆ alkyl or C₆-C₁₀ aryl,
   R₅, R₇, R₉, R₁₂, and R₁₅ are independently hydrogen, C₁-C₆ alkyl, C₂-C₆ alkenyl, C₂-C₆ alkynyl, C₃-C₈ cycloalkyl, C₆-C₁₀ aryl, 4- to 15-membered heterocyclyl, 5- to 15-membered heteroaryl; or R₄ and R₅ together with the intervening carbon atom therebetween form a C₅-C₆ cycloalkyl ring;
   R₆ and R₁₀ are independently hydrogen or halogeno;
   R₈ is hydrogen, C₁-C₆ alkyl, C₂-C₆ alkenyl, C₂-C₆ alkynyl, or 5- to 15-membered heteroaryl;
   R₁₁ is each independently C₁-C₆ alkyl, C₂-C₆ alkenyl, C₂-C₆ alkynyl, C₃-C₈ cycloalkyl, or C₆-C₁₀ aryl;
   R₁₃, R₁₄, R₁₆, and R₁₇ are independently hydrogen, halogeno, C₁-C₆ alkyl, C₂-C₆ alkenyl, C₂-C₆ alkynyl, or C₁-C₆ alkoxy;
   wherein the alkyl, alkenyl, alkynyl, cycloalkyl, aryl, heterocyclyl, heteroaryl, alkoxy, and ether are optionally substituted. 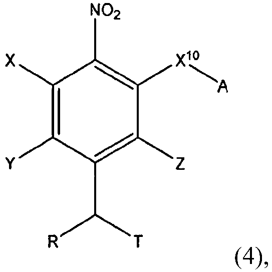
   wherein the definitions of X, Y, Z, R, T, A, and X¹⁰ are as described in the claims of the patent application PCT/US2016/021581 with publication number WO2016145092A1 (corresponding to the Chinese patent application No. 2016800150788 with publication number CN107530556A), and the synthesis and preparation methods of the specific compounds are also described in the above application, which is incorporated herein by reference in its entirety. The specific definitions are as follows:
      X¹⁰ is O, S, SO, or SO₂;
      A is C₆-C₁₀ aryl, 5- to 15-membered heteroaryl or -N=CR¹R²;
      R¹ and R² are each independently hydrogen, C₁-C₆ alkyl, C₃-C₈ cycloalkyl, C₆-C₁₀ aryl, 4- to 15-membered heterocyclyl, ether group, -CONR¹³R¹⁴, or -NR¹³COR¹⁴;
      X, Y and Z are each independently hydrogen, CN, halogeno, C₁-C₆ alkyl, C₂-C₆ alkenyl, C₂-C₆ alkynyl, C₃-C₈ cycloalkyl, C₆-C₁₀ aryl, 4- to 15-membered heterocyclyl, ether group, -CONR¹³R¹⁴, or -NR¹³COR¹⁴;
      R is hydrogen, C₁-C₆ alkyl, C₂-C₆ alkenyl, C₂-C₆ alkynyl, C₃-C₈ cycloalkyl, C₆-C₁₀ aryl, 4- to 15-membered heterocyclyl, ether group, -CONR¹³R¹⁴, or -NR¹³COR¹⁴;
      R¹³ and R¹⁴ are each independently hydrogen, C₁-C₆ alkyl, C₃-C₈ cycloalkyl, C₆-C₁₀ aryl, 4- to 15-membered heterocyclyl, or ether group;
      T comprises a phosphoramidate alkylating agent comprising one or more Z⁵-X⁵-Y⁵ moieties bonded to an -O-P(Z¹) moiety, wherein Z⁵ is a heteroatom comprising nitrogen, sulfur or oxygen, X⁵ is substituted or unsubstituted ethylene, Y⁵ is halogeno or another leaving group, or Z⁵-X⁵-Y⁵ together form an aziridinyl (NCH₂CH₂) moiety, and Z¹ is O or S; and
      wherein the alkyl, alkenyl, alkynyl, cycloalkyl, aryl, heterocyclyl, heteroaryl, and ether are substituted or unsubstituted. 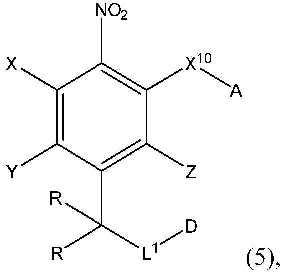
      wherein the definitions of X, Y, Z, R, D, L¹, A and X¹⁰ are as described in the claims of the patent application PCT/US2016/025665 with publication number WO2016161342A3 (corresponding to the Chinese patent application No. 2016800200132 with publication number CN108136214A), and the synthesis and preparation methods of the specific compounds are also described in the above application, which is incorporated herein by reference in its entirety. The specific definitions are as follows:
         X¹⁰ is O, S, SO, or SO₂;
         A is C₆-C₁₀ aryl, 5- to 15-membered heteroaryl, or-N=CR¹R²;
         R¹ and R² are each independently hydrogen, C₁-C₆ alkyl, C₃-C₈ cycloalkyl, C₆-C₁₀ aryl, 4- to 15-membered heterocyclyl, 5- to 15-membered heteroaryl, ether, -CONR¹³R¹⁴, or -NR¹³COR¹⁴;
         X, Y and Z are each independently hydrogen, CN, halogeno, C₁-C₆ alkyl, C₂-C₆ alkenyl, C₂-C₆ alkynyl, C₃-C₈ cycloalkyl, C₆-C₁₀ aryl, 4- to 15-membered heterocyclyl, 5 to 15 membered heteroaryl, ether group, -CONR¹³R¹⁴, or -NR¹³COR¹⁴;
         each R is independently hydrogen, C₁-C₆ alkyl, C₂-C₆ alkenyl, C₂-C₆ alkynyl, C₃-C₈ cycloalkyl, C₆-C₁₀ aryl, 4- to 15-membered heterocyclyl, 5- to 15-membered heteroaryl, ether group, -CONR¹³R¹⁴, or -NR¹³COR¹⁴;
         R¹³ and R¹⁴ are each independently hydrogen, C₁-C₆ alkyl, C₃-C₈ cycloalkyl, C₆-C₁₀ aryl, 4- to 15- membered heterocyclyl, 5- to 15-membered heteroaryl, or ether;
         wherein the definitions of L¹ and D are as follows:
            L¹ is selected from the group consisting of: 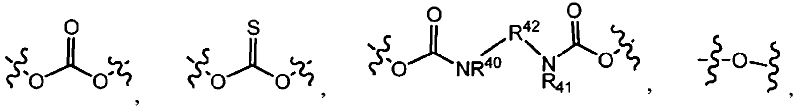 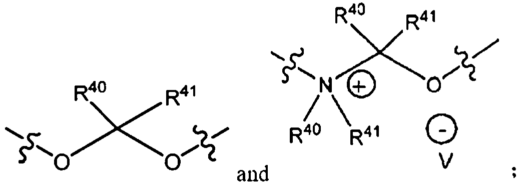
            R⁴⁰ and R⁴¹ are independently hydrogen, C₁-C₆ alkyl, C₂-C₆ alkenyl, C₂-C₆ alkynyl, C₃-C₈ cycloalkyl, C₆-C₁₀ aryl, 4- to 15-membered heterocyclyl, or 5- to 15-membered heteroaryl;
            R⁴² is C₂-C₃ alkylene or heteroalkylene which are optionally substituted with 1 to 3 C₁-C₆ alkyl groups;
            V (-) is any anion, preferably a pharmaceutically acceptable anion;
            D is a moiety such that D-OH is an anticancer drug, wherein OH is an aliphatic hydroxyl group or a phenolic hydroxyl group, or is an OH moiety attached to a phosphorus atom as provided herein; or
            L¹ is: 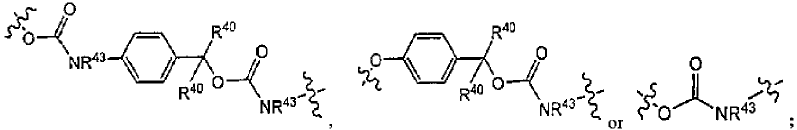
            R⁴⁰ is as defined above, R⁴³ is hydrogen or together with D forms a heterocycle, and the phenylene moiety is optionally substituted, and
            D is a moiety such that D-NR⁴³H is an anticancer drug; or
            L¹ is a bond, -O-C(R⁴⁰R⁴¹)-, -O-C(R⁴⁰R⁴¹)-NR⁴⁰R⁴¹(+)-C(R⁴⁰R⁴¹)-, or 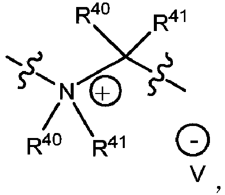
            wherein R⁴⁰, R⁴¹, and V are as defined above; and
            D is an anticancer drug containing a tertiary or secondary nitrogen atom, wherein the tertiary or secondary nitrogen atom is bonded to L¹; and
            wherein the alkyl, alkenyl, alkynyl, cycloalkyl, aryl, heterocyclyl, heteroaryl, and ether are optionally substituted. 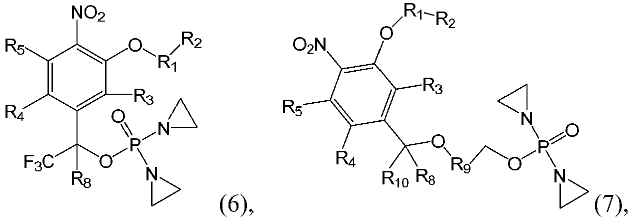
            wherein the definitions of R₁, R₂, R₃, R₄, R₅, R₈, R₉, and R₁₀ are described in the claims of the patent application PCT/CN2020/089692 with publication number WO2020228685A9 (corresponding to the Chinese patent application No. 2020800358890 with publication number CN113853379A), and the synthesis and preparation methods of the specific compounds are also described in the above application, which is incorporated herein by reference in its entirety. The specific definitions are as follows:
               R₁ is C₆-C₁₀ aryl or Z-substituted aryl, 4- to 15-membered heterocyclyl or Z-substituted heterocyclyl, 5- to 15-membered heteroaryl or Z-substituted heteroaryl, 7- to 15-membered fused ring or Z-substituted fused ring;
               R₂ is hydrogen, halogen, cyano or isocyano, hydroxyl, sulfhydryl, amino, OTs, OMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4- to 15-membered heterocyclyl or Z-substituted heterocyclyl, 5- to 15-membered heteroaryl or Z-substituted heteroaryl, ether of 1 to 6 carbon atoms or Z-substituted alkoxy group of 1 to 6 carbon atoms, -CONR⁶R⁷, -SO₂NR⁶R⁷, -SO₂R⁶, -OCOO-R⁶, -COOR⁶, -NR⁶COR⁷, -OCOR⁶, -NR⁶SO₂R⁷ or -NR⁶SO₂NR⁶R⁷, or R² together with the atom in the group R¹ to which it is bonded form a 7-15-membered fused ring or Z-substituted fused ring;
               R₃ is hydrogen, halogen, cyano or isocyano, hydroxyl, sulfhydryl, amino, OTs, OMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4- to 15-membered heterocyclyl or Z-substituted heterocyclyl, 5- to 15-membered heteroaryl or Z-substituted heteroaryl, C₁-C₆ alkoxy or Z-substituted C₁-C₆ alkoxy, -CONR⁶R⁷, -SO₂NR⁶R⁷, -SO₂R⁶, -OCO-R⁶, -OCOO-R⁶, - COOR⁶, -NR⁶COR⁷, -OCOR⁶, or -NR⁶SO₂R⁷;
               R₄ and R₅ are each independently hydrogen, halogen, cyano or isocyano, hydroxyl, sulfhydryl, amino, OTs, OLCMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4- to 15-membered heterocyclyl or Z-substituted heterocyclyl, 5- to 15-membered heteroaryl or Z-substituted heteroaryl, C₁-C₆ alkoxy or Z-substituted C₁-C₆ alkoxy, -CONR⁶R⁷, - SO₂NR⁶R⁷, -SO₂R⁶, -OCOO-R⁶, -COOR⁶, -NR⁶COR⁶, -OCOR⁶ or -NR⁶SO₂R⁷, or R⁴ and R⁵ together with the atom in the benzene ring to which they are bonded to form a 7- to 15-membered fused ring or Z-substituted fused ring;
               R⁶ and R⁷ are each independently hydrogen, cyano or isocyano, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4- to 15-membered heterocyclyl or Z-substituted heterocyclyl, 5- to 15-membered heteroaryl or Z-substituted heteroaryl, C₁-C₆ alkoxy or Z-substituted C₁-C₆ alkoxy, or R⁶ and R⁷ together with the atom to which they are bonded form a 5- to 7-membered heterocyclyl or Z-substituted 5- to 7-membered heterocyclyl;
               R₈ and R₁₀ are each independently hydrogen, deuterium, aryl or Z-substituted aryl, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, and at least one of R⁸ and R¹⁰ must be hydrogen or deuterium;
               R₉ is substituted C₆-C₁₀ aryl with at least one fluorine atom or nitro substituent, substituted 4- to 15-membered heterocyclyl with at least one fluorine atom or nitro substituent, or substituted 5- to 15-membered heteroaryl with at least one fluorine atom or nitro substituent;
               the substituent Z is halogen, cyano or isocyano, hydroxyl, sulfhydryl, amino, OTs, OMS, C₁-C₃ alkyl or substituted alkyl, C₁-C₃ alkoxy or substituted alkoxy, C₂-C₃ alkenyl or substituted alkenyl, C₂-C₃ alkynyl or substituted alkynyl, C₃-C₈ cycloalkyl or substituted cycloalkyl, aromatic ring group or substituted aromatic ring group, heterocyclyl or substituted heterocyclyl, heteroaromatic ring group or substituted heteroaromatic ring group, or fused ring group or substituted fused ring group, wherein the pattern of substitution is mono- or gem-di-substitution;
               in R₉, the substituent in the substituted C₆-C₁₀ aryl, substituted 4- to 15-membered heterocyclyl or substituted 5- to 15-membered heteroaryl is halogen, nitro, cyano or isocyano, hydroxyl, amino, C₁-C₃ alkyl or alkoxy, alkenyl, alkynyl, cycloalkyl, benzene ring group or substituted benzene ring group, C₁-C₃ alkoxy or halogen atom-substituted alkoxy. 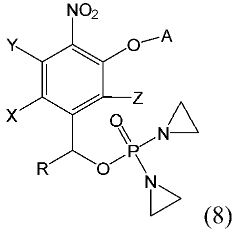 wherein:
                  A is substituted or unsubstituted C₆-C₁₀ aryl, biaryl or substituted biaryl, 5- to 15-membered heteroaryl, or -N=CR¹R²; wherein the substituent is selected from the group consisting of halogeno, -CN, -NO₂, -O-(CH₂)-O-, -CO₂H and salt thereof, -OR¹⁰⁰, - CO₂R¹⁰⁰, -CONR¹⁰¹R¹⁰², -NR¹⁰¹R¹⁰², -NR¹⁰⁰SO₂R¹⁰⁰, -SO₂R¹⁰⁰, -SO₂NR¹⁰¹R¹⁰², C₁-C₆ alkyl, and C₃-C₁₀ heterocyclyl;
                  wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5- to 7-membered heterocycle;
                  wherein the alkyl group and the aryl group are each substituted by 1-3 halogeno groups or 1-3 C₁-C₆ alkyl groups;
                  R¹ and R² are each independently phenyl or methyl;
                  X, Y, and Z are each independently hydrogen or halogeno; and
                  R is hydrogen or C₁-C₆ alkyl or halogen-substituted alkyl. 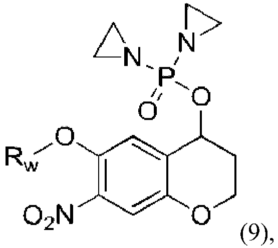
                  wherein the definitions of R_{w} are described in the claims of the patent application PCT/CN2020/120281 with publication number WO2021068952A1 (corresponding to the Chinese patent application No. 202080071652.8 with publication number CN114555574A), and the synthesis and preparation methods of the specific compounds are also described in the above application, which is incorporated herein by reference in its entirety. The specific definitions are as follows:
                     Rw is 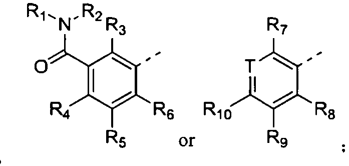
                     R₁ is H, C₁₋₆ alkyl, C₃₋₆ cycloalkyl, 4- to 6-membered heterocycloalkyl, 5- to 6-membered heteroaryl or phenyl, wherein the C₁₋₆ alkyl, C₃₋₆ cycloalkyl, 4- to 6-membered heterocycloalkyl, 5-6-membered heteroaryl, and phenyl are optionally substituted with 1, 2 or 3 R^{a} groups;
                     each R^{a} is independently H, F, Cl, Br, I, -CN, -OH, C₁₋₃ alkoxy, or C₁₋₃ alkyl;
                     R₂ is H or C₁₋₆ alkyl;
                     or R₁ and R_{2,} together with the N atom to which they are attached, form a 4- to 6-membered heterocycloalkyl, wherein the 4- to 6-membered heterocycloalkyl is optionally substituted with 1, 2 or 3 R^{b} groups;
                     each R^{b} is independently H, F, Cl, Br, I, -CN, -OH, -NH₂, -OCH₃, -OCH₂CH₃, -CH₃, or -CH₂CH₃;
                     R₃ is H, F, Cl, Br, I, -OH, -NH₂, C₁₋₃ alkoxy, or C₁₋₃ alkyl;
                     or R₂ and R₃ are attached together to make the structural unit 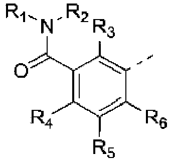 to be 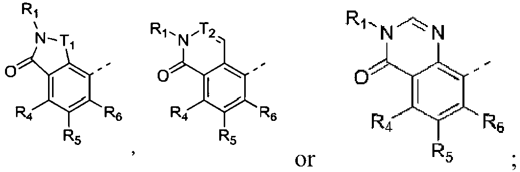
                     T₁ is -(CR^{c}R^{d})ₘ- or -(CR^{c}R^{d})ₙ-O-;
                     m is 1, 2 or 3;
                     n is 1 or 2;
                     T₂ is N or CH;
                     R^{c} and R^{d} are each independently H, F, C₁₋₃ alkyl, or C₁₋₃ alkoxy;
                     R₄, R₅ and R₆ are each independently H, F, Cl, Br, I, C₁₋₃ alkyl, or C₁₋₃ alkoxy;
                     T is N or CH;
                     R₇ and R₈ are each independently H, F, Cl, Br, or I;
                     R₉ and R₁₀ are each independently H, F, Cl, Br, I, -CN; or
                     the 4- to 6-membered heterocycloalkyl and 5- to 6-membered heteroaryl each comprise 1, 2, 3 or 4 heteroatoms independently selected from the group consisting of N, -O- and -S-. 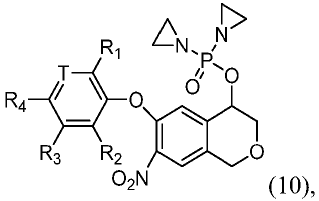
                     wherein the definitions of R₁, R₂, R₃, R₄, and T have been described in the claims of the patent application PCT/CN2021/118597 with publication number WO2022057838A1, and the synthesis and preparation methods of the specific compounds are also described in the above application, which is incorporated herein by reference in its entirety. The specific definitions are as follows:
                        T is N or CH;
                        R₁ and R₂ are each independently H, F, Cl, Br, I, or C₁₋₃ alkyl, wherein said C₁₋₃ alkyl is optionally substituted with 1, 2 or 3 Rₐ groups;
                        each Rₐ is independently F, Cl, Br, I, -CN, -OH, or-NH₂;
                        R₃ and R₄ are each independently H, F, Cl, Br, I, CN, C₁₋₃ alkyl, C₁₋₃ alkoxy, 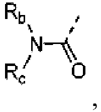 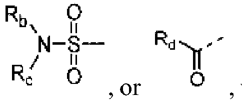 wherein the C₁₋₃ alkyl is optionally substituted with 1, 2 or 3 Rₑ;
                        R_{b} and R_{c} are each independently H, -CH₃, -CH₂CH₃, -(CH₂) ₂CH₃, or -CH (CH₃) ₂;
                        R_{d} is-CH₃, -CH₂CH₃, -(CH₂)₂CH₃, or -CH(CH₃)₂;
                        each Rₑ is independently F, Cl, Br, I, -CN, -OH, or-NH₂. 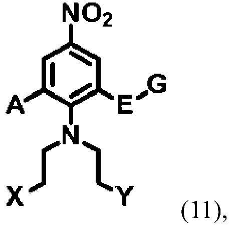
                        wherein the definitions of A, E, G, X, and Y are described in the claims of the patent application PCT/NZ2019/050030 with publication number WO2019190331A1 (corresponding to the Chinese patent application No. 2019800234236 with publication number CN111918864A), and the synthesis and preparation methods of the specific compounds are also described in the above application, which is incorporated herein by reference in its entirety. The specific definitions are as follows:
                           A is H, C₁-C₆ alkyl, C₁-C₆ alkenyl, C₁-C₆ alkynyl, CFH₂, CF₂H, CF₃, F, Cl, Br, I, OCF₃, COR, or CON(R)₂;
                           E is SO or SO₂;
                           X is Cl, Br, I, or OSO₂R;
                           Y is Cl, Br, I, or OSO₂R;
                           each R is independently H or C₁-C₆ alkyl;
                           G is a free radical group selected from the group consisting of Formulae (B)-(AA): 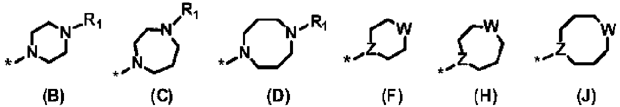 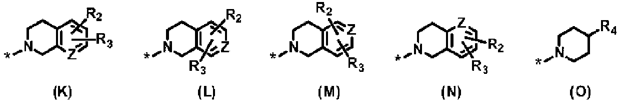 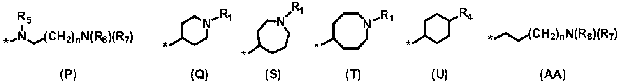 wherein:
                              R₁ is H, C₁-C₆ alkyl, CH₂(CH₂)ₙOH, CH₂CH(OH)CH₂OH, phenyl, pyridyl, benzyl, or pyridylmethyl, provided that when R₁ is phenyl, pyridyl, benzyl, or pyridylmethyl, R₁ is optionally substituted at any available position with C₁-C₆ alkyl, C₁-C₆ alkenyl, C₁-C₆ alkynyl, OR₆, N(R₆)(R₇), CFH₂, CF₂H, CF₃, F, Cl, Br, **I,** OCF₃, COR₆, CON(R₆)(R₇), SOR₆, SON(R₆)(R₇), SO₂R₆, SO₂N(R₆)(R₇), CN, or NO₂;
                              R₂ and R₃ are each independently H, C₁-C₆ alkyl, C₁-C₆ alkenyl, C₁-C₆ alkynyl, OR₆, N(R₆)(R₇), CFH₂, CF₂H, CF₃, F, Cl, Br, **I,** OCF₃, COR₆, CON(R₆)(R₇), SOR₆, SON(R₆)(R₇), SO₂R₆, SO₂N(R₆)(R₇), CN, or NO₂;
                              R₄ is N(R₆)(R₇), OH, OCH₂(CH₂)ₙN(R₆)(R₇), or CH₂(CH₂)ₙN(R₆)(R₇);
                              R₅ is H or a C₁-C₆ alkyl group;
                              R₆ and R₇ are each independently H or C₁-₆ alkyl, or R₆ and R₇ together form a substituted or unsubstituted 5-membered or 6-membered heterocycle;
                              Z is CH or N;
                              W is CH₂, O, S, SO, or SO₂;
                              n is 0 to 6;
                              * represents the point of attachment to Formula (I). 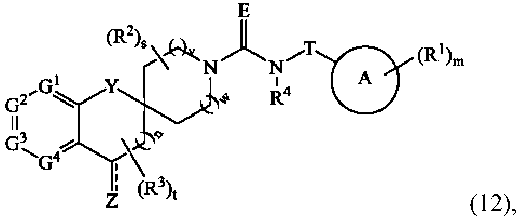
                              wherein the definitions of R₁, R₂, R₃, R₄, G₁, G₂, G₃, G₄, E, T, Y, Z, m, n, s, t, v, w, and ring A have been described in the claims of the patent application CN202210585771.6 with publication number CN115403579A, and the synthesis and preparation methods of the specific compounds are also described in the above application, which is incorporated herein by reference in its entirety. The specific definitions are as follows:
                                 G¹, G², G³, or G⁴ is identical or different, and is each independently CR⁵ or N atom;
                                 Each R⁵ is identical or different, and is each independently selected from the group consisting of hydrogen, halogen, alkyl, alkenyl, alkynyl, alkoxy, hydroxyl, cyano, amino, nitro, -NR^{a}R^{b}, -C(O)NR^{a}R^{b}, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted by one or more substituents selected from the group consisting of halogen, alkyl, haloalkyl, alkoxy, haloalkoxy, hydroxyalkyl, hydroxyl, oxo, cyano, amino, nitro, cycloalkyl, heterocyclyl, aryl, and heteroaryl;
                                 Y is selected from the group consisting of -(C(R^{y2}R^{y3}))_{f}-NR^{y1}-, -(C(R^{y2}R^{y3}))_{g}-O-, - (C(R^{y2}R^{y3}))ₕ-S-, -(C(R^{y2}R^{y3}))ₕ-S(O)-, -(C(R^{y2}R^{y3}))ₕ-S(O)₂-, -C(R^{y2}R^{y3})-, -NR^{y1}-(C(R^{y2}R^{y3}))_{f}-, -O-(C(R^{y2}R^{y3}))_{g}-, -S-(C(R^{y2}R^{y3}))ₕ-, -S(O)-(C(R^{y2}R^{y3}))ₕ- and -S(O)₂-(C(R^{y2}R^{y3}))ₕ-;
                                 R^{y1} is selected from the group consisting of hydrogen, alkyl, haloalkyl, hydroxyalkyl, cycloalkyl, and heterocyclyl;
                                 R^{y2} and R^{y3} are identical or different, and are independently selected from the group consisting of hydrogen, halogen, alkyl, haloalkyl, hydroxyalkyl, cycloalkyl, and heterocyclyl;
                                 or R^{y2} and R^{y3} together form =O;
                                 Z is O or OH;
                                 -̅ -̅ -̅ -̅ -̅ is a single bond or a double bond, Z is OH when -̅ -̅ -̅ -̅ -̅ is a single bond, and Z is O when -̅ -̅ -̅ -̅ -̅ is a double bond;
                                 E is selected from the group consisting of NH, O atom and S atom;
                                 T is selected from the group consisting of -C(R^{T1}R^{T2})-, -NR^{T3}- and -O-;
                                 R^{T1} and R^{T2} are identical or different, and are independently selected from the group consisting of hydrogen, deuterium, halogen, alkyl, haloalkyl, hydroxyalkyl, cycloalkyl, and heterocyclyl;
                                 or, R^{T1} and R^{T2} together with the carbon atom to which they are attached form cycloalkyl or heterocyclyl, wherein the cycloalkyl or heterocyclyl is each independently optionally substituted by one or more substituents selected from the group consisting of halogen, alkyl and hydroxyl;
                                 R^{T3} is selected from the group consisting of hydrogen, alkyl, haloalkyl, hydroxyalkyl, cycloalkyl, and heterocyclyl;
                                 Ring A is 6- to 10-membered aryl or 5- to 10-membered heteroaryl;
                                 Each R¹ is identical or different, and is each independently selected from the group consisting of hydrogen, deuterium, halogen, alkyl, alkenyl, alkynyl, alkoxy, hydroxyl, cyano, -NR^{a}R^{b}, -C(O)NR^{a}R^{b}, -S(O)NR^{a}R^{b}, -S(O)₂NR^{a}R^{b}, -S(O)R^{c}, -S(O)₂R^{c}, -B(OR^{d})₂, nitro, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted by one or more substituents selected from the group consisting of halogen, alkyl, haloalkyl, alkoxy, haloalkoxy, hydroxyalkyl, hydroxyl, oxo, cyano, - NR^{a}R^{b}, -C(O)NR^{a}R^{b}, -S(O)NR^{a}R^{b}, -S(O)₂NR^{a}R^{b}, -S(O)R^{c}, -S(O)₂R^{c}, -B(OR^{d})₂, nitro, cycloalkyl, heterocyclyl, aryl, and heteroaryl;
                                 R^{a} and R^{b} are identical or different, and are each independently selected from the group consisting of hydrogen, alkyl, haloalkyl, hydroxyl, hydroxyalkyl, -C(O)R^{e}, cycloalkyl, and heterocyclyl; or R^{a} and R^{b} together with the nitrogen atom to which they are attached form cycloalkyl or heterocyclyl, wherein the cycloalkyl or heterocyclyl is optionally substituted by one or more substituents selected from the group consisting of halogen, alkyl, haloalkyl, alkoxy, haloalkoxy, hydroxyalkyl, hydroxyl, cyano, amino, nitro, cycloalkyl, heterocyclyl, aryl, and heteroaryl;
                                 R^{c} is selected from the group consisting of alkyl, cycloalkyl, heterocyclyl, aryl, and heteroaryl, and the alkyl, cycloalkyl, heterocyclyl, aryl, and heteroaryl are optionally substituted by one or more substituents selected from the group consisting of halogen, alkyl, haloalkyl, alkoxy, haloalkoxy, hydroxyalkyl, hydroxyl, cyano, amino, nitro, cycloalkyl, heterocyclyl, aryl, and heteroaryl;
                                 R^{d} is hydrogen or C₁₋₆ alkyl;
                                 R^{e} is selected from the group consisting of alkyl, alkoxy, cycloalkyl, heterocyclyl, aryl, and heteroaryl, and the alkyl, alkoxy, cycloalkyl, heterocyclyl, aryl, and heteroaryl are optionally substituted by one or more substituents selected from the group consisting of halogen, alkyl, haloalkyl, alkoxy, haloalkoxy, hydroxyalkyl, hydroxyl, cyano, amino, nitro, cycloalkyl, heterocyclyl, aryl, and heteroaryl;
                                 Each R² is identical or different, and is each independently selected from the group consisting of hydrogen, deuterium atom, halogen, alkyl, haloalkyl, alkoxy, haloalkoxy, hydroxyalkyl, oxo, hydroxyl, cyano, amino, nitro, cycloalkyl, heterocyclyl, aryl, and heteroaryl;
                                 Each R³ is identical or different, and is each independently selected from the group consisting of hydrogen, deuterium, halogen, alkyl, haloalkyl, alkoxy, haloalkoxy, hydroxyalkyl, oxo, hydroxyl, cyano, amino, nitro, cycloalkyl, heterocyclyl, aryl, and heteroaryl;
                                 R⁴ is selected from the group consisting of hydrogen, alkyl, haloalkyl, hydroxyl, and hydroxyalkyl;
                                 n is 0, 1, 2 or 3;
                                 v is 0, 1 or 2;
                                 w is 0, 1 or 2;
                                 f is 0, 1 or 2;
                                 g is 0, 1 or 2;
                                 h is 0, 1 or 2;
                                 m is 0, 1, 2, 3, 4, or 5;
                                 s is 0, 1, 2, 3, 4, 5, 6, 7, or 8;
                                 t is 0, 1, 2, 3, 4, 5, or 6;
                                 provided that,
                                 when Y is -O- atom and E is O atom, ring A is phenyl or 5- to 6-membered heteroaryl, and G³ is CR⁵ or N atom, R⁵ is not hydrogen atom;
                                 when Y is -O- atom and E is S atom, ring A is phenyl or 5- to 6-membered heteroaryl; when G¹, G², G³, and G⁴ are all CR⁵, Y is NR^{y1}, n, v, and w are all 1, and E is O atom, 1) T is not CH₂ or CD₂, 2) at least one of R² and R³ groups is deuterium atom, 3) R⁴ is selected from the group consisting of alkyl, haloalkyl, hydroxyl, and hydroxyalkyl, 4) one of R¹ groups is 3- to 8-membered cycloalkyl or 5- to 8-membered heterocyclyl, and the 3- to 8-membered cycloalkyl or 5- to 8-membered heterocyclyl is substituted by one or more substituents selected from the group consisting of halogen, alkyl, haloalkyl, alkoxy haloalkoxy, hydroxyalkyl, hydroxyl, oxo, cyano, amino, nitro, cycloalkyl, heterocyclyl, aryl, and heteroaryl, and 5) ring A is R^{d}, which is hydrogen atom or C₁₋₆ alkyl. 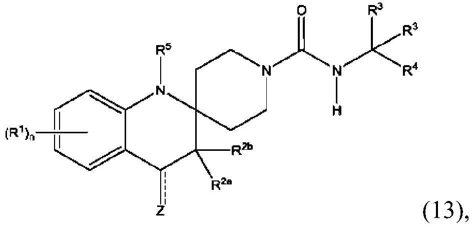
                                 wherein the definitions of R¹, R^{2a}, R^{2b}, R³, R⁴, R⁵, n, and Z are described in the claims of the patent application PCT/IB2020/057285 with publication number WO2021005586A1 (corresponding to the Chinese patent application No. CN202080053804.1 with publication number CN114206870A), and the synthesis and preparation methods of the specific compounds are also described in the above application, which is incorporated herein by reference in its entirety. The specific definitions are as follows:
                                    -̅ -̅ -̅ -̅ -̅ is a single bond or a double bond;
                                    Z is OH when -̅ -̅ -̅ -̅ -̅ is a single bond; or Z is O when -̅ -̅ -̅ -̅ -̅ is a double bond;
                                    each R¹ is independently selected from the group consisting of (C₁-C₆)alkyl, (C₁-C₆)alkoxy, (C₀-C₄)alkylN(R⁸)₂, and halogeno;
                                    R^{2a} and R^{2b} are each independently selected from the group consisting of H, (C₁-C₆) alkyl, and halogeno;
                                    each R³ is independently selected from the group consisting of H and halogeno;
                                    R⁴ is selected from the group consisting of aryl, 5- to 6-membered heteroaryl comprising 1, 2, 3, or 4 heteroatoms independently selected from the group consisting of N, O, and S; and 9- to 10-membered fused bicyclic heteroaryl comprising 1, 2, 3, or 4 heteroatoms independently selected from the group consisting of N, O, and S; wherein any one of the foregoing groups is optionally substituted with one or more R⁶ groups; R⁵ is selected from the group consisting of H; (C₁-C₆)alkyl; (C₂-C₆)alkenyl; (C₀-C₄)alkylOR⁸; (C₁-C₄)alkyl(C₃-C₁₀)cycloalkyl; halo(C₁-C₆)alkyl; (C₂-C₃)alkynyl; and (C₁-C₄)alkylN(R¹⁰)₂;
                                    each R⁶ is independently selected from the group consisting of halogeno;(C₁-C₆)alkyl; (C₁-C₆)alkoxy; halo(C₁-C₆)alkyl; OH; aryl; 3- to 6-membered heterocyclyl; 5- to 6-membered heteroaryl; (C₀-C₄)alkylS(O)ₘ(C₁-C₆)alkyl; halo(C₁-C₆)alkoxy; (C₀-C₄)alkylS(O)ₘN(R⁸)₂; (C₀-C₄)alkylN(R⁸)₂; (C₀-C₄)alkyl(CO)OR⁷; N(R⁸)S(O)ₘ(C₁-C₆)alkyl; N(R⁸)S(O)ₘ(C₃-C₆)cycloalkyl; OP(O)(OH)₂; (C₀-C₃)alkyl(CO)NHR¹¹; (C₀-C₃)alkylOR⁷, and (C₃-C₁₀)cycloalkyl; wherein each R⁶, when not being halogeno, OH, or OP(O)(OH)₂, is optionally substituted with one to three R⁹ groups; or two adjacent R⁶, together with the atoms to which they attach, form a 5- to 7- membered heterocyclyl or (C₅-C₈)cycloalkyl;
                                    each of R⁷ and R⁸ is independently selected from the group consisting of H or (C₁-C₆)alkyl optionally substituted with one to three R⁹ groups;
                                    each R⁹ is independently selected from the group consisting of halogeno; -OH; amino, (C₁-C₄)alkylamino, di(C₁-C₄)alkylamino, OP(O)(OH)₂; (C₁-C₆)alkyl; (C1-C3)alkynyl; (C₁-C₆)alkoxy; halo(C₁-C₆)alkyl; (C₀-C₄)alkylS(O)ₘ(C₁-C₆)alkyl; halo(C₁-C₆)alkoxy; 3- to 6-membered heterocyclyl which is optionally substituted with oxo (=O); (C₀-C₄)alkylS(O)ₘN(R¹⁰)₂; (C₀-C₄)alkyl(CO)R¹⁰; (C₀-C₄)alkyl(CO)OR¹⁰; (C₀-C₄)alkylNR¹⁰S(O)ₘ(C₁-C₆)alkyl; (C₀-C₄)alkylOR¹⁰; (C₀-C₄)alkylN(R¹⁰)₂; (C₀-C₄)alkylCN; (C₀-C₄)alkylN(R¹⁰)₂; and (C₀-C₄)alkyl(CO)N(R¹⁰)₂;
                                    each R¹⁰ is independently selected from the group consisting of H, (C₁-C₆)alkyl, and 3-to 6-membered heterocyclyl, wherein the 3- to 6-membered heterocyclyl is optionally substituted with one or more of (C₁-C₆)alkyl; and oxo(=O);
                                    each R¹¹ is selected from the group consisting of H; 4- to 6-membered heterocyclyl which is optionally substituted with one to four R¹² groups; (C₃-C₆)cycloalkyl which is optionally substituted with one to four R¹²; (C₀-C₃)alkyl(C₃-C₆)cycloalkyl (C₁-C₃)alkyl which is optionally substituted with halogeno;CH₂-aryl which is optionally substituted with one to three R¹² groups; (C₁-C₆)alkyl; (C₂-C₆)alkenyl; and (C₂-C₆)alkynyl, wherein each of the (C₁-C₆)alkyl; (C₂-C₆)alkenyl; and (C₂-C₆)alkynyl is optionally substituted with one or more groupsR¹³ groups;
                                    each R¹² is independently selected from the group consisting of OH, (C₁-C₃)alkoxy, NH₂; and (C₁-C₃)alkyl optionally substituted with one or more OH groups;
                                    each R¹³ is independently selected from the group consisting of halogeno, OH, amino, (C₁-C₄)alkylamino, di(C₁-C₄)alkylamino, (C₁-C₃)alkoxy; and C(O)-(C₃-C₈)cycloalkyl; m is 0, 1, or 2; and
                                    n is 0, 1, or 2. 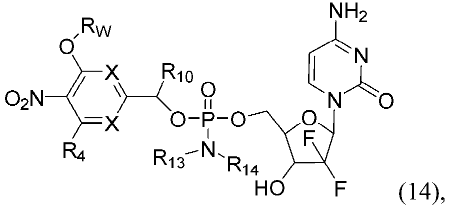
                                    wherein the definitions of R_{w}, X, R₄, R₁₀, R₁₃, and R₁₄ have been described in the claims of the patent application PCT/CN2022/098082 with publication number WO2022258043A1, and the synthesis and preparation methods of the specific compounds are also described in the above application, which is incorporated herein by reference in its entirety. The specific definitions are as follows:
                                       two X groups are each independently CR¹⁵ or N;
                                       R₁₃ and R₁₄ are each independently hydrogen, C₁-C₆ alkyl, cycloalkyl, alkenyl, alkynyl, C₆-C₂₀ aryl, 5- to 20-membered heterocyclyl; halogen-substituted C₁-C₆ alkyl, cycloalkyl, alkenyl, alkynyl; halogen-substituted C₆-C₂₀ aryl; or halogen-substituted 5-to 20-membered heterocyclyl, wherein R₁₃ and R₁₄ are not hydrogen at the same time;
                                       R₁₀ is hydrogen, C₁-C₆ alkyl, cycloalkyl, alkenyl, alkynyl, C₆-C₂₀ aryl, 5- to 20-membered heterocyclyl; halogen-substituted C₁-C₆ alkyl, cycloalkyl, alkenyl or alkynyl; halogen-substituted C₆-C₂₀ aryl; or halogen-substituted 5- to 20-membered heterocyclyl; or, R₁₀ and R₁₃ or R₁₄ may be connected to form a 5- to 9-membered ring under the condition that they meet the above definitions for R₁₀, R₁₃, and R₁₄;
                                       R₄ and R¹⁵are each independently hydrogen, halogen, C₁-C₆ alkyl, cycloalkyl, alkenyl, alkynyl, alkoxy, cyano, 5- to 20-membered heterocyclyl, C₆-C₂₀ aryl; or halogen-substituted C₁-C₆ alkyl, cycloalkyl, alkenyl, alkynyl, alkoxy, 5- to 20-membered heterocyclyl, C₆-C₂₀ aryl;
                                       or, R₁₀ and R¹⁵ can form a 4- to 12-membered cyclic hydrocarbon or heterocyclyl under the condition that they meet the above definitions for R₁₀ and R¹⁵;
                                       Rw is 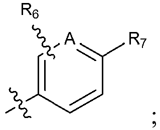
                                       A is CR¹⁶ or N, and the position of A can be changed on the ring;
                                       R¹⁶ is hydrogen, C₁-C₆ alkyl, cycloalkyl, alkenyl, alkynyl, C₆-C₂₀ aryl, 5- to 20-membered heterocyclyl; halogen-substituted C₁-C₆ alkyl, cycloalkyl, alkenyl, alkynyl; halogen-substituted C₆-C₂₀ aryl; or halogen-substituted 5- to 20-membered heterocyclyl; R₆ and R₇ meet the following conditions:
                                          R₆ and R₇ are each independently hydrogen, halogen, cyano, hydroxyl, C₁-C₆ alkyl, cycloalkyl, alkenyl, alkynyl, alkoxy, 5- to 20-membered heterocyclyl, C₆-C₂₀ aryl; or halogen-substituted C₁-C₆ alkyl, cycloalkyl, alkenyl, alkynyl, alkoxy, 5- to 20-membered heterocyclyl, C₆-C₂₀ aryl; or cyano-substituted C₁-C₆ alkyl, cycloalkyl, alkenyl, alkynyl, alkoxy, 5- to 20-membered heterocyclyl, C₆-C₂₀ aryl; or hydroxyl-substituted C₁-C₆ alkyl, cycloalkyl, alkoxy, 5- to 20-membered heterocyclyl, C₆-C₂₀ aryl; or -CONR¹¹R¹²; or -CH₂NR¹¹R¹²;
                                          or, R₆ and R₇ are connected to form
                                          a 5- to 8-membered single or fused heterocycle containing at least one N or S or O, or two or three of N, S and O at the same time;
                                          or, a 5- to 8-membered single or fused heterocycle containing at least one N or S or O, or two or three of N, S and O at the same time, and the single or fused heterocycle is substituted with C₁-C₆ alkyl;
                                          R₆ and CR¹⁶ can be connected to form a 5- to 9-membered ring, heterocycle, or aromatic heterocycle;
                                          R¹¹ and R¹² meet the following conditions:
                                             R¹¹ and R¹² are each independently C₁-C₆ alkyl or halogen-substituted C₁-C₆ alkyl, or on condition that R¹¹ and R¹² meet the above definitions for R¹¹ and R¹², they form a 5-to 7-membered ring with N atom in -CONR¹¹R¹², or form a 5- to 7-membered ring with N atom in -CH₂NR¹¹R¹². 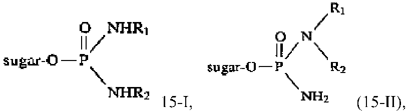
                                             wherein the definitions of Sugar, R₁ and R₂ are as described in the claims of the patent application US5622936A, and the synthesis and preparation methods of the specific compounds are also described in the above application, which is incorporated herein by reference in its entirety. The specific definitions are as follows:
                                                the sugar moiety is attached to a phosphamide mustard residue (15-I) or an ifosfamide mustard residue (15-II), R₁ and R₂ may be the same or different and are selected from the group consisting of hydrogen, C₁-C₄ alkyl, and C₁-C₆ haloalkyl;
                                                and the sugar moiety is an isomer or enantiomer form of any existing monosaccharide, disaccharide or polysaccharide.

For other structures of DNA alkylating agents activated by β-D-Glucosidase, Carboxylesterase, Esterase and Caspase-3, Cathepsin B, γ-Glutamyltranspeptidase, and β-galactosidase, please refer to the review reference (Han HH, Wang HM, Jangili P, et al. The design of small-molecule prodrugs and activatable phototherapeutics for cancer therapy. Chem Soc Rev. 2023;52(3):879-920. Published on Feb 6, 2023, doi:10.1039/d2cs00673a).

The compound of Formula (1) is selected from the group consisting of the compounds of the following Formulae:

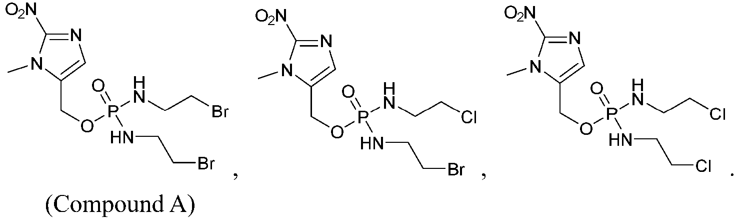

The synthesis and preparation methods of the compounds are also described in Patent Application PCT/US2006/025881 with publication number WO2007002931 (corresponding to the Chinese application No. 2006800300828 with publication number CN101501054A), which is incorporated herein by reference in its entirety.

The compound of Formula (2) is selected from the group consisting of the compounds of the following Formulae:

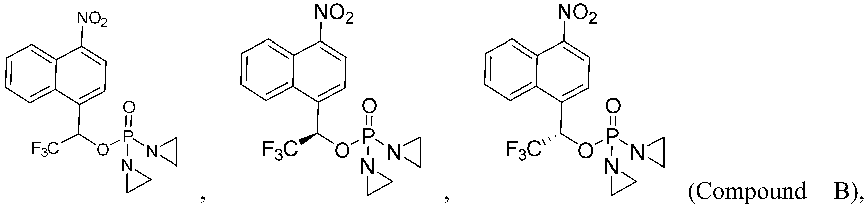

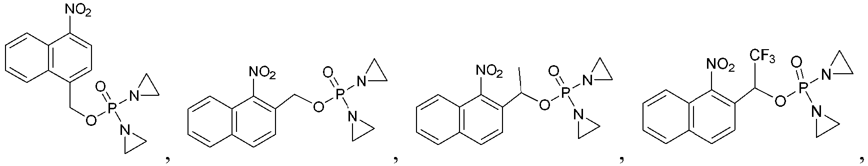

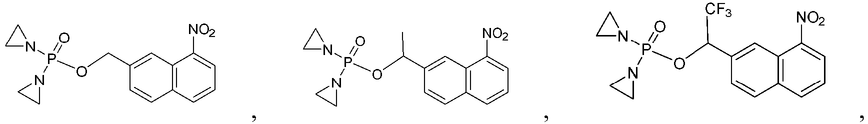

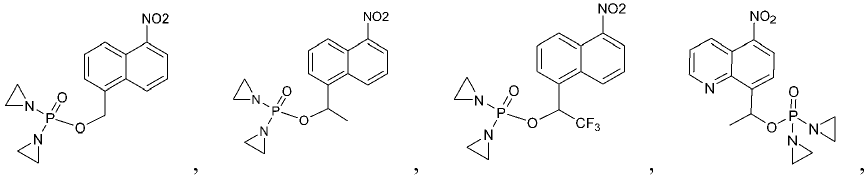

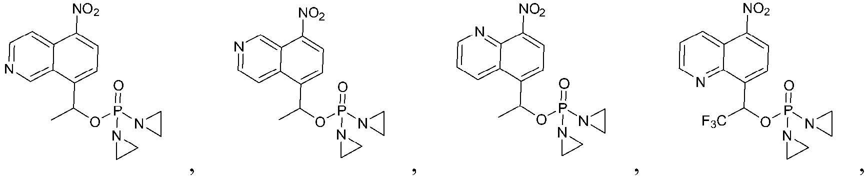

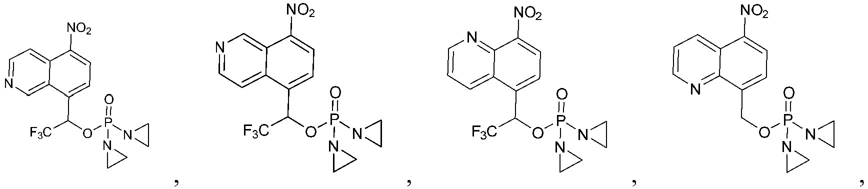

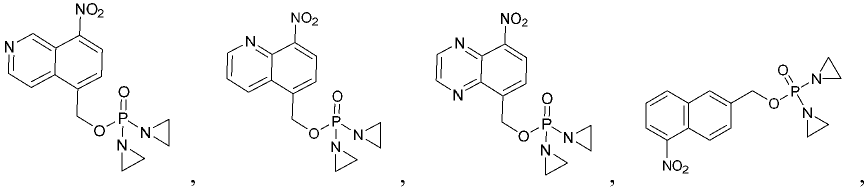

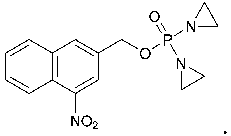

The synthesis and preparation methods of the compounds are also described in Patent Application PCT/CN2020/114519 with publication number WO2021120717A1 (corresponding to the Chinese application No. 2020800673113 with publication number CN114466853A), which is incorporated herein by reference in its entirety.

The compound of Formula (3) is selected from the group consisting of the compounds of the following Formulae:

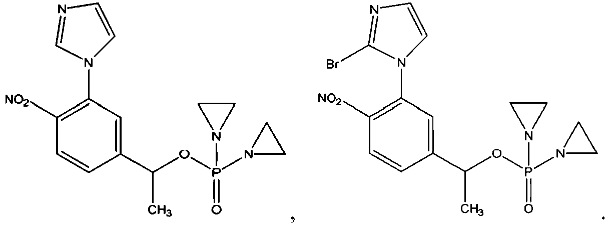

The synthesis and preparation methods of the compounds are also described in Patent Application PCT/US2016/039092 with publication number WO2016210175A1 (corresponding to the Chinese application No. 2016800368985 with publication number CN108024974A), which is incorporated herein by reference in its entirety.

The compound of Formula (4) is selected from the group consisting of the compounds of the following Formulae:

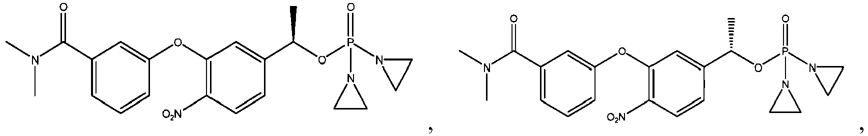

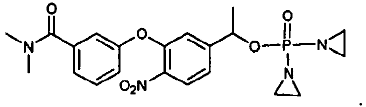

The synthesis and preparation methods of the compounds are also described in Patent Application PCT/US2016/021581 with publication number WO2016145092A1 (corresponding to the Chinese application No. 2016800150788 with publication number CN107530556A), which is incorporated herein by reference in its entirety.

The compound of Formula (5) is selected from the group consisting of the compound of the following Formula:

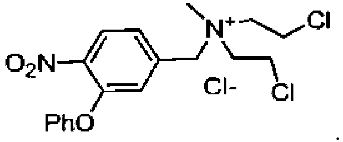

The synthesis and preparation method of the compound is also described in Patent Application PCT/US2016/025665 with publication number WO2016161342A3 (corresponding to the Chinese application No. 2016800200132 with publication number CN108136214A), which is incorporated herein by reference in its entirety.

The compounds of Formulae (6) and (7) are selected from the group consisting of the compounds of the following Formulae:

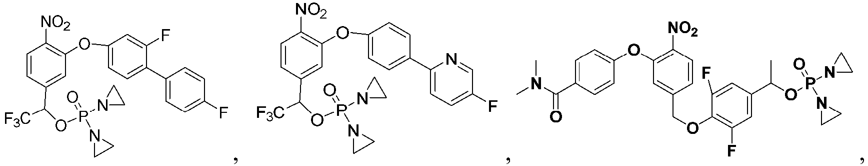

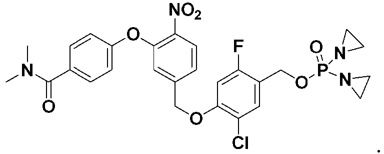

The synthesis and preparation methods of the compounds are also described in Patent Application PCT/CN2020/089692 with publication number WO2020228685A9 (corresponding to the Chinese application No. 2020800358890 with publication number CN113853379A), which is incorporated herein by reference in its entirety.

The compound of Formula (8) is selected from the group consisting of the compounds of the following Formulae:

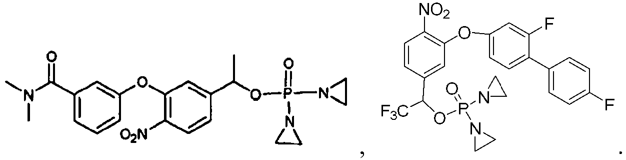

The compound of Formula (9) is selected from the group consisting of the compounds of the following Formulae:

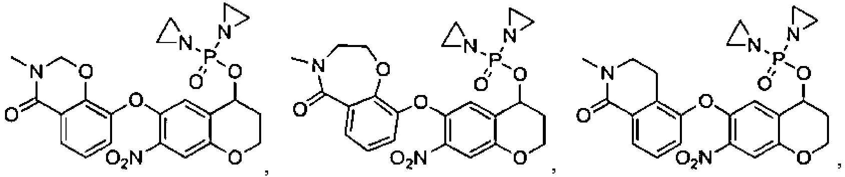

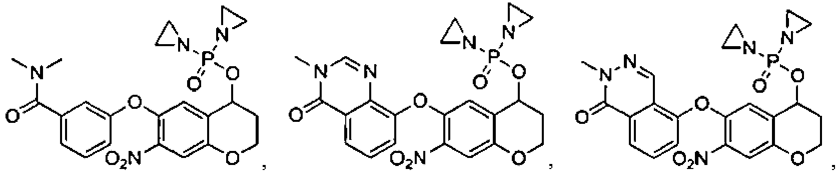

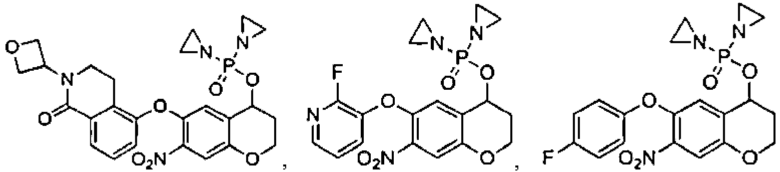

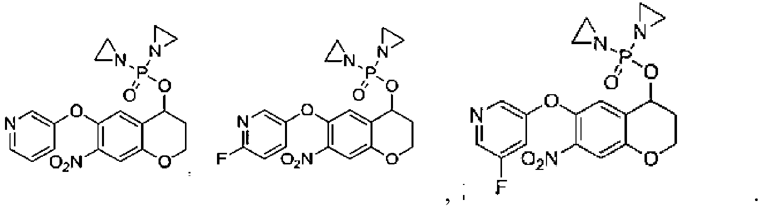

The synthesis and preparation methods of the compounds are also described in Patent Application PCT/CN2020/120281 with publication number WO2021068952A1 (corresponding to the Chinese application No. 202080071652.8 with publication number CN114555574A), which is incorporated herein by reference in its entirety.

The compound of Formula (10) is selected from the group consisting of the compounds of the following Formulae:

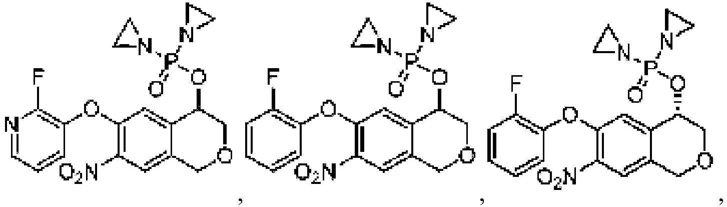

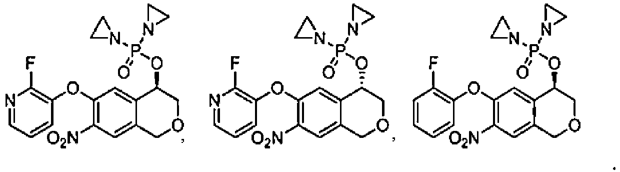

The synthesis and preparation method of the compound is also described in patent application PCT/CN2021/118597 publication number WO2022057838 A1, which is incorporated herein by reference in its entirety.

The compound of Formula (11) is selected from the group consisting of the compounds of the following Formulae.

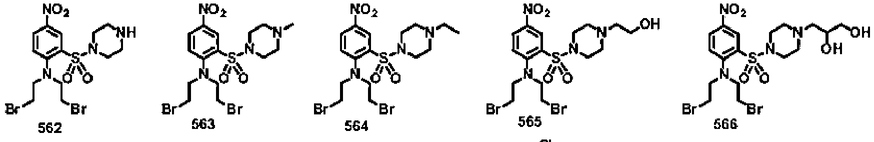

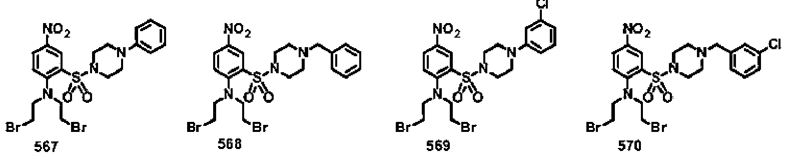

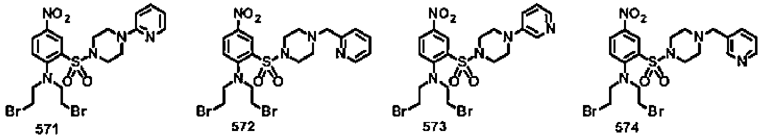

The synthesis and preparation methods of the compounds are also described in Patent Application PCT/NZ2019/050030 with publication number WO2019190331A1 (corresponding to the Chinese application No. 2019800234236 with publication number CN111918864A), which is incorporated herein by reference in its entirety.

The compound of Formula (12) is selected from the group consisting of the compounds of the following Formulae.

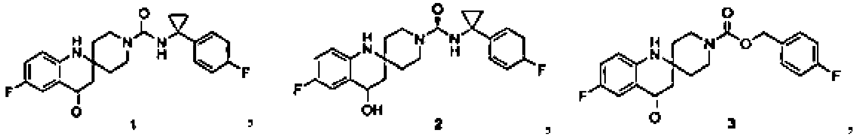

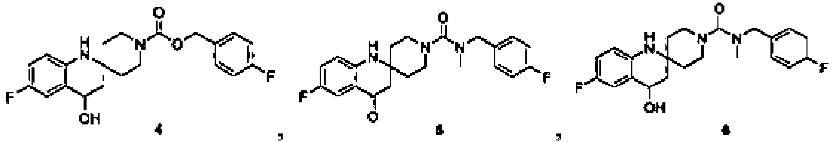

The synthesis and preparation methods of the compounds are also described in patent application CN202210585771.6 with publication number CN115403579A, which is incorporated herein by reference in its entirety.

The compound of Formula (13) is selected from the group consisting of the compounds of the following Formulae:

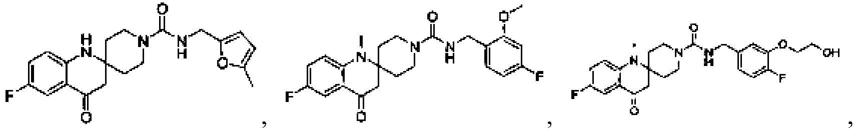

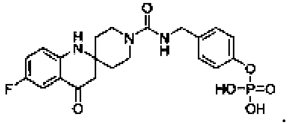

The synthesis and preparation methods of the compounds are also described in Patent Application PCT/IB2020/057285 with publication number WO2021005586A1 (corresponding to the Chinese application No. CN202080053804.1 with publication number CN114206870A), which is incorporated herein by reference in its entirety.

The compound of Formula (14) is selected from the group consisting of the compounds of the following Formulae:

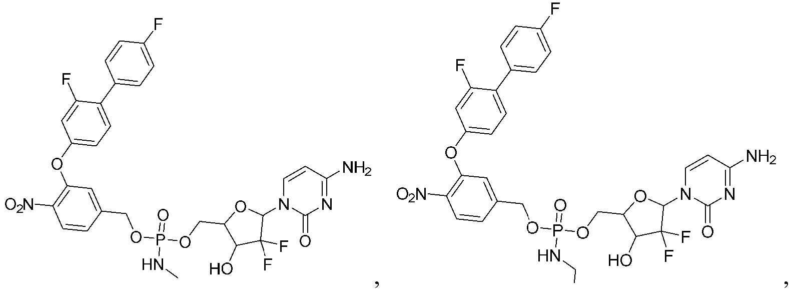

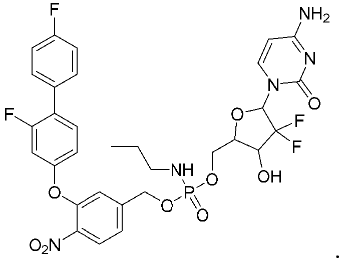

The synthesis and preparation methods of the compounds are also described in patent application PCT/CN2022/098082 with publication number WO2022258043A1, which is incorporated herein by reference in its entirety.

The compound of Formula (15) is selected from the group consisting of the compounds of the following Formulae:

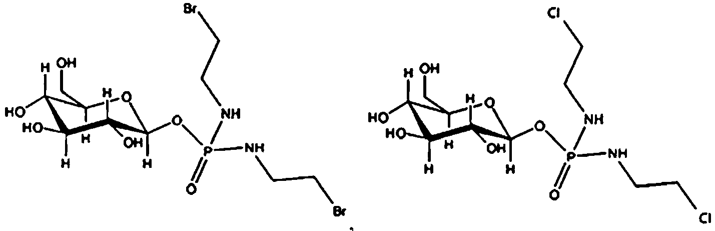

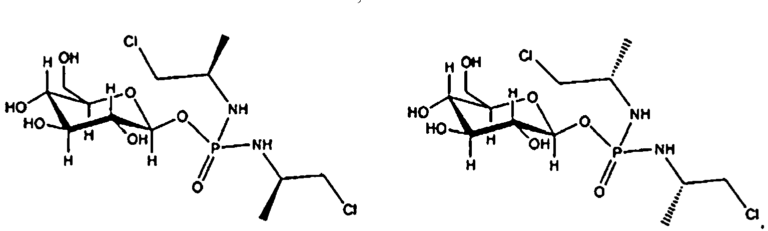

The synthesis and preparation methods of the compounds are also described in U.S. Patent Application No. 5,622,936 A, PCT Application No. PCT/US2007/074012 with publication number WO2008011588, which is incorporated herein in its entirety.

The drug described herein refers to a pharmaceutical product or preparation, the prepared drug comprises a specific dose range of a compound, or a salt, ester, solvate or isotopic isomer thereof, as active ingredient, and/or the prepared drug is administered in a specific dosage form and a specific administration method.

With respect to the compounds described herein, they contain the chemical structure such as an organic amine structure and a P=O double bond structure, and therefore the compounds may also be administered as a salt, i.e. the present invention provides pharmaceutically acceptable salts of the compounds, which can be a basic salt, including salts formed by the compounds and inorganic bases (e.g. alkali metal hydroxides, alkaline earth metal hydroxides, etc.) or formed by the compounds and organic bases (e.g. monoethanolamine, diethanolamine, or triethanolamine, etc.). Alternatively, the salt may be an acid salt, including salts formed by the compounds and inorganic acids (such as hydrochloric acid, hydrobromic acid, hydroiodic acid, nitric acid, perchloric acid, sulfuric acid, or phosphoric acid, and the like), or formed by the compounds and organic acids (such as methanesulfonic acid, trifluoromethanesulfonic acid, ethanesulfonic acid, benzenesulfonic acid, p-toluenesulfonic acid, fumaric acid, oxalic acid, maleic acid, citric acid, and the like). Similarly, the compounds can also react with certain acids or alcohols to form esters, and therefore the compounds may also be administered as esters.

Similarly, the concept of the above compound also comprises various crystal forms of the compound, for example a crystal form of the AKR1C3-activated anti-cancer prodrug compound disclosed in the PCT application PCT/CN2023/080261 with publication number WO2023169462A1:

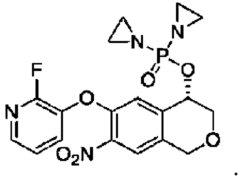

For various reasons, these compounds may also form solvates with certain solvents, which are hydrates or alcoholates, and therefore the compounds may also be administered as solvates. The selection and preparation of acceptable salts, esters, solvates, and the like of the compounds are well-known in the art.

The term "isotopic variant" refers to a compound containing an isotope in a non-natural ratio at one or more positions in the atoms of such compound. In certain embodiments, an "isotopic variant" of a compound contains one or more isotopes in a non-natural ratio, including but not limited to, hydrogen (¹H), deuterium (²H), tritium (³H), carbon-11 (¹¹C), carbon-12 (¹²C), carbon-13 (¹³C), carbon-14 (¹⁴C), nitrogen-13 (¹³N), nitrogen-14 (¹⁴N), nitrogen-15 (¹⁵O), oxygen-15 (¹⁵O), oxygen-16 (¹⁶O), oxygen-17 (¹⁷O), oxygen-18 (¹⁸O), fluorine-17 (¹⁷F), fluorine-18 (¹⁸F), phosphor-31 (³¹P), phosphor-32 (³²P), phosphor-33 (³³P), sulfur-32 (³²S), sulfur-33 (³³S), sulfur-34 (³³S), sulfur-35 (³⁵S), sulfur-36 (³⁶S), chlorine-35(³⁵Cl), chlorine-36(³⁶Cl), chlorine-37(³⁷Cl), bromine-79 (⁷⁹Br), bromine-81 (⁸¹Br), iodine-123 (¹²³I), iodine-125 (¹²⁵I), iodine-127 (¹²⁷I), iodine-129 (¹²⁹I), and iodine-131 (¹³¹I). In certain embodiments, an "isotopic variant" of a compound is in a stable form, i.e., non-radioactive. In certain embodiments, an "isotopic variant" of a compound contains one or more isotopes in a non-natural ratio, including but not limited to, hydrogen (¹H), deuterium (²H), carbon-12 (¹²C), carbon-13 (¹³C), nitrogen-14 (¹⁴N), nitrogen-15 (¹⁵N), oxygen-16 (¹⁶O), oxygen-17 (¹⁷O), oxygen-18 (¹⁸O), fluorine-17 (¹⁷F), phosphorus-31 (³¹P), sulfur-32 (³²S), sulfur-33 (³³S), sulfur-34 (³⁴S), sulfur-36 (³⁶S), chlorine-35 (³⁵Cl), chlorine-37 (³⁷Cl), bromine-79 (⁷⁹Br), bromine-81 (⁸¹Br), and iodine-127 (¹²⁷I). In certain embodiments, an "isotopic variant" of a compound is in an unstable form, i.e., radioactive. In certain embodiments, an "isotopic variant" of a compound contains one or more isotopes in a non-natural ratio, including but not limited to, tritium (³H), carbon-11 (¹¹C), carbon-14 (¹⁴C), nitrogen-13 (¹³N), oxygen-14 (¹⁴O), oxygen-15 (¹⁵O), fluorine-18 (¹⁸F), phosphorus-32 (³²P), phosphorus-33 (³³P), sulfur-35 (³⁵S), chlorine-36 (³⁶Cl), iodine-123 (¹²³I), iodine-125 (¹²⁵I), iodine-129 (¹²⁹I), and iodine-131 (¹³¹I). It is to be understood that when those skilled in the art determine that it is feasible, in the compounds provided herein, any hydrogen can be, for example, ²H (i.e., D), or any carbon can be, for example, ¹³C, or any nitrogen can be, for example, ¹⁵N, and any oxygen can be ¹⁸O. In certain embodiments, an "isotopic variant" of a compound contains deuterium (D) in a non-natural ratio.

In addition to the compounds of Formulae (1)-(15), the above-mentioned medicaments should also be supplemented with pharmaceutically acceptable adjuvants or excipients according to the specific drug, medicament or formulation. The medicament can be in any dosage form for clinical administration, such as tablets, suppositories, dispersible tablets, enteric-coated tablets, chewable tablets, orally disintegrating tablets, capsules, dragees, granules, dry powders, oral solutions, small injections, lyophilized powder injections, or large infusions. Depending on specific dosage form and administration manner, the pharmaceutically acceptable adjuvants or excipients in the medicament may include one or more of the following: diluents, solubilizers, disintegrants, suspending agents, lubricants, binders, fillers, flavoring agents, sweetening agents, antioxidants, surfactants, preservatives, encapsulating agents, and pigments.

"Cancer" refers to leukemias, lymphomas, carcinomas, and other malignant tumors (including solid tumors) or hyperplasia (malignant hyperplasia) characterized by potentially unrestrained growth that can expand locally through invasion and expand systemically by metastasis. Examples of cancer include, but are not limited to, cancer of the adrenal gland, bone, brain, breast, bronchus, colon, and/or rectum, gallbladder, head and neck, kidney, larynx, liver, lung, nervous tissue, pancreas, prostate, parathyroid, skin, stomach, and thyroid. Certain other examples of cancer include acute and chronic lymphocytic and granulocytic neoplasms, adenocarcinoma, adenoma, basal cell carcinoma, cervical dysplasia and carcinoma in situ, Ewing's sarcoma, epidermoid carcinoma, giant cell tumor, glioblastoma multiforme, hairy cell tumor, intestinal ganglioneuroma, hyperplastic corneal nerve tumor, islet cell carcinoma, Kaposi's sarcoma, leiomyoma, leukemia, lymphoma, malignant carcinoid, malignant melanoma, malignant hypercalcemia, Marfanoid habitus tumor, medullary epithelioma, metastatic skin cancer, mucosal neuroma, myeloma, mycosis fungoides, neuroblastoma, osteosarcoma, osteogenic and other sarcomas, ovarian tumor, pheochromocytoma, polycythemia vera, primary brain tumor, small cell lung cancer, squamous cell carcinoma of both ulcerative and papillary types, hyperplasia, seminoma, soft tissue sarcoma, retinoblastoma, rhabdomyosarcoma, renal cell tumor, topical skin lesion, reticulum cell sarcoma, and Wilm's tumor.

For recommended dosage and administration formulation of AST-3424 or such AKR1C3 enzyme-activated DNA alkylating agent prodrugs for treating cancer, reference may be made to the patent applications filed by Threshold Company and companies such as Ascentawits, OBI, Vybio, etc. (such as WO2017087428A1, WO2017087428A1, WO2019062919A1, and WO2021008520A1) and clinical trials registered with FDA or NMPA (CTR20201915, CTR20201908, CTR20191399, CTR20191371, CTR20220957, NCT04315324, and NCT03592264).

The p53 gene, i.e., the Tumor protein p53 gene, is also known as the TP53 gene.

At present, relevant detection kits have been approved for commercial use and can be commercially available for detection, such as the AmoyDx^{®} TP53 Six Mutations Detection Kit produced by Amoy Diagnostics Co., Ltd. (Xiamen, China); the FISH Detection Kit for the p53 gene (fluorescence in situ hybridization) produced by Celnovte Biotechnology Co., Ltd. (Henan, China); and the Archer^{®}VariantPlex^{™} p53 kit for Illumina^{®} produced by Integrated DNA Technologies, Inc. (USA).

The above term "negative for P53 gene mutation" includes cases where no mutation is detected or where the mutation level is below a predetermined value and would not affect gene expression.

The KRAS gene includes G12A, G12C, G12D, G12R, G12S, and G12V. The most common way for activating the KRAS gene is point mutation, with 95% of the KRAS mutations occurring mainly at codon 12 (>80%) and codon 13 of exon 2. Common mutation forms include KRAS-G12C mutation (accounting for 13% of all KRAS mutations), KRAS-G12V mutation (20%) and KRAS-G12D mutation (29%) (Loong HHF, Du N, Cheng C, Lin H,Guo J, Lin G, Li M, Jiang T, Shi Z, Cui Y, Jin X, Yao J, Xing Y, Yao M, Wang K, Mok TSK, Liu L. KRAS G12C mutations in Asia: A landscape analysis of 11,951 Chinese tumor samples. Transl Lung Cancer Res 2020. Doi: 10.21037/tlcr-20-455).

Any one or two gene mutations in the corresponding genes of KRAS (such as G12A, G12C, G12D, G12R, G12S, and G12V) can be detected and diagnosed using commercially available (companion) diagnostic kits, such as the diagnostic kits approved in China:
Xiamen Amoy Dx Human KRAS Gene Mutation Detection Kit (fluorescent PCR method), State instrument registration 20153401126;
Shanghai Tellgen Life Science Human K-RAS Gene Seven Mutations Detection Kit (PCR fluorescence method), State instrument registration 20163401341.

Of course, NGS sequencing (such as YS 450 gene NGS large panel) can also be used to determine the specific KRAS mutation subtype.

More preferably, the KRAS mutation is selected from the KRAS-G12D mutation.

The above term "positive for KRAS gene mutation" means that gene mutation has been detected.

The BRCA gene includes BRCA1 and BRCA2 genes.

The term "positive for BRCA gene mutation" means that mutations occur in any one or two genes of the genes corresponding to BRCA1 and BRCA2, which can be detected using commercially available (companion) diagnostic kits:
Olaparib Companion Detection Kit BRACAnalysisCDx;
BRCA1/2 Gene Mutation Detection Kit (combined probe anchoring polymerization sequencing); and
Human BRCA1 Gene and BRCA2 Gene Mutation Detection Kit (reversible terminal termination sequencing).

BRCA1 and BRCA2 mutations include the BRCA1 and BRCA2 mutations of germline mutations (gBRCAm) and somatic mutations (sBRCAm).

The above BRCA1 and BRCA2 mutations are preferably pathogenic mutations.

The above terms "negative (no mutation is detected or the mutation level is below a predetermined value ) or positive (mutation is detected or the mutation level is higher than a predetermined value) for P53 gene mutation, KRAS gene mutation, or BRCA gene mutation" is determined based on the patient's tumor or cancerous tissue or other biological samples (such as blood or saliva).

Preferably, if the gene mutation status is positive, then preferably the corresponding TMB (Tumor Mutation Load (burden)) level is medium or high.

Since the TMB (Tumor Mutation Load (burden)) levels are different (high or low) among different tumor types: it is generally considered that: a TMB exceeding 20 mutations/Mb (Mb represents per million bases) is high; a TMB below 10 mutations/Mb is low; and a TMB therebetween is medium. At the 2017 World Conference on Lung Cancer, Squibb Inc. published the results of a clinical trial named CheckMate-032. This is a Phase II clinical trial that enrolled 401 patients with advanced lung cancer who had failed first-line therapy and were treated with a PD-1 inhibitor alone or in combination with Ipilimumab. According to TMB levels, the patients were divided into three categories of patients: high TMB, medium TMB, and low TMB, and among those who received the combination therapy, the effective rates of the three groups were respectively 62%, 20%, and 23%, i.e., the effective rate of the high TMB group was three times higher than those of the two groups; and the median overall survival of the three groups was respectively 22.0 months, 3.6 months, and 3.4 months, i.e., a 6-fold difference (22.0 months *vs.* 3.4 months). This trial demonstrates that for different anticancer drugs, different TMB levels have a significant impact on the efficacy of the drugs.

The treatment in the present application includes monotherapy and combination therapy with other drugs.

Single drug refers to monotherapy. Drug combination refers to combination therapy. Monotherapy refers to use of only one anticancer drug in a course of treatment. Combination therapy refers to simultaneous or sequential use of two or more anticancer drugs in a course of treatment.

Generally, for combination therapy, combination therapy requires exploration of different administration dosages and administration cycles according to the characteristics of diseases and the types of drugs to be used in combination. Only according to the above conditions, the therapeutic regimen of combination therapy could realize better therapeutic effect that that of monotherapy.

The drug administration dosages and administration cycles in the therapeutic regimen of both monotherapy and combination therapy need to be explored through clinical trials with reference to the dosage and administration regimen of AST-3424 and analogues thereof and combined drugs as described above.

Examples of AST-3424 in combination with other drugs have been described in the following references:
For the combination of AST-3424, abiraterone acetate/abiraterone and prednisolone, or sunitinib, or gemcitabine, please refer to the patent application PCT/CN2021/078115 with publication number WO2022178821 and the academic literature (Meng, Fanying et al. "A novel selective AKR1C3-activated prodrug AST-3424/OBI-3424 exhibits broad anti-tumor activity." American journal of cancer research vol. 11, 7 3645-3659. 15 Jul. 2021). Therein, the combination of AST-3424, abiraterone acetate/abiraterone and prednisolone is a triple therapy of using three drugs, that is, on the basis of the existing combination of abiraterone acetate/abiraterone and prednisolone, the third drug AST-3424 is used for the combination treatment of three drugs.

For the combination of AST-3424 and 5-fluorouracil, please refer to the patent application PCT/CN2021/078115 with publication number WO2022178821 and the academic literature (Meng, Fanying et al. "A novel selective AKR1C3-activated product AST-3424/OBI-3424 exhibits broad anti-tumor activity." American journal of cancer research vol. 11, 7 3645-3659.15 Jul. 2021) and the academic literature (Zhang, Yu et al. "The In-Vitro Antitumor Effects of AST-3424 Monotherapy and Combination Therapy with Oxaliplatin or 5-Fluorouracil in Primary Liver Cancer." Frontiers in oncology vol. 12 885139. 22 Jul. 2022, doi: 10.3389/fonc.2022. 885139).

For the combination of AST-3424 and oxaliplatin, please refer to the academic literature (Zhang, Yu et al. "The In-Vitro Antitumor Effects of AST-3424 Monotherapy and Combination Therapy with Oxaliplatin or 5-Fluorouracil in Primary Liver Cancer." Frontiers in oncology vol. 12 885139. 22 Jul. 2022, doi: 10.3389/fonc.2022. 885139).

For the combination of AST-3424 and PD-1/L1 inhibitors, please refer to the patent application PCT/US2021/29552 with publication number WO2022231580, and the academic conference poster (Chun-Chung Wang, Wan-Fen Li, Chih-Chan Lee, Lu-Tzu Chen, Jhih-Jie Yang, Jiann-Shiun Lai, Ming-Tain Lai; Abstract 6111: OBI-3424, an AKR1C3-activated prodrug, exhibits in vivo synergistic anti-tumor effect in combination with pembrolizumab by induction of immunogenic cell death. Cancer Res 15 June 2022; 82 (12 _ Supplement): 6111. https://doi.org/10.1158/1538-7445.AM2022-6111. The poster can be downloaded from the official website of OBI Pharma Inc at https://www.obipharma.com/zh-hant/news-zh-hant/news-2022-zh-hant/poster-presentations-at-aacr-2022-annual-meeting-for-obi-3424-and-globo-h-science//).

For the combination of AST-3424 and apatinib, sorafenib/donafenib, or elemene, please refer to the academic literature (Xun, Chen et al. "A novel AKR1C3 specific prodrug AST-3424 and its combination therapy in hepatocellular carcinoma." Journal of pharmacological sciences vol. 152, 2 (2023): 69-75. doi: 10.1016/j.jphs.2023. 03.004). Donafenib is a deuterated drug of sorafenib, and the combination threapy of AST-3424 and sorafenib has been described in the above references; thus, those skilled in the art can readily deduce that AST-3424 can also be used in combination with its deuterated drug donafenib.

Preferably, other drugs used in combination therapy include immune checkpoint inhibitors, CDK inhibitors, ATR inhibitors, CHK inhibitors, and WEE1 inhibitors.

Immune checkpoint molecules are regulatory molecules that play an inhibitory role in immune system, and are essential for maintaining self-tolerance, preventing autoimmune reactions, and minimizing tissue damage by controlling time and intensity of immune response. Immune checkpoint molecules are expressed on immune cells and would inhibit the function of immune cells, thereby preventing the body from generating an effective anti-tumor immune response and causing immune escape of tumors. Tumor-related immune checkpoint molecules mainly include PD1, PD-L1, CTLA4, Tim3, and LAG3, etc. Currently, PD1, PD-L1, and CTLA4 are most extensively studied. Immune checkpoint inhibitors are some monoclonal antibody drugs developed for corresponding immune checkpoints. Their main function is to block the interaction between tumor cells expressing immune checkpoints and immune cells, thereby blocking the inhibitory effect of tumor cells on immune cells. Immunotherapy drugs are selected from the group consisting of PD-1 monoclonal antibodies and PD-L1 monoclonal antibodies.

CDK inhibitors are selected from the group consisting of Palbociclib, Ribociclib, Abemaciclib, Trilaciclib, Dalpiciclib, Adavosertib, Ro-3306, Dinaciclib, Cirtuvivint, Rintodestrant, DS96432529, THZ1, THZ531, Seliciclib, Flavopiridol, AZD4573, SR-4835, Simurosertib, Fadraciclib, NVP-2, SNS-032, (E/Z)-Zotiraciclib, AZD-5438, AT7519, Mevociclib, Kenpaullone, YKL-5-124 TFA, NG 52, GSK 3 Inhibitor IX, OTS964, Samuraciclib, Flavopiridol, KB-0742 dihydrochloride, (+)-Enitociclib, AUZ 454, SY-5609, SEL120-34A monohydrochloride, CCT-251921, MBQ-167, XL413 hydrochloride, BI-1347, THAL-SNS-032, JNJ-7706621, TG003, LDC4297, BMS-265246, Roniciclib, CGP60474, (R)-CR8 trihydrochloride, R547, Milciclib, T025, AS2863619, Senexin A, BSJ-4-116, CLK-IN-T3, CDK12-IN-3, CVT-313, Atuveciclib, PHA-793887, Indirubin-3'-monoxime, YKL-5-124, PHA-767491 hydrochloride, KH-CB19, Cucurbitacin E, Purvalanol A, BSJ-03-204, ON123300, CDK5 inhibitor 20-223, Riviciclib, FN-1501, CP-10, THZ2, Abemaciclib metabolite M2, BS-181, CDK12-IN-E9, Samuraciclib, RGB-286638, CDK2-IN-4, LDC000067, ML167, Purvalanol B, NU6300, CLK1-IN-1, FMF-04-159-2, CKI-7, CDKI-73, MSC2530818, BSJ-04-132, NU6102, Voruciclib, Olomoucine, and the like. The list of these compounds, and their specific structures and supply information can be obtained by accessing commercial reagent websites, such as the website of MedChemExpress (MCE): https://www.medchemexpress.cn/Targets/CDK.html?effectName=Inhibitor.

Preferably, the WEE1 inhibitors are selected from the group consisting of WEE1-IN-5, WEE1-IN-3, WEE1-IN-4, LEB-03-146, LEB-03-144, Adavosertib, LEB-03-153, LEB-03-145, PD407824, PD0166285, PD0166285 dihydrochloride, Pomalidomide-C3-adavosertib, DB0614, FMF-06-098-1, and the like. The list of these compounds, and their specific structures and supply information can be obtained by accessing commercial reagent websites, such as the website of MedChemExpress (MCE): https://www.medchemexpress.cn/search.html?q=Wee&ft=&fa=&fp=&fsp=&ftag=&fs c=.

Preferably, the CHK inhibitors are selected from the group consisting of AZD7762, Prexasertib, SCH900776, GDC-0425, Chk1-IN-6, CCT245737, BML-277, CCT241533, PD407824, CHIR-124, CCT244747, PF477736, GDC-0575, SB-218078, MRT00033659, ANI-7, SAR-020106, CCT241533, CHK1-IN-4, VER-00158411, CHK1-IN-3, Chk1-IN-5, CHK1-IN-2, CHK-IN-1, and the like. The list of these compounds, and their specific structures and supply information can be obtained by accessing commercial reagent websites, such as the website of MedChemExpress (MCE):
https://www.medchemexpress.cn/Targets/Checkpoint%20Kinase%20(Chk).html.

Preferably, the ATR inhibitors are selected from the group consisting of Ceralasertib, Berzosertib, Gartisertib, BAY1895344, BAY-937, AZ20, ETP-46464, Dactolisib, VE-821, M1774, ATRN-199, RP-3500, and ART-0380. Information of these compounds can be found in the literature: YUAN Yinghui, DUAN Jilong, HUI Zi, et al. Research progress of ATR kinase-targeted inhibitors in the cancer therapy [J]. Acta Pharmaceutica Sinica, 2022(057-003).

Preferably, prior to administration of these drugs containing the AKR1C3 enzyme-activated anti-cancer prodrug compounds, the AKR1C3 expression level should be detected.

Evidently, the applicant noted that the patients carrying KRAS mutations generally have high AKR1C3 enzyme expression. Therefore, for some patients carrying KRAS gene mutations, it may not be necessary to perform the detection of AKR1C3 expression level prior to use of the drugs containing the AKR1C3 enzyme-activated anti-cancer prodrug compounds. This is because carrying a KRAS mutation implies that the patient has high AKR1C3 expression level and may benefit from the AKR1C3 enzyme-activated anti-cancer prodrugs.

Therefore, using a drug containing an AKR1C3 enzyme-activated anti-cancer prodrug compound, or a salt, ester, solvate or isotopic isomer thereof, alone or in combination with other drugs, for treating a cancer patient negative for P53 gene mutation and positive for KRAS gene mutation / treating a cancer patient positive for both KRAS gene mutation and BRCA gene mutation, does not necessarily involve detection of AKR1C3 expression level; and these features, such as negative for P53 gene mutation or positive for BRCA gene mutation, could further enable the patients to obtain potential better therapeutic effects.

Taking the AST-3424 (OBI-3424) developed by the applicant as an example, when it is used for treating liver cancer, the patient with a strongly positive AKR1C3 expression in liver tumor tissue may benefit from the administration of the drug.

The liver tumor tissue of the patient is determined to be strongly positive for AKR1C3 expression if one of the following conditions is met:
1. the H score is greater than or equal to 100, preferably greater than or equal to 135, as detected by the AKR1C3 detection method described in WO2022048492; and
2. the proportion of tumor cells having a staining intensity of 2+ and/or 3+ is ≥ 70%, as detected by the AKR1C3 detection method described in WO2022048492.

Criterion 1 is determined according to the OBI-3424 Phase I clinical trial NCT03592264 conducted in the United States, and reference may be made to the academic literature (Journal of Clinical Oncology. 40.3030-3030.10.1200/JCO.2022. 40.16_suppl.3030) or the academic literature (Tsimberidou, A.M., Verschraegen, C.F., Wesolowski, R. et al. Phase 1 dose-escalation study evaluating the safety, pharmacokinetics, and clinical activity of OBI-3424 in patients with advanced or metastatic solid tumors. Br J Cancer 129, 266-274 (2023). https://doi.org/10. 1038/s41416-023-02280-4).

Criterion 2 is determined according to the AST-3424 Phase I clinical trial CTR20191371 conducted in China, i.e., currently, the Phase II clinical trial is conducted according to this criterion. For the enrollment criteria of the Phase II clinical trial, please refer to CTR20191399.

In the text described in WO2022048492, the score calculation method of the H-scoring system is as follows: 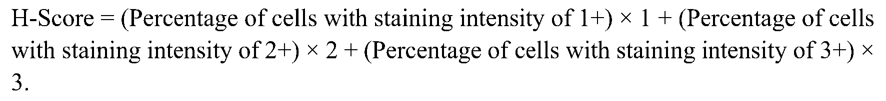 H-Score = (Percentage of cells with staining intensity of 1+) × 1 + (Percentage of cells with staining intensity of 2+) × 2 + (Percentage of cells with staining intensity of 3+) × 3.

If the IHC staining result of a certain liver cancer tissue is that the percentage of the cells with staining intensity of 1 is 10%, the percentage of the cells with staining intensity of 2 is 20%, and the percentage of the cells with staining intensity of 3 is 50%, then the H-score is 10 + 40 +150 = 200.

### BRIEF DESCRIPTION OF THE DRAWINGS

Figure 1 is a graph showing the correlation between AKR1C3 RNA expression level and NRF2 RNA expression level in a KRAS-G12D mutation model;
Figure 2 is a graph showing the correlation between AKR1C3 RNA expression level and NRF2 RNA expression level in a KRAS-G12C mutation model;
Figure 3 is a graph showing the correlation between AKR1C3 RNA expression level and NRF2 RNA expression level in a KRAS-G13D mutation model;
Figure 4 shows the results of Western Blotting experiments of target proteins after cell lysis under the treatment with different concentrations of SFN, wherein the left panel is the Western Blotting image under the treatment with different concentrations of SFN, and the right panel is a bar chart of density analysis of NRF2 and AKR1C3 protein bands, and wherein in each concentration group, the left bar represents NRF2 and the right bar represents AKR1C3;
Figure 5 shows the inhibition rate curves of the *in vitro* proliferation of H460 cells treated with the combination of AST-3424/AST and Nutlin-3;
Figure 6 shows the inhibition rate experimental results of the *in vitro* proliferation of H460 cells treated with AST-3424 alone, Nutlin-3 alone and the combination of AST-3424 and Nutlin-3 with different administration sequences;
Figure 7 shows the experimental results of the effects of AST-3424 alone, Nutlin-3 alone, and the combination of AST-3424 and Nutlin-3 with different administration sequences on colony formation of H460/HPAF-II cells;
Figure 8 shows the experimental results of the effects of AST-3424 alone, Nutlin-3 alone and the combination of AST-3424 and Nutlin-3 with different doses on the apoptosis process of H460 cells;
Figure 9 shows the first experimental result of the effects of the combination of AST-3424 and Nutlin-3 on the cell cycle G2/M arrest of H460/HPAF-II cells. The bar chart respectively shows G2/M, S, and G0/G1 phases from top to bottom;
Figure 10 shows the second experimental result of the effects of the combination of AST-3424 and Nutlin-3 on the cell cycle G2/M arrest. The bar chart respectively shows G2/M, S, and G0/G1 phases from top to bottom;
Figure 11 is a bar chart showing the inhibition results of the *in vitro* proliferation of H460 cells treated with AST alone, Nutlin-3 alone and the combination of AST and Nutlin-3. The three bars from left to right in the chart respectively indicate the inhibition rate values of AST alone, Nutlin-3 alone and the combination of AST and Nutlin-3;
Figure 12 is a bar chart showing the inhibition results of the *in vitro* proliferation of H460 cells treated with AST alone, RITA alone and the combination of AST and Nutlin-3. The three bars from left to right in the chart respectively indicate the inhibition rate values of AST alone, RITA alone and the combination of AST and RITA;
Figure 13 is a bar chart showing the inhibition results of the *in vitro* proliferation of HPAF-II cells treated with AST alone, Nutlin-3 alone and the combination of AST and Nutlin-3. The three bars from left to right in each group in the figure respectively indicate the inhibition rate values of AST alone, Nutlin-3 alone and the combination of AST and Nutlin-3. The six groups of bar charts from left to right respectively correspond to the experimental groups 1-6 in the table;
Figure 14 is a bar chart showing the inhibition results of the *in vitro* proliferation of HPAF-II cells treated with AST alone, RITA alone and the combination of AST and RITA. The three bars from left to right in the chart respectively indicate the inhibition rate values of AST alone, RITA alone and the combination of AST and RITA;
Figure 15 shows the WB detection results of the cell protein lysate in the first experiment of treating H460 cells with AST-3424 alone, Nutlin-3 alone and the combination of AST-3424 and Nutlin-3. In this figure, the upper panel is the image of the protein bands in the WB detection, and the lower panel shows the ratios of the corresponding proteins to the internal reference protein β-actin;
Figure 16 shows the WB detection results of the proteins in the cell lysate in the second experiment of treating H460 cells with AST-3424 alone, Nutlin-3 alone and the combination of AST-3424 and Nutlin-3. In this figure, the upper panel is the image of the protein bands in the WB detection, and the middle and the lower panels show the ratios of the corresponding proteins to the internal reference protein β-actin;
Figure 17 shows the WB detection results of the proteins in the cell lysate in the third experiment of treating H460 cells with AST-3424 alone, Nutlin-3 alone and the combination of AST-3424 and Nutlin-3. In this figure, the upper panel is the image of the protein bands in the WB detection, and the middle and the lower panels show the ratios of the corresponding proteins to the internal reference protein β-actin;
Figure 18 shows the WB detection results of the RAD51 protein in the cell lysate in the first experiment of treating H460 cells with AST-3424 alone, Nutlin-3 alone and the combination of AST-3424 and Nutlin-3. In this figure, the left panel is the image of the protein bands in the WB detection, and the right panel shows the ratios of the corresponding proteins to the internal reference protein β-actin;
Figure 19 shows the WB detection results of the RAD51 protein in the cell lysate in the second experiment of treating H460 cells with AST-3424 alone, Nutlin-3 alone and the combination of AST-3424 and Nutlin-3. In this figure, the upper panel is the image of the protein bands in the WB detection, and the lower panel shows the ratios of the corresponding proteins to the internal reference protein β-actin;
Figure 20 shows the WB detection results of the RAD51 protein in the cell lysate in the third experiment of treating H460 cells with AST-3424 alone, Nutlin-3 alone and the combination of AST-3424 and Nutlin-3. In this figure, the upper panel is the image of the protein bands in the WB detection, and the lower panel shows the ratios of the corresponding proteins to the internal reference protein β-actin;
Figure 21 shows the WB detection results of the RAD51 protein in the cell lysate in the experiment of treating H460 cells with the combination of AST-3424, Nutlin-3 and MG-132. In this figure, the upper panel is the image of the protein bands in the WB detection, and the lower panel shows the ratios of the corresponding proteins to the internal reference protein β-actin;
Figure 22 shows the WB detection results of the RAD51 protein in the cell lysate in the experiment of treating H460 cells with the combination of AST-3424, Nutlin-3 and Cycloheximide. In this figure, the upper panel is the image of the protein bands in the WB detection, and the lower panel shows the ratios of the corresponding proteins to the internal reference protein β-actin;
Figure 23 shows the curves of changes in the relative levels of RAD51 at different time points after addition of 4 µM Cycloheximide, following 24-hour treatment with different drugs: 1% DMSO, 0.1 nM AST-3424, 5 µM Nutlin-3, 0.1 nM AST-3424 + 5 µM Nutlin-3;
Figure 24 shows the WB detection results of the γH2AX protein in the cell lysate in the first experiment of treating H460 cells with AST-3424 alone, Nutlin-3 alone and the combination of AST-3424 and Nutlin-3. In this figure, the upper panel is the image of the protein bands in the WB detection, and the lower panel shows the ratios of the corresponding proteins to the internal reference protein β-actin;
Figure 25 shows the WB detection results of the γH2AX protein in the cell lysate in the second experiment of treating H460 cells with AST-3424 alone, Nutlin-3 alone and the combination of AST-3424 and Nutlin-3. In this figure, the upper panel is the image of the protein bands in the WB detection, and the lower panel shows the ratios of the corresponding proteins to the internal reference protein β-actin;
Figure 26 shows the WB detection results of the γH2AX protein in the cell lysate in the third experiment of treating H460 cells with AST-3424 alone, Nutlin-3 alone and the combination of AST-3424 and Nutlin-3. In this figure, the upper panel is the image of the protein bands in the WB detection, and the lower panel shows the ratios of the corresponding proteins to the internal reference protein β-actin;
Figure 27 shows the WB detection results of the p53, Rad51, MDM2, and p21 proteins in the cell lysate in the experiment of treating HPAF-II cells with AST alone, Nutlin-3 alone and the combination of AST and Nutlin-3. In this figure, the upper panel is the image of the protein bands in the WB detection, and the lower panel is bar charts showing the ratios of the corresponding proteins to the internal reference protein β-actin;
Figure 28 shows the WB detection results of the cell protein lysate in the experiment of treating HPAF-II cells with AST-3424 alone, Nutlin-3 alone and the combination of AST-3424 and Nutlin-3. In this figure, the upper panel is the image of the protein bands in the WB detection, and the lower panel is bar charts showing the ratios of the corresponding proteins to the internal reference protein β-actin;
Figure 29 shows the curves of the inhibition rates of the *in vitro* proliferation of H460, H460 P53 KO #1, H460 P53 KO #7, and H460 P53 KO #12 cells treated with AST-3424;
Figure 30 shows the curves of the inhibition rates of the *in vitro* proliferation of H460, H460 P53 KO #1, H460 P53 KO #7, and H460 P53 KO #12 cells treated with AST;
Figure 31 shows the curves of the inhibition rates of the *in vitro* proliferation of H460, H460 P53 KO #1, H460 P53 KO #7, and H460 P53 KO #12 cells treated with compound A;
Figure 32 shows the curves of the inhibition rates of the *in vitro* proliferation of H460, H460 P53 KO #1, H460 P53 KO #7, and H460 P53 KO #12 cells treated with compound B;
Figure 33 shows the images of the protein bands in the WB detection showing the effects of ±Nutlin-3 on Total P53, MDM2, P21, AKR1C3, and Actin proteins in H460 and H460 P53 KO cells;
Figure 34 shows the ratios of the corresponding protein to the internal reference protein β-actin in the WB detection showing the effects of ±Nutlin-3 on Total P53, MDM2, P21, and AKR1C3 proteins in H460 and H460 P53 KO cells;
Figure 35 shows the curves of the inhibition rates of the *in vitro* proliferation of NCI-H460 cells treated with compound C and AST-3424 under normoxia conditions;
Figure 36 shows the curves of the inhibition rates of the *in vitro* proliferation of NCI-H460 P53KO #1 cells treated with compound C and AST-3424 under normoxia conditions.

### DETAILED DESCRIPTION OF THE INVENTION

The present invention will be described below with the reference to specific Examples. Those skilled in the art will understand that these Examples are only used to illustrate the present invention and do not limit the scope of the present invention in any way.

The experimental methods in the following Examples are all conventional methods unless specified otherwise. The pharmaceutical raw materials, reagent materials and the like used are all commercially available products unless otherwise specified.

The terms "Patient" and "individual" can be used interchangeably and refer to a mammal in need of cancer treatment. Typically, a patient is a human. Typically, a patient is a human diagnosed with cancer. In certain embodiments, a "patient" or "individual" may refer to a non-human mammal used for screening, characterizing, and evaluating drugs and therapies, such as a non-human primate, a dog, a cat, a rabbit, a pig, a mouse, or a rat.

"Treatment" or "treatment of patients" is to administer, use or apply a therapeutically effective amount of a medicament in relation to the present invention to patients.
"administering", "applying" or "using" a medicament to a patient refers to direct administration or direct application (which may be administered or applied to a patient by a medical professional or self-administration or self-application, and/or indirect administration or indirect application (which may be the act of prescribing a medicament). For example, a physician who instructs a patient to self-administer a medicament or self-apply a drug and/or provides a prescription for a medicament to a patient is administering or applying the medicament to the patient.

A "therapeutically effective amount" of a drug refers to an amount of a drug that, when administered or applied to a patient with cancer, will have the intended therapeutic effect (e.g., alleviation, amelioration, relief, or elimination of one or more clinical manifestations of a cancer in the patient). The therapeutic effect does not necessarily occur by administering or applying one dose, and may occur only after administering or applying a series of doses. Thus, a therapeutically effective amount may be administered or applied in one or more times.

"Treatment of" a condition or patients refers to taking steps to obtain a beneficial or desired result (including clinical results). For purposes of the present invention, beneficial or desired clinical results include, but are not limited to, alleviation or amelioration of one or more symptoms of a cancer; diminishment of the extent of a disease; delay or slowing of disease progression; improvement, relief, or stabilization of disease status; or other beneficial results. In some cases, treatment of a cancer may result in a partial response or stable disease.

The term "tumor cell" refers to a tumor cell of any suitable species like a mammal, such as a murine, dog, cat, horse, or human.

The above description of the specific embodiments of the present application does not limit the present invention. Those skilled in the art can make various modifications and changes according to the present invention without departing from the spirit of the invention, which should be covered by the scope of the claims appended to the present application.

### Example 1

The following specific experiments of this application are provided to illustrate the effects of BRCA pathogenic mutations on the *in vitro* pharmacodynamics of AST-3424, AST-3424 analogous compound S, and TH-302.

For the contents of the specific experiments, please refer to Example 1 and the corresponding figures in the patent application PCT/CN2023/081542 with publication number WO2023174319A (METHOD FOR TREATING PATIENT WITH BRCA-MUTATED CANCER).

### Example 2

The following animal *in vivo* experiments illustrate the effects of BRCA pathogenic mutations on the *in vivo* pharmacodynamics of AST-3424, AST-3424 analogous compound S and TH-302 .

For the contents of the specific experiments, please refer to Example 2 and the corresponding figures in the patent application PCT/CN2023/081542 with publication number WO2023174319A (METHOD FOR TREATING PATIENT WITH BRCA-MUTATED CANCER).

The above Examples 1 and 2 both directly cite the data in the applicant's previous patent application. The above patent application PCT/CN2023/081542 with publication number WO2023174319A is incorporated herein by reference in its entirety. The analogous compound S of AST-3424 is the compound AST of the present application.

As can be known from Examples 1 and 2, the results of the *in vitro* and *in vivo* pharmacodynamic experiments of AST-3424, Compound S and TH-302 show that the cell lines and mouse tumor models of BRCA pathogenic mutations are more sensitive to the above three compounds, suggesting that in development of future pre-clinical pharmacodynamic experiments and clinical experiments, BRCA pathogenic mutations can be used as a target to screen animal models and clinical patients that are more sensitive to AST-3424, Compound S, and TH-302.

### Example 3

The following specific experiments of this application are provided to illustrate the effects of the KRAS-G12D mutation on the therapeutic effect of AST-3424 and Compound AST in an animal gastric cancer model.

For the specific experiments, please refer to Example 1: Pharmacodynamics Evaluation of the Test Substances AST, AST-3424 and Ifosfamide in the HuPrime^{®} Gastric Cancer GA6201 Subcutaneous Xenograft Model (a model of KRAS pathogenic mutation having G12D amino acid mutation, i.e., positive for KRAS-G12D mutation) and the corresponding figures in the patent application PCT/CN2022/120817 with publication number WO2023046060A1 (TREATMENT OF CANCER PATIENTS HAVING KRAS MUTATIONS).

### Example 4

The following specific experiments of this application are provided to illustrate the effects of the KRAS-G12D mutation on the therapeutic effect of Compound AST in an animal pancreatic cancer model.

For the specific experiments, please refer to Example 2: Pharmacodynamics and Safety Evaluation of the Test Substances AST and Gemcitabine in the HuPrime^{®} Pancreatic Cancer PA1222 Subcutaneous Xenograft Model (a model of KRAS pathogenic mutation having G12D amino acid mutation, i.e., positive for KRAS-G12D mutation) and the corresponding figures in the patent application PCT/CN2022/120817 with publication number WO2023046060A1 (TREATMENT OF CANCER PATIENTS HAVING KRAS MUTATIONS).

### Example 5

The following specific experiments of this application are provided to illustrate the effects of the KRAS-G12C mutation on the therapeutic effect of Compound AST in an animal lung cancer model.

For the specific experiments, please refer to Example 3: Pharmacodynamics and Safety Evaluation of the Test Substances AST and Cisplatin in the HuPrime^{®} Lung Cancer LU11693 Subcutaneous Xenograft Model (a model of KRAS pathogenic mutation having G12C amino acid mutation, i.e., positive for KRAS-G12C mutation) and the corresponding figures in the patent application PCT/CN2022/120817 with publication number WO2023046060A1 (TREATMENT OF CANCER PATIENTS HAVING KRAS MUTATIONS).

### Example 6

The following specific experiments of this application are provided to illustrate the effects of the KRAS-G12D mutation on the therapeutic effect of Compound AST and AST-3424 in an animal pancreatic cancer model.

For the specific experiments, please refer to Example 4: Anti-Tumor Effect and Safety Evaluation of the Test Substances AST, AST-3424 and Ifosfamide in Human-Derived Pancreatic Cancer HPAF-II Subcutaneous Xenograft Model (a model of KRAS pathogenic mutation having G12D amino acid mutation, i.e., positive for KRAS-G12D mutation) and the corresponding figures in the patent application PCT/CN2022/120817 with publication number WO2023046060A1 (TREATMENT OF CANCER PATIENTS HAVING KRAS MUTATIONS).

### Example 7

The following specific experiments of this application are provided to illustrate the effects of the KRAS-G12D mutation on the therapeutic effect of Compound AST and AST-3424 in an animal lung cancer model.

For the specific experiments, please refer to Example 5: Anti-Tumor Effect and Safety Evaluation of the Test Substances AST, AST-3424 and Ifosfamide Monotherapy in the HuPrime^{®} Lung Cancer LU5161 Subcutaneous Model (a model of KRAS pathogenic mutation having G12D amino acid mutation, i.e., positive for KRAS-G12D mutation) and the corresponding figures in the patent application PCT/CN2022/120817 with publication number WO2023046060A1 (TREATMENT OF CANCER PATIENTS HAVING KRAS MUTATIONS).

### Example 8

The following specific experiments of this application are provided to illustrate the effects of the KRAS-G12D mutation on the therapeutic effect of Compound AST in an animal intestinal cancer model.

For the specific experiments, please refer to Example 6: Anti-Tumor Effect and Safety Evaluation of the Test Substances AST and Ifosfamide Monotherapy in the HuPrime^{®} Intestinal Cancer CR3820 Subcutaneous Model (a model of KRAS pathogenic mutation having G12D amino acid mutation, i.e., positive for KRAS-G12D mutation) and the corresponding figures in the patent application PCT/CN2022/120817 with publication number WO2023046060A1 (TREATMENT OF CANCER PATIENTS HAVING KRAS MUTATIONS).

### Example 9

The following specific experiments of this application are provided to illustrate the effects of the KRAS-G12D mutation on the therapeutic effect of Compound AST and AST-3424 in an animal pancreatic cancer model.

For the specific experiments, please refer to Example 7: Anti-Tumor Effect and Safety Evaluation of the Test Substances AST, AST-3424 and Ifosfamide Monotherapy in the HuPrime^{®} Pancreatic Cancer PA2637 Subcutaneous Model (a model of KRAS pathogenic mutation having G12D amino acid mutation, i.e., positive for KRAS-G12D mutation) and the corresponding figures in the patent application PCT/CN2022/120817 with publication number WO2023046060A1 (TREATMENT OF CANCER PATIENTS HAVING KRAS MUTATIONS).

### Example 10

The following specific experiments of this application are provided to illustrate the effects of the KRAS-G12C mutation on the therapeutic effect of Compound AST in an animal lung cancer model.

For the specific experiments, please refer to Example 8: Anti-Tumor Effect and Safety Evaluation of the Test Substances AST and Ifosfamide Monotherapy in the HuPrime^{®} Lung Cancer LU11873 Subcutaneous Model (a model of KRAS pathogenic mutation having G12C amino acid mutation, i.e., positive for KRAS-G12C mutation) and the corresponding figures in the patent application PCT/CN2022/120817 with publication number WO2023046060A1 (TREATMENT OF CANCER PATIENTS HAVING KRAS MUTATIONS).

### Example 11

The following specific experiments of this application are provided to illustrate the effects of the KRAS-G12C mutation on the therapeutic effect of Compound AST in an animal pancreatic cancer model.

For specific experiments, please refer to Example 9: Antitumor Effect and Safety Evaluation of Test Substances AST and Ifosfamide Monotherapy in the HuPrime^{®} Pancreatic Cancer PA1383 Subcutaneous Model (a model of KRAS pathogenic mutation having G12C amino acid mutation i.e., positive for KRAS-G12C mutation) and the corresponding figures in the patent application PCT/CN2022/120817 with publication number WO2023046060A1 (TREATMENT OF CANCER PATIENTS HAVING KRAS MUTATIONS).

The above Examples 3-11 directly cite the data in in the applicant's previous patent application. The above patent application PCT/CN2022/120817 with publication number WO2023046060A1 is incorporated herein by reference in its entirety.

As can be seen from Examples 3 to 11, the results of the *in vitro* and *in vivo* pharmacodynamic experiment of AST-3424 and Compound AST show that the cell lines and mouse tumor models of KRAS pathogenic mutations are more sensitive to the aforementioned compounds, suggesting that in development of future pre-clinical pharmacodynamic experiments and clinical experiments, KRAS pathogenic mutations can be used as a target to screen animal models and clinical patients that are more sensitive to AST-3424 and Compound AST.

### Example 12 Distribution of AKR1C3 and analysis of its correlation with NRF2 in the KRAS G12D PDXs

The data of a total of 179 models with the KRAS-G12D mutation were retrieve from the publicly available CrownBio tumor PDX model gene database (https://www.crownbio.cn/model-systems/in-vivo/pdx-models/), and the RNA expression levels of AKR1C3 in these models were analyzed. The results are shown in Table 1.

**Table 1 Statistical results of the correspondence between AKR1C3 RNA expression levels (represented by LOG2(FPKM)) and KRAS-G12D**

| **AKR1C3 RNA Expression** | **AKR1C3 RNA Expression Level** | **% Distribution in KRAS G12D mutant PDXs** |
|---|---|---|
| ≥8 | H | 8.9% |
| 6-7.9 | M-H | 57.5% |
| 4-5.9 | M | 24.0% |
| 2-3.9 | M-L | 7.8% |
| <2 | L | 1.7% |
| Total #PDX | | ADC #PDX 179 |

FPKM is Fragments Per Kilobase of exon model per Million mapped fragments.

H represents High, M represents Middle, and L represents Low.

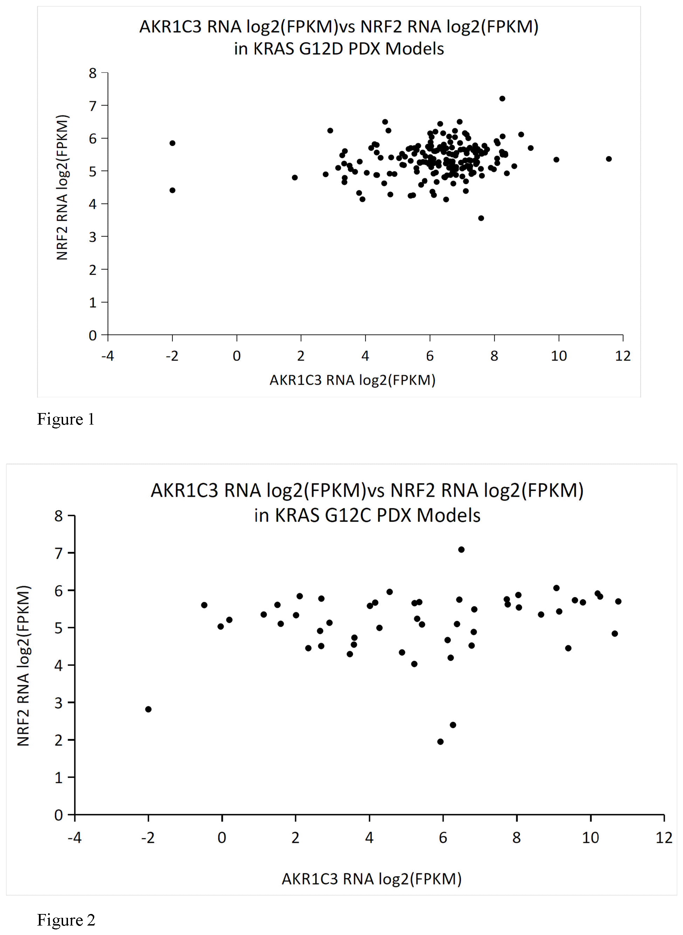

To further perform a statistical analysis on the NRF2 expression levels in these KRAS-G12D mutation models, the RNA expression levels of AKR1C3 and the RNA expression levels of NRF2 in these 179 PDX models were plotted on an X-Y coordinate graph to generate Figure 1.

The distribution trend of AKR1C3 expression levels in the KRAS G12D PDX models is mainly concentrated in the medium to high expression range (LOG2 (FPKM) ≥ 4), accounting for 90.4%.

In the KRAS G12D PDX models, the distribution trend of NRF2 expression levels is also mainly concentrated in the medium to high expression range, which is consistent with the protein expression trend of AKR1C3, showing a certain degree of correlation.

### Example 13 Distribution of AKR1C3 and analysis of its correlation with NRF2 in the KRAS G12C PDXs

The data of a total of 51 models with the KRAS-G12C mutation were retrieve from the publicly available CrownBio tumor PDX model gene database (https://www.crownbio.cn/model-systems/in-vivo/pdx-models/), and the RNA expression levels of AKR1C3 in these models were analyzed. The results are shown in Table 2.

**Table 2 Statistical results of the correspondence between AKR1C3 RNA expression levels (represented by LOG2(FPKM)) and KRAS-G12C**

| **AKR1C3 RNA Expression** | **AKR1C3 RNA Expression Level** | **% Distribution in KRAS G12C mutant PDXs** |
|---|---|---|
| ≥8 | H | 23.5% |
| 6-7.9 | M-H | 21.6% |
| 4-5.9 | M | 21.6% |
| 2-3.9 | M-L | 19.6% |
| <2 | L | 13.7% |
| Total #PDX | | ADC #PDX 51 |

To further perform a statistical analysis on the NRF2 expression levels in these KRAS-G12C mutation models, the RNA expression levels of AKR1C3 and the RNA expression levels of NRF2 in these 51 PDX models were plotted on an X-Y coordinate graph to generate Figure 2.

AKR1C3 expression levels in the KRAS G12C PDX models were evenly distributed across low, medium, and high expression ranges, with medium or higher expression levels (LOG2 (FPKM) ≥ 4) accounting for 66.7%.

In the KRAS G12C PDX models, the distribution trend of NRF2 expression levels showed a weakly correlation with the distribution of AKR1C3 protein expression levels.

### Example 14 Distribution of AKR1 C3 and analysis of its correlation with NRF2 in the KRAS G13D PDXs

The data of a total of 41 models with the KRAS-G13D mutation were retrieve from the publicly available CrownBio tumor PDX model gene database (https://www.crownbio.cn/model-systems/in-vivo/pdx-models/), and the RNA expression levels of AKR1C3 in these models were analyzed. The results are shown in Table 3

**Table 3 Statistical results of the correspondence between AKR1C3 RNA expression levels (represented by LOG2(FPKM)) and KRAS-G13D**

| **AKR1C3 RNA Expression** | **AKR1C3 RNA Expression Level** | **% Distribution in KRAS G12C mutant PDXs** |
|---|---|---|
| ≥8 | H | 8.3% |
| 6-7.9 | M-H | 39.6% |
| 4-5. 9 | M | 35.4% |
| 2-3.9 | M-L | 10.4% |
| <2 | L | 6.3% |
| Total #PDX | | ADC #PDX 48 |

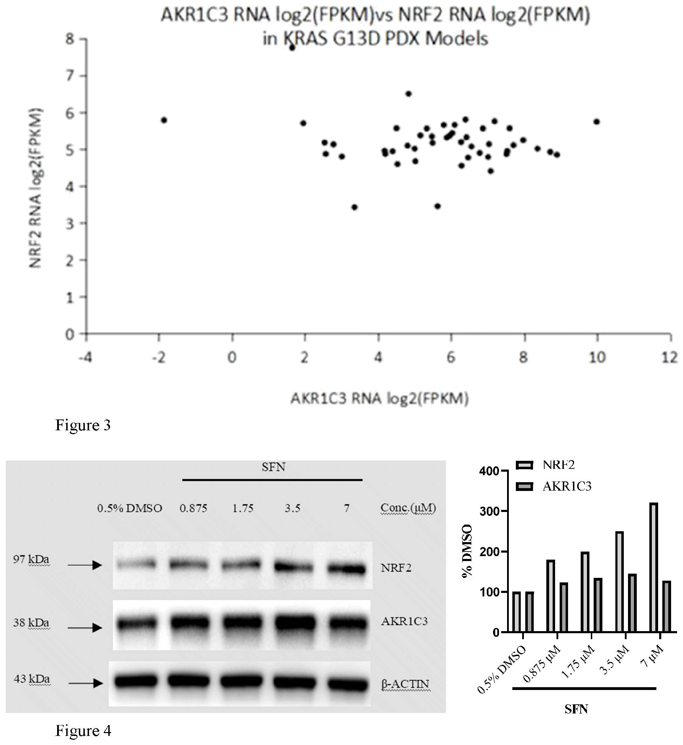

To further perform a statistical analysis on the NRF2 expression levels in these KRAS-G13D mutation models, the RNA expression levels of AKR1C3 and the RNA expression levels of NRF2 in these 48 PDX models were plotted on an X-Y coordinate graph to generate Figure 3.

AKR1C3 expression levels in the KRAS G13D PDX models were evenly distributed across low, medium, and high expression ranges, with medium or higher expression accounting for 83.3%.

In the KRAS G13D PDX models, the distribution trend of NRF2 expression levels showed a certain degree of correlation with the distribution of AKR1C3 protein expression levels.

The above statistical analyses reveal a positive correlation between NRF2 expression levels and AKR1C3 expression levels: high NRF2 expression often indicates high AKR1C3 expression; in the cells and tissues with KRAS mutations, AKR1C3 expression is often at medium to high levels. In summary, KRAS mutations (including KRAS-G13D, KRAS-G12C, and KRAS-G12D) are associated with medium to high expressions of NRF2 and AKR1C3: KRAS mutations (including KRAS-G13D, KRAS-G12C, and KRAS-G12D) are often accompanied by medium to high expressions of NRF2 and AKR1C3.

To further elucidate the relationship between KRAS mutations and NRF2 and AKR1C3, the inventors conducted relevant experiments for mechanism study.

### Example 15: The expression of AKR1C3 expression in the tumor cells with KRAS G12D mutations is reugulated by NRF2

A solution of SFN compound (Sulforaphane, NRF2 activator) was added to HPAF II cell suspension. The mixture was cultured overnight in a 37°C, 5% CO₂ incubator. The cell protein lysate was collected for Western Blotting (WB) detection.

The experiment consisted of five groups: 0.5% DMSO, 0.875 µM SFN, 1.75 µM SFN, 3.5 µM SFN, and 7 µM SFN.

HPAF II cells were treated with different concentrations of the NRF2 activator SFN, and then subjected to Western Blotting to detect the expressions of NRF2 and AKR1C3. The results of the Western Blotting and the density analysis of NRF2 and AKR1C3 protein bands under treatment with different concentrations of SFN are shown in Figure 4.

The experimental results show that as the treatment concentration of SFN increases, the expression of NRF2 increases significantly, and the expression of AKR1C3 also increases simultaneously.

The experimental results indicate that the NRF2 activating factor SFN upregulates the expression of NRF2 in a concentration dependent manner. Under the same experimental conditions, the expression of AKR1C3 is upregulated simultaneously with increasing SFN concentration. This result clearly indicates that the expression of AKR1C3 is regulated by NRF2. Furthermore, combined with the significant correlation between NRF2 mRNA and AKR1C3 mRNA in the medium to high expression ranges in Examples 12-14, it indicates that AKR1C3 expression is positively correlated with NRF2 expression in the KRAS G12D mutant PDX models.

The AKR1C3 enzyme-activated prodrugs AST-3424 and AST have significant anti-tumor effects and are well tolerated in the PDX and CDX models with high expression of AKR1C3 enzyme (PCT/CN2022/120817 with publication number WO2023/046060, Examples 1-9).

Furthermore, combining the experimental conclusions in Examples 12-14 that AKR1C3 expression is positively correlated with NRF2 expression, those skilled in the art can reasonably infer that KRAS gene mutations can upregulate or activate NRF2, thereby promoting high expression of AKR1C3, which in turn enables the AKR1C3 enzyme-activated prodrugs AST-3424 and AST to exert significant anti-tumor effects.

The compounds of general Formulae (4)-(14) in the present application are all AKR1C3 enzyme-activated anti-cancer prodrugs. Under the action of the AKR1C3 enzyme, they are metabolized and converted into anti-cancer active drugs such as AST-2660, paclitaxel, SN-38, gemcitabine, KARS inhibitors, etc. Therefore, those skilled in the art can reasonably infer that KRAS gene mutations upregulate or activate NRF2, leading to high expression of AKR1C3 enzyme, which in turn activates and metabolizes the compounds of general Formula (4)-(14) into anti-cancer active drugs. That is, patients detected with KRAS gene mutations can benefit from treatment with the compounds of general Formulae (4)-(14), especially from treatment with AST-3424 or AST.

### Example 16 Phase II clinical trial of AST-3424 conducted in China

The clinical trial registration number is CTR20191399.

This trial was approved by the ethics committee of the medical institution participating in the clinical trial and was conducted in accordance with the principles of the Declaration of Helsinki, and the informed consent form was obtained from all subjects.

### Inclusion Criteria

1. Male or female, aged ≥18 years.
2. Pathohistologically confirmed advanced HCC that cannot be controlled by surgical resection or local therapy.
3. Previously received standard systemic therapy, including but not limited to, Sorafenib and/or Oxaliplatin-containing systemic chemotherapy, Lenvatinib, Regorafenib, and/or Nivolumab, experienced disease progression, toxicity intolerance, or refusal to continue treatment with these drugs.
4. At least one measurable lesion according to RECIST 1.1 criteria. Previously irradiated lesions cannot be considered as measurable lesions unless clear radiographic progression is observed after radiotherapy.
5. Ability to provide pathological paraffin blocks or sections (including archived pathological paraffin blocks and sections) for AKR1C3 expression analysis, and with confirmation of strongly positive AKR1C3 expression in liver tumor tissues (the central laboratory immunohistochemistry results confirm that the proportion of tumor cells with AKR1C3 staining intensity of 2 + and/or 3 + is ≥ 70%).
6. Eastern Cooperative Oncology Group (ECOG) performance status score of 0 or 1.
7. Life expectancy ≥ 12 weeks.
8. With or without HBV or HCV infection.
   a. Subjects with HBV infection must have HBV-DNA level less than 2,000 IU/ml, and receive antiviral therapy with entecavir, tenofovir disoproxil fumarate, or tenofovir alafenamide fumarate according to the national guidelines for the prevention and treatment of chronic hepatitis B. The therapy must be maintained during the study and continue for 6 months after the last dose.
   b. Subjects with HCV infection (with detectable HCV-RNA or anti-HCV antibodies) may receive treatment according to medical practice.
9. Child-Pugh score ≤ 6 points.
10. No history of hepatic encephalopathy.
11. Before starting to use the study drug, all toxicities from previous anticancer therapies (except alopecia, fatigue, or peripheral neuropathy) must have recovered to Grade 1 or baseline levels (NCI CTCAE Version 5).
12. Laboratory tests must meet the following criteria. During 14 days before laboratory tests at screening, indicators cannot be corrected by blood transfusion, hematopoietic stimulating factor or albumin infusion is allowed to meet the inclusion criteria.
   a. Hemoglobin ≥ 90 g/L;
   b. Platelet count ≥ 80 × 10⁹/L;
   c. Absolute neutrophil count (ANC) ≥ 1.5 × 10⁹/L;
   d. Serum total bilirubin ≤ 3 mg/dL;
   e. ALT and AST ≤ 5.0 × ULN;
   f. International normalized ratio (INR) ≤ 2.3 or prothrombin time prolongation ≤ 6 seconds;
   g. Albumin ≥ 29 g/L;
   h. Creatinine clearance > 50 mL/min as measured according to the Cockcroft-Gault equation.
13. No history of alcohol abuse, drug addiction or substance abuse within the past year.
14. Fertile female patients must be in non-lactating and have a negative pregnancy test result within 5 days before the initiation of treatment (positive urine pregnancy test result needs to be confirmed by a serum pregnancy test).
15. Fertile female and male subjects must agree to use effective contraceptive measures (e.g. surgical sterilization or condom or diaphragm contraceptive measures combined with spermicidal gel or intrauterine contraceptive device [IUD], etc.) with their partners from the start of study participation until 6 months after the last dose.
16. Voluntary participation in this study, fully understanding of relevant risks, good compliance, and signing the informed consent form. Subjects may also sign a consent form for future biomedical research (FBR). However, subjects who would not participate in FBR may still participate in the main trial.

### Exclusion criteria:

1. Untreated active central nervous system (CNS) metastases or leptomeningeal disease. Subjects with adequately treated CNS metastases may participate if they are confirmed to be stable for at least 4 weeks by clinical examination and brain imaging (MRI or CT) during screening.
2. History of other malignant tumors within 2 years, except for adequately treated basal cell carcinoma, carcinoma in situ at other sites, or other tumors of which the natural medical history and the treatment will not interfere with the safety or efficacy assessment of the current study.
3. Major surgery (other than diagnostic surgery) performed within 4 weeks prior to the first dose.
4. Radiotherapy, surgical treatment, chemotherapy, immunotherapy, biological therapy for cancer, targeted therapy, or hormonal therapy received within 4 weeks prior to the first dose (a 6-week washout period is required for nitrosourea or mitomycin C therapy; a 2-week washout period is required for oral fluorouracil drugs; a 2-week washout period is required for small-molecule targeted therapy).
5. Participation in a (diagnostic or therapeutic) drug study or a device study within 4 weeks prior to the first dose.
6. Requirement for concomitant use of potent CYP3A4 inhibitors or inducers during the study.
7. Uncontrolled active bacterial, viral, or fungal infections requiring systemic therapy.
8. Known infection with human immunodeficiency virus (HIV) or syphilis-positive status.
9. Clinically significant ascites, defined as being detected by physical examination and requiring abdominal paracentesis for control, or requiring increased medication intervention for symptom maintenance (patients with ascites detectable only by imaging can be included).
10. Female subjects who are pregnant, lactating or planning to become pregnant.
11. Concomitant diseases or symptoms that may interfere with the conduct of the study, or physical abnormalities which the investigators consider would pose an excessive risk to the patients, including but not limited to, a history of gastrointestinal bleeding within three months or a higher risk of bleeding, active peptic ulcer or gastritis, changes in mental status, or psychiatric disorders that may interfere with the patients' understanding of the informed consent form.
12. A history of allergy to ethanol or propylene glycol.
13. Subjects who are unwilling or unable to comply with the study protocol for any reason.

### Study drug:

Injection concentrate solution of AST-3424: manufactured by a pharmaceutical enterprise entrusted by Shenzhan ASCENTAWITS PHARMACEUTICALS, LTD.; specification: 10 mg/1 mL, containing 0.75 mL ethanol, 0.25 mL propylene glycol,l and 10 mg AST-3424.

### Dosing Regimen:

One cycle involves 21 days: administration is performed on Day 1 and Day 8; the dose is 6 mg/m² per administration; and the subject will be permitted to receive treatment for a maximum of 34 cycles.

### Specific administration procedure:

Before administration, 0.1 ml of 5% sodium bicarbonate injection was added to 100 ml of commercially available sterile 5% dextrose injection water (D5W) in an intravenous infusion bag without DEHP (di(2-ethylhexyl)phthalate). The calculated required volume (precise to 0.01 mL) of the injection concentrate solution of AST-3424 was added to the pH-adjusted D5W bag to prepare the AST-3424 injection for intravenous infusion.

The solutes of the aqueous solution for intravenous injection consist of the active pharmaceutical ingredient AST-3424, glucose, ethanol, propylene glycol, and the pH regulator sodium bicarbonate, wherein the concentration of the active pharmaceutical ingredient AST-3424 is 0.004-0.94 mg/ml, the pH is 7.4, the glucose content is 4.5-5.0% by mass, and the solution is an isotonic solution.

If the subject is not suitable for glucose injection, then normal saline may be used instead:
Before administration, 0.1 ml of 5% sodium bicarbonate injection was added to 100 ml of commercially available sterile 0.9% normal saline for injection in an intravenous infusion bag without DEHP (di(2-ethylhexyl)phthalate). The calculated required volume (precise to 0.01 mL) of the injection concentrate solution of AST-3424 was added to the pH-adjusted normal saline bag to prepare the AST-3424 injection for intravenous infusion.

The solutes of the aqueous solution for intravenous injection consist of the active pharmaceutical ingredient AST-3424, sodium chloride, ethanol, propylene glycol, and the pH regulator sodium bicarbonate, wherein the concentration of the active pharmaceutical ingredient AST-3424 is 0.004-0.94 mg/ml, the pH is 7.4, the sodium chloride content of 0.81-0.90% by mass, and the solution is an isotonic solution.

The precise calculation method for the required volume of the injection concentrate solution of AST-3424 is as follows:
For a patient with a height of 175cm and a weight of 75kg, the corresponding equivalent body surface area BSA (m²) = ([Height (cm) × Weight (kg)]/3600)^{1/2}= 1.90, then the corresponding dose is 1.90 × 6.0 = 11.40 mg; accordingly, the volume of the injection concentrate solution of AST-3424 of the above specification to be drawn is 11.40 ÷ 10 × 1 = 1.14 ml.

The prepared intravenous AST-3424 injection should be injected within 8 hours.

### Clinical evaluation

Efficacy evaluation includes clinical efficacy assessment.

The clinical efficacy assessment criteria adopt the efficacy evaluation criteria for solid tumors RECIST 1.1. Lesions are evaluated by MRI/CT, and the same evaluation method should be used for the same lesion throughout the study. Subjects must have measurable tumor lesions at the baseline.

Efficacy evaluation indicators include complete response (CR), partial response (PR), stable disease (SD), and progressive disease (PD).

Complete response (CR): all target lesions disappear, and all pathological lymph nodes (including target and non-target nodes) must be reduced to < 10mm in short-axis diameter.

Partial response (PR): the sum of the diameters of the target lesions is reduced by at least 30% as compared with the baseline level.

Progressive disease (PD): taking the minimum of the sum of all measurable diameters of the target lesions throughout the experimental study as reference, the sum of the diameters of the target lesions is increased by at least 20% ( if the baseline measurement is the minimum, then the baseline value is used as reference). In addition, an absolute increase of at least 5 mm in the sum of the diameters shall be achieved (the appearance of one or more new lesions is also considered as progressive disease).

Stable disease (SD): the degree of reduction of the target lesions does not meet the criteria for PR and the degree of increase does not meet the criteria for PD, falling therebetween. The minimum of the sum of the diameters can be used as reference in the study.

### Study Endpoint

The efficacy of AST-3424 monotherapy on HCC and other malignant tumors is preliminarily evaluated based on the objective response rate (ORR), disease control rate (DCR), duration of response (DOR), and progression-free survival (PFS) of the subjects.

Objective response rate (ORR): the percentage of cases which achieve complete response (CR) and partial response (PR) after treatment among all evaluable cases.

Disease Control Rate (DCR) refers to the percentage of cases with confirmed complete response (CR), partial response (PR), and stable disease (SD) among all evaluable subjects.

### Trial Results

A total of 20 subjects with liver cancer were enrolled, Among them, 18 subjects completed the clinical efficacy evaluation, wherein 1 subject achieved PR, 10 subjects achieved SD, and 7 subjects achieved PD. Finally, the ORR was 5.6% (1/18) and the DCR was 61.1% (11/18).

In particular, 10 of these cases were subjected to p53 gene mutation or deficiency detection, wherein negative (-) indicates no mutation or deficiency was detected and positive (+) indicates mutation or deficiency was detected (which may or may not affect protein expression). The details are shown in Table 4 below.

**Table 4: Efficacy data of liver cancer cases during the clinical trial phase**

| Case screening number | Percentage of 2 + & 3 + in AKR1C3 IHC assay | Positive/ negative for p53 gene mutation or deficiency detection | Positive/ negative for KRAS gene mutation or deficiency detection | Positive/ negative for BRCA gene mutation or deficiency detection | Efficacy evaluation | PFS |
|---|---|---|---|---|---|---|
| 03003 | 90% | Negative (-) | Negative (-) | Positive (+) | PR | >6.6 |
| 04001 | 95% | Negative (-) | Negative (-) | Positive (+) | SD, lesion reduction | 7.8 |
| 05003 | 90% | Negative (-) | Negative (-) | Negative (-) | SD, lesion reduction | >2.8 |
| 04003 | 85% | Positive (+), whether it affects protein expression is unknown | Negative (-) | Negative (-) | SD, lesion increase | 2.9 |
| 06003 | 95% | Positive (+), may affect protein expression | Negative (-) | Positive (+) | SD, lesion increase | 1.4 |
| 06004 | 80% | Positive (+), may affect protein expression | Positive (+) | Negative (-) | SD, lesion increase | 1.4 |
| 03006 | 75% | Positive (+), may affect protein expression | Negative (-) | Negative (-) | PD | 1.4 |
| 05001 | 100% | Negative (-) | Negative (-) | Positive (+) | PD | 1.7 |
| 03009 | 100% | Negative (-) | Negative (-) | Positive (+) | SD, lesion increase | >1.6 |
| 02005 | 95% | Positive (+), may affect protein expression | Negative (-) | Negative (-) | PD | 1.38 |

| | | | | | | |
|---|---|---|---|---|---|---|
| PFS, cut-off date: September 1, 2023. The PFS is an estimated value, calculated using the equation: Survival (OS) (months) = (Date of death or Date of last follow-up - Date of enrollment + 1) / 30.437 | | | | | | |

Further analysis of the relationship between efficacy and negative/positive for p53 gene mutation:
Among the 5 negative (-) cases, 1 achieved PR, 3 achieved SD, and 1 achieved PD. Namely, in this subgroup of cases, the DCR was 80% (4/5), the ORR was 20% (1/5), and the mean PFS was calculated as 4.1 months.
Among the 5 positive (+) cases, 3 achieved SD with lesion enlargement, and 2 achieved PD. Namely, in this subgroup of cases, the DCR was 60% (all 3 cases show lesion enlargement); the ORR was 0%; and the mean PFS was calculated as 1.756 months.

In summary, after receiving the same dose level of AST-3424, the efficacy evaluation of the subgroups negative/positive for p53 gene mutation shows significant difference: in the subgroup of liver cancer patients negative (-) for p53 gene mutation or deficiency, the ORR is 20%, the DCR is 80%, and the PFS is 4.1 months; the corresponding data of the positive (+) subgroup are 0, 60%, and 1.756 months. The subgroup of patients negative (-) for p53 gene mutation has a better therapeutic effect.

Therefore, for the clinical results at the current stage, those skilled in the art could reasonably believe that AST-3424 produce a significantly superior therapeutic effect on cancer or tumor patients negative (-) for p53 gene mutation or deficiency compared with those positive (+) for p53 gene mutation or deficiency. Accordingly, the applicant infers that AST-3424 will have a better therapeutic effect in the treatment of tumor/cancer patients with negative detection results (-) for p53 gene mutation or deficiency, i.e., such tumor/cancer patients with negative detection results (-) for p53 gene mutation or deficiency will obtain more significant clinical benefits from the treatment with AST-3424.

Further analysis of the relationship between efficacy and negative/positive for BRCA gene mutation:
Among the 5 positive (+) cases, 1 achieved PR, 3 achieved SD, and 1 achieved PD. Namely, in this subgroup of cases, the DCR was 80% (4/5), the ORR was 20% (1/5), and the mean PFS was calculated as 3.82 months.
Among the 5 negative (-) cases, 3 achieved SD, and 2 achieved PD. Namely, in this subgroup of cases, the DCR was 60% (3/5) (all 3 cases show lesion enlargement); the ORR was 0%; and the mean PFS was calculated as 1.976 months.

In summary, after receiving the same dose level of AST-3424, the efficacy evaluation of the subgroups negative/positive BRCA gene mutation shows significant difference: in the subgroup of liver cancer patients positive (+) for BRCA gene mutation or deficiency, the ORR is 20%, the DCR is 80%, and the PFS isa 3.82 months; the corresponding data of the negative (-) subgroup are 0, 60%, and 1.976 months. The subgroup of patients positive (+) for BRCA gene mutation has a better therapeutic effect.

As of March 11, 2024, the progress of the above-mentioned ongoing clinical trials is as follows.

### Trial Results

As of March 11, 2024, a total of 30 subjects were enrolled: 5 were still receiving treatment, and 25 had exited the study (1 withdrew informed consent form, 10 died, and 14 were in survival follow-up). The current longest PFS was >11.5 months, and the longest OS was >17.6 months. A total of 11 subjects were enrolled by the end of January 2023, wherein 6 had an OS of more than 12 months. The details are shown in Table 6 below.

**Table 6: Clinical data on the correlation between the efficacy of AST-3424 in hepatocellular carcinoma and gene mutations as of March 11, 2024**

| Cases | AKR1C3 | p53 | KRAS | BRCA | Efficacy evaluation | PFS | OS |
|---|---|---|---|---|---|---|---|
| 03003 | 90% | Negative (-) | Negative (-) | Positive (+) | PR | >11.5 | >13.8 |
| 04001 | 95% | Negative (-) | Negative (-) | Positive (+) | SD, lesion reduction | 7.8 | 12.3 |
| 05003 | 90% | Negative (-) | Negative (-) | Negative (-) | SD, lesion reduction | 2.8 | >9.6 |
| 04003 | 85% | Positive (+), whether it affects protein expression is unknown | Negative (-) | Negative (-) | SD, lesion increase | 2.9 | >17.6 |
| 06003 | 95% | Positive (+), may affect protein expression | Negative (-) | Positive (+) | SD, lesion increase | 1.4 | 11.4 |
| 06004 | 80% | Positive (+), may affect protein expression | Positive (+) | Negative (-) | SD, lesion increase | 1.4 | >14.0 |
| 03006 | 75% | Positive (+), may affect protein expression | Negative (-) | Negative (-) | PD | 1.4 | >11.3 |
| 05001 | 100% | Negative (-) | Negative (-) | Positive (+) | PD | 1.7 | 10.8 |
| 03009 | 100% | Negative (-) | Negative (-) | Positive (+) | SD, lesion increase | 3.2 | 6.3 |
| 02005 | 95% | Positive (+), may affect protein expression | Negative (-) | Negative (-) | PD | 1.4 | >7.3 |
| 03007 | 100% | Positive (+), may affect protein expression | Negative (-) | Not detected | PD | 1.6 | 2.2 |
| 03008 | 95% | Positive (+), may affect protein expression | Negative (-) | Not detected | PD | 1.5 | >8.7 |
| 05004 | 80% | Positive (+), may affect protein expression | Negative (-) | Negative (-) | PD | 1.2 | >7.1 |
| 06009 | 85% | Negative (-) | Negative (-) | Negative (-) | SD, lesion increase | 2.7 | 2.9 |
| 04011 | 85% | Negative (-) | Negative (-) | Negative (-) | PD | 1.5 | >5.7 |
| 06013 | 75% | Positive (+), may affect protein expression | Negative (-) | Positive (+) | PD | 1.6 | >2.3 |
| 04013 | 100% | Positive (+), whether it affects protein expression is unknown | Not detected | Not detected | SD, lesion increase | 1.0 | >1.5 |
| 03010 | 90% | Positive (+), whether it affects protein expression is unknown | Not detected | Not detected | SD, lesion increase | 1.6 | >1.8 |
| 06014 | 100% | Negative (-) | Not detected | Not detected | SD, lesion increase | 1.6 | >2.0 |
| 06010 | 85% | Negative (-) | Not detected | Not detected | PR | 4.7 | >5.7 |
| 04008 | 100% | Not detected | Not detected | Not detected | SD, lesion increase | 3.2 | >5.9 |
| 06007 | 100% | Not detected | Not detected | Not detected | Not evaluated | 2.4 | 2.4 |
| 03004 | 100% | Not detected | Not detected | Not detected | SD, lesion reduction | 1.4 | >2.1 |
| 02004 | 100% | Not detected | Not detected | Not detected | Not evaluated | 3.3 | 3.3 |
| 04005 | 95% | Not detected | Not detected | Not detected | SD, lesion increase | 3.2 | 10.6 |
| 07006 | 100% | Not detected | Not detected | Not detected | SD, lesion increase | 2.7 | >17.0 |
| 07005 | 95% | Not detected | Not detected | Not detected | SD, lesion increase | 2.7 | >17.6 |
| 07001 | 80% | Not detected | Not detected | Not detected | PD | 1.4 | 5.7 |

Among the 26 enrolled subjects with efficacy evaluation results, 20 had p53 gene mutation detection results. The efficacy was observed by grouping according to the p53 detection results:
Wild-type (WT) with no mutation, i.e., the detection results for p53 gene mutation is negative (-);
Variant of Uncertain Significance (VUS): i.e., the detection results for p53 gene mutation is positive (+), and whether it affects protein expression is unknown;

Mutations that may affect protein function (MUT), i.e., the detection results for p53 gene mutation is positive (+), which may affect protein expression. The statistical analyses were conducted based on the above three groups and the results are shown in Tables 7, 8 and 9 below.

**Table 7: Efficacy data of 8 patients positive for P53 mutation (MUT)**

| Cases | AKR1C3 | p53 | KRAS | BRCA | Efficacy evaluation | PFS | OS |
|---|---|---|---|---|---|---|---|
| 06003 | 95% | Positive (+), may affect protein expression | Negative (-) | Positive (+) | SD, lesion increase | 1.4 | 11.4 |
| 06004 | 80% | Positive (+), may affect protein expression | Positive (+) | Negative (-) | SD, lesion increase | 1.4 | >14.0 |
| 03006 | 75% | Positive (+), may affect protein expression | Negative (-) | Negative (-) | PD | 1.4 | >11.3 |
| 02005 | 95% | Positive (+), may affect protein expression | Negative (-) | Negative (-) | PD | 1.4 | >7.3 |
| 03007 | 100% | Positive (+), may affect protein expression | Negative (-) | Not detected | PD | 1.6 | 2.2 |
| 03008 | 95% | Positive (+), may affect protein expression | Negative (-) | Not detected | PD | 1.5 | >8.7 |
| 05004 | 80% | Positive (+), may affect protein expression | Negative (-) | Negative (-) | PD | 1.2 | >7.1 |
| 06013 | 75% | Positive (+), may affect protein expression | Negative (-) | Positive (+) | PD | 1.6 | >2.3 |

The statistical results indicate that among the 8 cases, 2 have died and 6 are in survival follow-up. The current longest PFS is 1.6 months, the longest OS is >14.0 months, the mean PFS is 1.4 months, and the mean OS is more than 9.3 months. Among the 8 cases positive (+) for p53 gene mutation which may affect protein expression, 0 achieves PR, 2 achieve SD, and 7 achieve PD. Namely, in this subgroup of cases, the DCR is 25% (2/8) and the ORR is 0% (0/5).

**Table 8: Efficacy data of 9 patients negative for P53 mutation (WT)**

| Cases | AKR1C3 | p53 | KRAS | BRCA | Efficacy evaluation | PFS | OS |
|---|---|---|---|---|---|---|---|
| 03003 | 90% | Negative (-) | Negative (-) | Positive (+) | PR | >11.5 | >13.8 |
| 04001 | 95% | Negative (-) | Negative (-) | Positive (+) | SD, lesion reduction | 7.8 | 12.3 |
| 05003 | 90% | Negative (-) | Negative (-) | Negative (-) | SD, lesion reduction | 2.8 | >9.6 |
| 05001 | 100% | Negative (-) | Negative (-) | Positive (+) | PD | 1.7 | 10.8 |
| 03009 | 100% | Negative (-) | Negative (-) | Positive (+) | SD, lesion increase | 3.2 | 6.3 |
| 06009 | 85% | Negative (-) | Negative (-) | Negative (-) | SD, lesion increase | 2.7 | 2.9 |
| 04011 | 85% | Negative (-) | Negative (-) | Negative (-) | PD | 1.5 | >5.7 |
| 06014 | 100% | Negative (-) | Not detected | Not detected | SD, lesion increase | 1.6 | >2.0 |
| 06010 | 85% | Negative (-) | Not detected | Not detected | PR | 4.7 | >5.7 |

The statistical results indicate that among the 9 cases, 4 have died and 5 are in survival follow-up. The current longest PFS is more than 11.5 months, the longest OS is >13.8 months, the mean PFS is more than 4.1 months, and the mean OS is more than 7.7 months. Among the 9 cases negative (-) for p53 gene mutation, 2 achieve PR, 5 achieve SD, and 2 achieve PD. Namely, in this subgroup of cases, the DCR is 77.8% (7/9) and the ORR is 22.2% (2/9).

**Table 9: Efficacy data of 3 patients positive for P53 mutation, whether it affects protein expression (VUS) is unknown**

| Cases | AKR1C3 | p53 | KRAS | BRCA | Efficacy evaluation | PFS | OS |
|---|---|---|---|---|---|---|---|
| 04003 | 85% | Positive (+),whether it affects protein expression is unknown | Negative (-) | Negative (-) | SD, lesion increase | 2.9 | >17.6 |
| 04013 | 100% | Positive (+), whether it affects protein expression is unknown | Not detected | Not detected | SD, lesion increase | 1 | >1.5 |
| 03010 | 90% | Positive (+), whether it affects protein expression is unknown | Not detected | Not detected | SD, lesion increase | 1.6 | >1.8 |

Therefore, for the clinical results at the current stage, those skilled in the art could reasonably believe that AST-3424 produces a significantly superior therapeutic effect on cancer or tumor patients negative (-) for p53 gene mutation or deficiency compared with those positive (+) for p53 gene mutation or deficiency. Accordingly, the applicant infers that AST-3424 will have a better therapeutic effect in the treatment of tumor/cancer patients with negative detection results (-) for p53 gene mutation or deficiency, i.e., such tumor/cancer patients with negative detection results (-) for p53 gene mutation or deficiency will obtain more significant clinical benefits from the treatment with AST-3424.

Among the 26 enrolled subjects with efficacy evaluation results, 14 had BRCA gene mutation detection results. The efficacy was observed by grouping according to the BRCA gene mutation detection results (negative and positive) and the results are shown in Tables 10 and 11 below.

**Table 10: Efficacy data of 6 patients positive for BRCA gene mutation**

| Cases | AKR1C3 | BRCA | Efficacy evaluation | PFS | OS |
|---|---|---|---|---|---|
| 06003 | 95% | Positive (+) | SD, lesion increase | 1.4 | 11.4 |
| 06013 | 75% | Positive (+) | PD | 1.6 | >2.3 |
| 03003 | 90% | Positive (+) | PR | >11.5 | >13.8 |
| 04001 | 95% | Positive (+) | SD, lesion reduction | 7.8 | 12.3 |
| 05001 | 100% | Positive (+) | PD | 1.7 | 10.8 |
| 03009 | 100% | Positive (+) | SD, lesion reduction | 3.2 | 6.3 |

The statistical results indicate that among the 6 cases, 4 have died and 2 are in survival follow-up. The current longest PFS is more than 11.5 months, the longest OS is more than 13.8 months, the mean PFS is 4.5 months, and the mean OS is more than 9.5 months. Among the 8 cases positive (+) for BRCA gene mutation, 1 achieve PR, 3 achieve SD, and 2 achieve PD. Namely, in this subgroup of cases, the DCR is 66.7% (4/6) and the ORR is 16.7% (1/6).

**Table 11: Efficacy data of 8 patients negative for BRCA gene mutation (WT)**

| Cases | AKR1C3 | BRCA | Efficacy evaluation | PFS | OS |
|---|---|---|---|---|---|
| 04003 | 85% | Negative (-) | SD, lesion increase | 2.9 | >17.6 |
| 06004 | 80% | Negative (-) | SD, lesion increase | 1.4 | >14.0 |
| 03006 | 75% | Negative (-) | PD | 1.4 | >11.3 |
| 02005 | 95% | Negative (-) | PD | 1.4 | >7.3 |
| 05004 | 80% | Negative (-) | PD | 1.2 | >7.1 |
| 05003 | 90% | Negative (-) | SD, lesion reduction | 2.8 | >9.6 |
| 06009 | 85% | Negative (-) | SD | 2.7 | 2.9 |
| 04011 | 85% | Negative (-) | PD | 1.5 | >5.7 |

The statistical results indicate that among the 8 cases, 1 has died and 7 are in survival follow-up. The current longest PFS is 2.9 months, the longest OS is more than 17.6 months, the mean PFS is 1.9 months, and the mean OS is more than 9.4 months. Among the 8 cases negative (-) for BRCA gene mutation, 0 achieve PR, 4 achieve SD, and 4 achieve PD. Namely, in this subgroup of cases, the DCR is 50% (4/8) and the ORR is 0% (0/8).

Therefore, for the clinical results at the current stage, those skilled in the art could reasonably believe that AST-3424 produces a significantly superior therapeutic effect on cancer or tumor patients positive (+) for BRCA gene mutation or deficiency compared with those negative (-) for BRCA gene mutation or deficiency. Accordingly, the applicant infers that AST-3424 will have a better therapeutic effect in the treatment of tumor/cancer patients with positive detection results (+) for BRCA gene mutation or deficiency, i.e., such t tumor/cancer patients with positive detection results (+) for BRCA gene mutation or deficiency will obtain more significant clinical benefits from the treatment with AST-3424.

### Example 17 Clinical detection of KRAS mutation and protein expression level of AKR1C3 enzyme conducted in China

In a clinical trial approved by the Chinese regulatory authorities, the Chinese patients enrolled were subjected to the clinical test for KRAS mutation and protein expression level of AKR1C3 enzyme.

This trial was approved by the Ethics Committee of the medical institution participating in the clinical trial and was conducted in accordance with the principles of the Declaration of Helsinki, and the informed consent form was obtained from all subjects.

The protein expression levels of AKR1C3 enzyme were detected according to the methods described in WO2022048492, and the results were scored using the H-score system to characterize the expression levels, wherein an H-score of 135 or higher was defined as high expression.

Among the 10 patients positive for KRAS, 7 showed high expression of AKR1C3. The specific detection results are shown in Table 5 below.

**Table 5: Clinical detection data of KRAS mutation and protein expression level of AKR1C3 enzyme**

| Case screening number/ enrollment number | Percentage of 2 + & 3 + in AKR1C3 IHC assay and H-score | Positive/negative for KRAS gene mutation or deficiency detection | Primary tumor |
|---|---|---|---|
| 01003/10202 | 25%/75 | Positive (+), G12D | Rectal cancer |
| 01005/10301 | 2%/9 | Positive (+), G12D | Rectal cancer |
| 03002/10303 | 65%/195 | Positive (+), G12D | Pancreatic cancer |
| 01007/10402 | 80%/225 | Positive (+), G12D | Appendiceal adenocarcinoma |
| 03004/10503 | 80%/230 | Positive (+), G12D | Intrahepatic cholangiocarcinoma |
| 01009/10501 | 80%/245 | Positive (+), G12D | Rectal cancer |
| 03005/10602 | 0/90 | Positive (+), G12R | Ampullary carcinoma |
| 03001/10302 | 65%/210 | Positive (+), G12D | Pancreatic cancer |
| 06002/10401 | 75%/225 | Positive (+), G12D | Cervical cancer |
| 02002/10601 | 100%/300 | Positive (+), G12V | Pancreatic cancer |

The following experiments were further conducted using p53 wild-type H460 cells, A549 cells, p53 mutant HPAF-II cells, and p53 knockout cells.

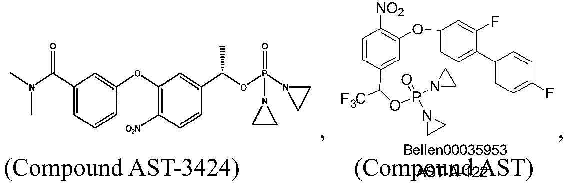

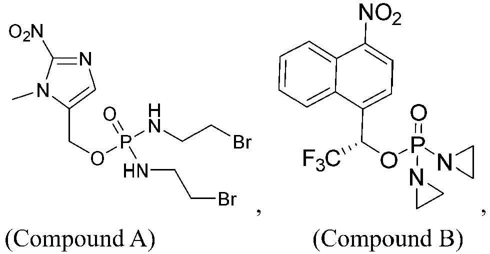

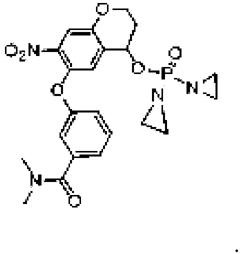

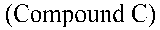

Unless otherwise specified, the aforementioned compounds were synthesized and prepared by the applicant with reference to the corresponding patent applications.

Further review of relevant literature reveals the following:
The main function of the p53 protein is to monitor DNA damage in cells. It can induce cells to enter cell cycle arrest, facilitate repair of DNA damage, and promote tumor cell apoptosis, ultimately inhibiting tumor growth (Marei, H.E., Althani, A., Afifi, N. et al. p53 signaling in cancer progression and therapy. Cancer Cell Int 21, 703 (2021). https://doi.org/10.1186/s12935-021-02396-8).

The p53 mutation/deficiency will lead to a series of reactions and manifestations:
The p53 mutation/deficiency can impair the apoptotic pathway, thereby resulting in subsequent drug resistance (Sturm I, Bosanquet A G, Hermann S, et al. Mutation of p53 and consecutive selective drug resistance in B-CLL occurs as a consequence of prior DNA-damaging chemotherapy[J].Cell Death & Differentiation, 2003, 10(4):477.DOI:10.1038/sj.cdd.4401194).

The p53 mutation/deficiency would impair cell cycle arrest, allowing damaged cells to continue dividing and surviving, thereby resulting in drug resistance (Zhao, D., Tahaney, W.M., Mazumdar, A. et al. Molecularly targeted therapies for p53-mutant cancers. Cell. Mol. Life Sci.2017,74, 4171-4187. https://doi.org/10.1007/s00018-017-2575-0).

The p53 mutation/deficiency causes improper activation of DNA repair pathways, leading to the accumulation of DNA damage in cancer cells and subsequent drug resistance (Williams AB, Schumacher B. p53 in the DNA-Damage-Repair Process. Cold Spring Harb Perspect Med. 2016,6(5):a026070. doi:10.1101/cshperspect.a026070.

The p53 mutation/deficiency accelerates and increases the efficiency of generating cancer stem cells (CSC) with drug-resistant properties, thereby resulting in drug resistance. The gain-of-function mutations of p53 can also promote CSC generation and subsequent chemoresistance (Ozaki T, Nakamura M, Shimozato O. Novel Implications of DNA Damage Response in Drug Resistance of Malignant Cancers Obtained from the Functional Interaction between p53 Family and RUNX2. Biomolecules. 2015,5(4):2854-2876. doi:10.3390/biom5042854).

The gain-of-function mutations of p53 can promote tumor progression, further leading to drug resistant and treatment-refractory state (Alvarado-Ortiz E, de la Cruz-López KG, Becerril-Rico J, Sarabia-Sánchez MA, Ortiz-Sánchez E, García-Carrancá A. Mutant p53 Gain-of-Function: Role in Cancer Development, Progression, and Therapeutic Approaches. Front Cell Dev Biol. 2021,8:607670. Published 2021 Feb 11. doi:10.3389/fcell.2020.607670).

In other words, p53 plays an important role in the process of DNA damage repair and cell apoptosis. Once the p53 gene is mutated or defective, it would lead to abnormal p53 protein, which would not exert the above functions and resulting drug resistance in tumors or cancer cells.

DNA alkylating agent prodrug compounds (such as AST-3424) cause DNA damage and breakage by releasing DNA alkylating agents (e.g., AST-2660, Br-IPM, and the like) *in vivo* to crosslink with DNA, leading to cell death. The p53 mutation/deficiency would inhibit the ability of the p53 protein to promote tumor cell apoptosis after DNA damage. Based on this, it can be inferred that the cells negative for TP53 gene mutation and with normal p53 protein expression, after p53 protein is activated or upregulated, can promote the apoptosis of tumor cells with DNA damage. A series of experimental phenomena associated with the above-mentioned enhanced cell apoptosis and DNA damage toxicity can be observed.

Accordingly, the following experiments were conducted using p53 wild-type H460 cells, A549 cells, p53 mutant HPAF-II cells, and p53 knockout cells. Unless otherwise specified, the H460 cells in the following Examples are all NCI-H460 cells.

Example 18: Experiments about the effect on *in vitro* cell proliferation and about the effect on cell colony formation

Nutlin-3 is a small-molecule MDM2-p53 inhibitor that indirectly activates p53 function by inhibiting the interaction between MDM2 and p53. Therefore, the experiments about the effect on cell proliferation and about the effect on cell colony formation were conducted after adding AST-3424/AST combined with Nutlin-3 for activating/upregulating p53 function.

H460 cells are p53 wild-type cells, i.e., negative for p53 gene mutation with normal expression of p53 protein.

HPAF-II cells are p53 mutant cells, i.e., positive for p53 gene mutation with abnormal expression of p53 protein.

### Effects of compounds on in vitro H460 cell proliferation under normoxia

### Experimental Procedure Overview:

1) H460 cell suspension was added to a 96-well plate (100 µL per well), with a cell density of 2000 cells/well.
2) The cells were cultured overnight in a 37°C, 5% CO₂ incubator.
3) Compound Treatment

Monotherapy: 24 hours after cell plating, each well was supplemented with 99.5 µL of growth medium. Then, 0.5 µL of test compounds at different concentrations was added. The plate was gently shaken to ensure even mixing, and then placed in a 37°C, 5% CO₂ incubator.

Combination therapy: 24 hours after cell plating, each well was supplemented with 99 µL of growth medium. Then, 0.5 µL of the combination compound Nutlin-3 was added. The plate was gently shaken to ensure even mixing, and then placed in an incubator for 2 hours. Then, 0.5 µL of test compounds at different concentrations was added. The plate was gently shaken to ensure even mixing, and then placed in a 37°C, 5% CO₂ incubator.
4) The cell plate was placed in the incubator for 72 hours.
5) The cell plate to be tested was placed at room temperature and equilibrated for 30 minutes, and 100 µL of medium was discarded from each well.
6) 25 µL of CTG reagent was added to each well, placed on a fast shaker to shake for 2 minutes, and placed at room temperature for 30 minutes (protected from light).
7) The chemiluminescence signal value was read using a multifunctional microplate reader with a reading time of 1000 ms.
8) IC₅₀ values were calculated using Graph Pad Prism 5 software.

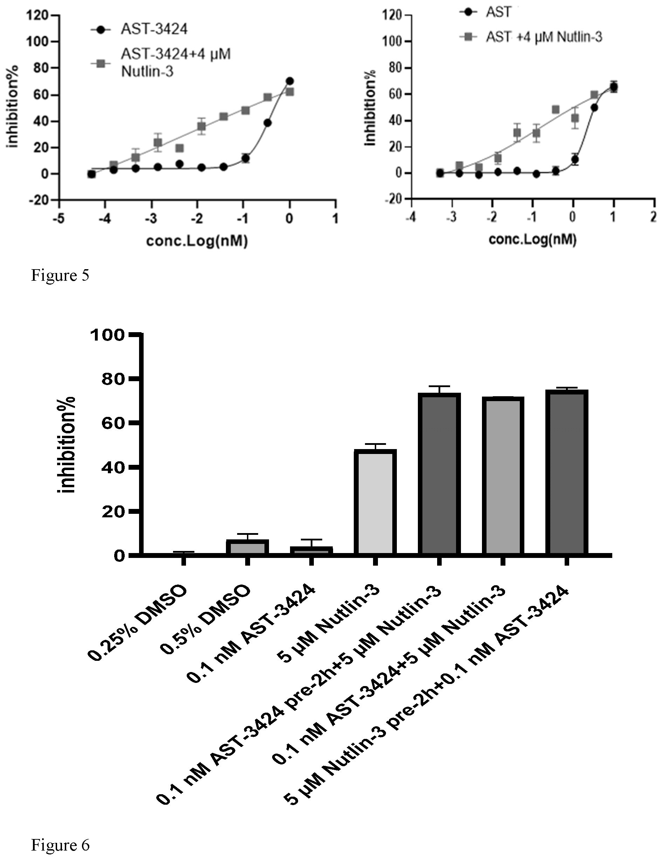

The experimental results are shown in figure 5.

The experiment shows that adding Nutlin-3 (which indirectly activates p53 function) prior to AST-3424, can significantly increase the proliferation inhibition rate of AST-3424.

Further combination therapy experiments with different administration sequences of AST-3424 were carried out, setting up dosing regimens during the above experiments.

Monotherapy: the single drug AST-3424 or Nutlin-3 was added to each well to treat the cells (DMSO 0.25% and 0.50%): 5 µL of the compound at different concentrations (400 times) and 5 µL of medium.

Combination therapy: cells were treated with both drugs AST-3424 and Nutlin-3 (5 µL each) in different administration sequences: AST-3424 pre-treatment for 2 hours, Nutlin-3 co-treatment for 6 hours; Nutlin-3 pre-treatment for 2 hours, AST-3424 co-treatment with for 6 hours; and AST-3424 and Nutlin-3 co-treatment for 6 hours.

The experimental results are shown in Figure 6.

The results of the cell proliferation experiments (3 days) indicate that the proliferation inhibition rate of AST-3424 is significantly increased after addition of Nutlin-3 (which indirectly activates p53 function), and this effect is independent of the sequence of adding Nutlin-3.

Experiments about the effect of different administration sequences of AST-3424 ± Nutlin-3 on colony formation of H460/HPAF-II cells

### Experimental Procedure Overview:

H460 cells: 1000 cells/2 ml/well/6-well plate
HPAF-II cells: 3000 cells/2 ml/well/6-well plate
   1) H460 cell suspension was added to a 6-well plate (2 mL per well), with a cell density of 1000 cells/well. HPAF-II cell suspension was added to a 6-well plate (2 mL per well), with a cell density of 3000 cells/well.
   2) 1990 µL of medium was used for cell seeding, and the cells were cultured overnight in a 37°C, 5% CO₂ incubator.
   3) Compound Treatment
      Monotherapy: the single drug AST-3424 or Nutlin-3 was added to each well to treat the cells (DMSO 0.25%) for 6 hours: 5 µL of the compound at different concentrations (400 times) and 5 µL of medium.
      Combination therapy: cells were treated with both drugs AST-3424 and Nutlin-3 (5 µL each) in different administration sequences: AST-3424 pre-treatment for 2 hours, Nutlin-3 co-treatment for 6 hours; Nutlin-3 pre-treatment for 2 hours, AST-3424 co-treatment with for 6 hours; and AST-3424 and Nutlin-3 co-treatment for 6 hours.
   4) After 6 hours of co-treatment, the cells were washed twice to remove the compounds, and 5 mL of medium was added to each well.
   5) The cells were cultured for 7 days (medium change time (once every 3 days) and observation end time could be determined according to specific cell proliferation condition).
   6) Staining was performed when the vast majority of cell colonies reached about 50 cells at 0 nM (0.5% DMSO). The medium was aspirated and discarded, and the cells were fixed and stained with 0.5%[w/v] crystal violet for 40 min. The plates were washed twice with tap water and dried before counting.
   7) Cell colony counting was performed, and the number of colonies containing more than 50 cells was recorded.

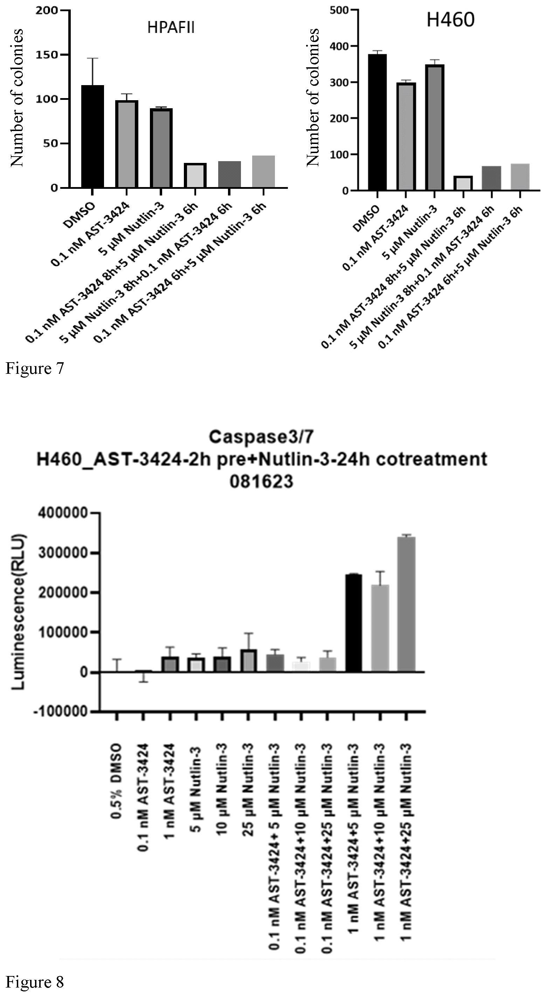

The experimental results are shown in Figure 7.

As compared with each single drug, the combination of Nutlin-3 and AST-3424 significantly inhibits colony formation of H460 and HPAF-II cells, exhibiting an additive effect.

Among the three groups of H460 cells treated with both drugs, the group in which AST-3424 is added first shows fewer colonies than the other two groups.

Among the three groups of HPAF-II cells treated with both drugs, different administration sequences have no significant effect.

For H460 cells, the colony numbers of the three combination therapy groups with different administration sequences were respectively compared with the DMSO group. The percentages of colony numbers in the combination therapy groups relative to the DMSO group were used to reflect the ability of the combination therapy to inhibit cell colony formation. A lower percentage indicates a stronger inhibitory ability of the drug on cell colony formation. The percentages of colony numbers of the combination therapy groups with three administration sequences relative to the DMSO group are 11.12%, 23.03% and 21.45%, respectively. Analysis of HPAF-II cell data in the same way shows that the percentages of colony numbers of the combination therapy groups with three administration sequences relative to the DMSO group are 24.14%, 30.41% and 40.30%, respectively.

Based on these results, it can be inferred that, relatively speaking, the combination with Nutlin-3 exhibits a significantly superior inhibitory effect on colony formation of p53 wild-type H460 cells compared with p53 mutant HPAF-II cells.

Experimental conclusion: the above experimental results show that Nutlin-3 can indeed enhance the *in vitro* cytotoxicity of AST-3424/AST against cancer cells, and the enhancement extent of *in vitro* cytotoxicity is greater in p53 wild-type H460 cells.

### Example 19: Effect of the combination of Nutlin-3 and AST-3424 on cell apoptosis and cell cycle g2/m arrest

Effects of AST-3424 alone, Nutlin-3 alone, or their combination on apoptosis process of H460 cells

### Experimental Procedure Overview:

1) H460 cell suspension was added to a 96-well white plat ( 99 µL per well), with a cell density of 15000 cells/well. Meanwhile, a 96-well transparent plate with the same cell density was plated for photographing and observation before detection.
2) The cells were cultured overnight in a 37°C, 5% CO₂ incubator.
3) Compound Treatment

Monotherapy: 24 hours after cell plating, each well was supplemented with 0.5 µL of medium. According to the protocol, 0.5 µL of the compound at the corresponding concentration was added to the designated cell wells. The plate was gently shaken to ensure even mixing, and then placed in a 37°C, 5% CO₂ incubator.

Combination therapy: 24 hours after cell plating, 0.5 µL of Compound Nutlin-3 at the corresponding concentration was added to the designated cell wells. The plate was gently shaken to ensure even mixing, and then placed in a 37°C, 5% CO₂ incubator and cultured for 2 hours. Then, 0.5 µL of AST-3424 was added to the designated cell wells.
4) The cell plate was placed in the incubator for 24 hours.
5) The cell plate to be tested was placed at room temperature and equilibrated for 5 minutes.
6) CaspaseGlo^{®}3/7 reagent was prepared and equilibrated to room temperature. The buffer and the substrate of the reagent were mixed thoroughly at a ratio of 1:1 to prepare the CaspaseGlo^{®}3/7 reagent. The prepared reagent can be stored at 4°C for 3 days.
7) 100 µL of the CaspaseGlo ^{®} 3/7 reagent was added to each well containing 100 µL of blank (no cell well), negative control cells, or treated cells. Due to the sensitivity of this assay, care was taken to prevent the pipette tip from touching the well containing the sample to avoid cross-contamination. The plate was covered with a plate sealer or lid.
8) The reagent solution was gently mixed using a shaker at 300-500 rpm for 30 seconds. The plates were placed at room temperature for 1 hour (protected from light).
9) The luminescence of each sample was measured using a plate reading photometer. The luminescence values of each group were calculated and analyzed as bar graphs using Relative Luminescence Units (RLU). The results are shown in Figure 8.

Compared with each single drug, the combination of AST-3424 (1 nM) and Nutlin-3 at effective concentrations significantly increases the content of Caspase 3/7, a marker of cancer cell apoptosis, indicating that the combination could enhance cell apoptosis. The content of the marker varies after treatment with different doses of Nutlin-3, demonstrating that the aforementioned increase in apoptosis is Nutlin-3 dose-dependent. Namely, as the concentration of Nutlin-3 increases, the content of the cell apoptosis detection marker also increases after treated with the combination of AST-3424 and Nutlin-3, also indicating that the aforementioned increase in apoptosis is Nutlin-3 dose-dependent.

In other words, the addition of Nutlin-3 (which indirectly activates p53 protein function) could enhance the cell apoptotic effect of AST-3424.

### Effects of AST-3424 alone, Nutlin-3 alone, or their combination on cell cycle G2/M arrest

The whole cell replication cycle can be described as G0/G1, S, and G2/M phases. In tumor pathological study, the proportion of cells in S phase is usually used as an indicator to judge the tumor proliferation status.

### Experimental Procedure Overview:

1) H460/HPAF-II cell suspensions were added to a 24-well plate, with a cell density of 100000 cells/well and 995 µL of medium per well.
2) The cells were cultured overnight in a 37°C, 5% CO₂ incubator.
3) Compound Treatment

24 hours after cell plating, according to the protocol, the experimental groups for each cell line were as follows:
Monotherapy: 24 hours after cell plating, 5 µL of test compounds at different concentrations was added: 1% DMSO, 5 µM Nutlin-3, and 0.1 nM AST-3424. The plate was gently shaken to ensure even mixing, and then placed in a 37°C, 5% CO₂ incubator.

### Combination therapy:

Cells were pretreated with AST-3424 for 2 hours, and then co-treatment with Nutlin-3 for 24 hours;
cells were pretreated with Nutlin-3 for 2 hours, and then co-treatment with AS -3424 for 24 hours; and
cells were co-treated with AST-3424 and Nutlin-3 for 24 hours.

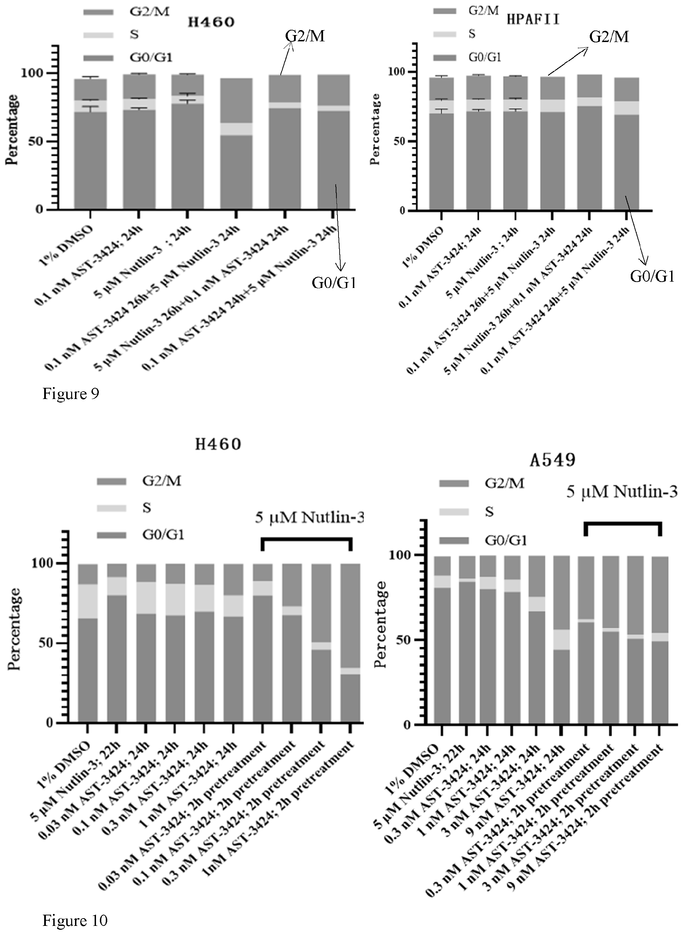

4) The cells were digested, centrifuged at 1000 g at 4°C, washed once with pre-cooled PBS, centrifuged at 1000 g at 4°C, and fixed with 1 mL of pre-cooled 70% ethanol at - 20°C overnight.
5) The cells were centrifuged at 3000 g at 4°C, washed once with pre-cooled PBS (phosphate buffer), and centrifuged at 3000g at 4°C. PI staining solution was prepared. The cells were stained at 37°Cfor 30 minutes (protected from light), and stored in an ice bath (protected from light). The cells were detected using a flow cytometer on the same day, and the proportions of cells in different cell cycle phases was analyzed using FlowJo. The results are shown in Figure 9.

The experimental results show that for p53 wild-type H460 cells, monotherapy treatment with 0.1 nM AST-3424 or 5 µM Nutlin-3 does not significantly alter the cell cycle; however, the combination of first adding 0.1 nM AST-3424 and then adding 5 µM Nutlin-3 significantly decreases the G0/G1 phase and increases the G2/M phase. The S phase decreases slightly in the other two groups. However, the cell cycle of HPAF-II cells with p53 pathogenic mutations is less affected.

Numerous studies have shown that cell cycle arrest at the G2/M phase can induce cell apoptosis.

To further study the effects of AST-3424 at different concentrations alone, Nutlin-3 alone, and their combination, as well as different administration sequences in combination therapy, on the cell cycle of H460/A549 cells (p53 wild-type, normal protein expression), a second experiment was further conducted.

The remaining operations were similar to those of the above experiment, and the compound treatment was as follows:

### Monotherapy:

For H460 cell line, 1% DMSO, adding 5 µM Nutlin-3 for treatment for 22 hours, adding 0.03 nM AST-3424 for treatment for 24 hours, adding 0.1 nM AST-3424 for treatment for 24 hours, adding 0.3 nM AST-3424 for treatment for 24 hours, and adding 1 nM AST-3424 for treatment for 24 hours;
For A549 cell line, 1% DMSO, adding 5 µM Nutlin-3 for treatment for 22 hours, adding 0.3 nM AST-3424 for treatment for 24 hours, adding 1 nM AST-3424 for treatment for 24 hours, adding 9 nM AST-3424 for treatment for 24 hours, and adding 9 nM AST-3424 for treatment for 24 hours.

### Combination therapy:

For H460 cell line,
Adding 0.03 nM AST-3424 for pre-treatment for 2 hours, and then adding 5 µM Nutlin-3 for co-treatment for 22 hours;
Adding 0.1 nM AST-3424 for pre-treatment for 2 hours, and then adding 5 µM Nutlin-3 for co-treatment for 22 hours;
Adding 0.3 nM AST-3424 for pre-treatment for 2 hours, and then adding 5 µM Nutlin-3 for co-treatment for 22 hours;
Adding 1 nM AST-3424 for pre-treatment for 2 hours, and then adding 5 µM Nutlin-3 for co-treatment for 22 hours.

For A549 cell line,
Adding 0.3 nM AST-3424 for pre-treatment for 2 hours, and then adding 5 µM Nutlin-3 for co-treatment for 22 hours;
Adding 1 nM AST-3424 for pre-treatment for 2 hours, and then adding 5 µM Nutlin-3 for co-treatment for 22 hours;
Adding 3 nM AST-3424 for pre-treatment for 2 hours, and then adding 5 µM Nutlin-3 for co-treatment for 22 hours;
Adding 9 nM AST-3424 for pre-treatment for 2 hours, and then adding 5 µM Nutlin-3 for co-treatment for 22 hours.

The results are shown in Figure 10.

Further experimental results show that AST-3424 induces concentration-dependent G2/M cell cycle arrest in p53 wild-type cancer cells (H460 and A549).

The combination of AST-3424 and Nutlin-3 significantly increases the G2/M phase arrest, decreases the G0/G1 phase, and exhibits AST-3424 concentration dependence in p53 wild-type cancer cells (H460 and A549).

The combination of AST-3424 and Nutlin-3 (AST-3424 pre-treatment for 2 hours) increases the G2/M phase arrest, and the results are consistent with the results of the above preliminary experiment with the same administration sequences.

The above results indicate that Nutlin-3 (which indirectly activates p53 function) can alter the cell cycle of p53 wild-type cancer cells (H460 and A549), leading to G2/M cell cycle arrest, and exhibit AST-3424 concentration dependence. However, Nutlin-3 has no effect on the cell cycle of HPAF-II cells with p53 pathogenic mutation. In other words, the combination of Nutlin-3 (which indirectly activates p53 function) and AST-3424 can regulate the cell cycle of p53 wild-type cancer cells (H460 and A549), increase the G/M cell cycle arrest, and exhibit AST-3424 concentration dependence, but has no effect on the cell cycle of HPAF-II cells with p53 pathogenic mutation.

According to Examples 18 and 19, p53 protein has a determinant effect on the efficacy of the drug AST-3424/AST. DNA alkylating agents such as AST-3424/AST have a stronger proliferation inhibitory effect on the cells negative for p53 gene mutation and with normal p53 protein. Their action mechanisms thereof include cell cycle G2/M arrest and cell colony inhibition.

To further confirm the above conclusion, the p53-MDM2 inhibitor RITA (which is also a p53-HDM-2 inhibitor) was selected and used to activate p53 function, and further *in vitro* cytotoxicity experiments were carried out.

The CAS number of RITA is 213261-59-7.

Example 20: Effects of AST alone, Nutlin-3 alone, RITA alone, or combinations thereof on HPAF-II/H460 cytotoxicity

### In vitro proliferation inhibition experiment

### Experimental Procedure Overview

1) H460 and HPAF-II cell suspensions were added to a 96-well plate (100 µL per well), with cell densities of 2000 cells/well and 20000 cells/well, respectively.
2) The cells were cultured overnight in a 37°C, 5% CO₂ incubator.
3) Compound Treatment

Combination therapy: 24 hours after cell plating, each well was supplemented with 99 µL of growth medium. Then, 0.5 µL of Nutlin-3 or RITA was added to each well. The plate was gently shaken to ensure even mixing, and then placed in an incubator for 2 hours. Then, 0.5 µL of test compounds at different concentrations was added. The plate was gently shaken to ensure even mixing, and then placed in a 37°C, 5% CO₂ incubator.

Monotherapy: 24 hours after cell plating, each well was supplemented with 99.5 µL of growth medium. Then, 0.5 µL of test compounds at different concentrations was added. The plate was gently shaken to ensure even mixing, and then placed in a 37°C, 5% CO₂ incubator.
4) The cell plate was placed in the incubator for 72 hours.
5) The cell plate to be tested was placed at room temperature and equilibrated for 30 minutes, and 100 µL of medium was discarded from each well.
6) 25 µL of CTG reagent was added to each well, placed on a fast shaker to shake for 2 minutes, and placed at room temperature for 30 minutes (protected from light).
7) The chemiluminescence signal value was read using a multifunctional microplate reader with a reading time of 1000 ms, and the corresponding inhibition rate data were calculated.

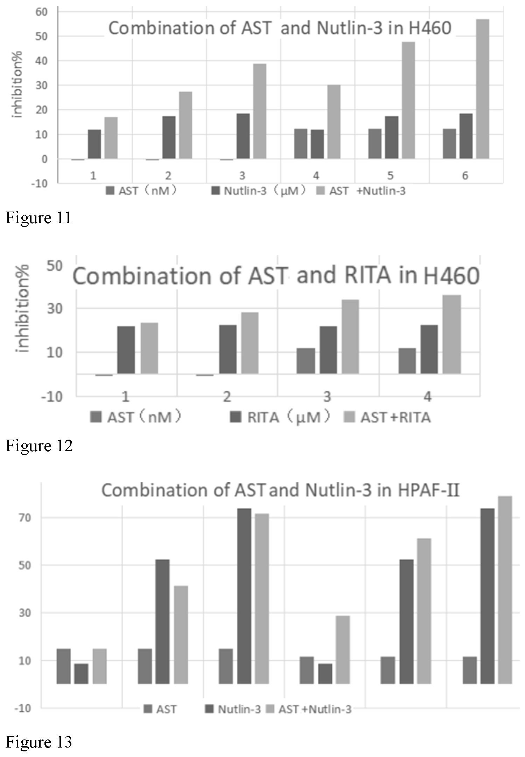

The dosing groups and experimental results of AST alone, Nutlin-3 alone, and their combination in the H460 cell proliferation inhibition experiment are shown in Table 12 below, and Figure 11 was plotted according to the inhibition rate data in Table 12.

**Table 12: The dosing groups and inhibition rate results of AST alone, Nutlin-3 alone, and their combination in the H460 cell proliferation inhibition experiment**

| Experimental group | 1 | 2 | 3 | 4 | 5 | 6 |
|---|---|---|---|---|---|---|
| AST (nM) | 0.5 | 0.5 | 0.5 | 1.5 | 1.5 | 1.5 |
| Nutlin-3 (µM) | 0.25 | 0.5 | 1 | 0.25 | 0.5 | 1 |
| AST + Nutlin-3 | 0.5+0.25 | 0.5+0.5 | 0.5+1 | 1.5+0.25 | 1.5+0.5 | 1.5+1 |

| Experimental group and inhibition rate | Inhibition rate result / % | | | | | |
|---|---|---|---|---|---|---|
| | 1 | 2 | 3 | 4 | 5 | 6 |
| AST | -0.29 | -0.29 | -0.29 | 12.19 | 12.19 | 12.19 |
| Nutlin-3 | 11.8 | 17.27 | 18.3 | 11.8 | 17.27 | 18.3 |
| AST + Nutlin-3 | 17.08 | 27.33 | 38.53 | 30.07 | 47.56 | 56.99 |

The dosing groups and experimental results of AST alone, RITA (a p53-HDM-2 inhibitor which indirectly upregulates p53 expression and function) alone, and their combination in the H460 cell proliferation inhibition experiment are shown in Table 13 below, and Figure 12 was plotted according to the inhibition rate data in Table 13.

**Table 13: The dosing groups and inhibition rate results of AST alone, RITA alone, and their combination in the H460 cell proliferation inhibition experiment**

| Experimental group | 1 | 2 | 3 | 4 |
|---|---|---|---|---|
| AST (nM) | 0.5 | 0.5 | 1.5 | 1.5 |
| RITA (µM) | 0.025 | 0.05 | 0.025 | 0.05 |
| AST + RITA | 0.5+0.025 | 0.5+0.05 | 1.5+0.025 | 1.5+0.05 |

| Experimental group and inhibition rate | Inhibition rate result / % | | | |
|---|---|---|---|---|
| | 1 | 2 | 3 | 4 |
| AST | -0.29 | -0.29 | 12.19 | 12.19 |
| RITA | 22.31 | 22.4 | 22.31 | 22.4 |
| AST + RITA | 23.91 | 28.34 | 34.21 | 36.57 |

The dosing groups and experimental results of AST alone, Nutlin-3 alone, and their combination in the HPAF-II cell proliferation inhibition experiment are shown in Table 4 below, and Figure 13 was plotted according to the inhibition rate data in Table 14.

**Table 14: The dosing groups and inhibition rate results of AST alone, Nutlin-3 alone and their combination in the HPAF-II cell proliferation inhibition experiment**

| Experimental group | 1 | 2 | 3 | 4 | 5 | 6 |
|---|---|---|---|---|---|---|
| AST (nM) | 20 | 20 | 20 | 60 | 60 | 60 |
| Nutlin-3 (µM) | 7.5 | 10 | 15 | 7.5 | 10 | 15 |
| AST + Nutlin-3 | 20+7.5 | 20+10 | 20+15 | 60+7.5 | 60+10 | 60+15 |

| Experimental group and inhibition rate | Inhibition rate result / % | | | | | |
|---|---|---|---|---|---|---|
| | 1 | 2 | 3 | 4 | 5 | 6 |
| AST | 14.9 | 14.9 | 14.9 | 11.75 | 11.75 | 11.75 |
| Nutlin-3 | 8.54 | 52.46 | 73.93 | 8.54 | 52.46 | 73.93 |
| AST + Nutlin-3 | 14.84 | 41.52 | 72.03 | 28.7 | 61.42 | 79.12 |

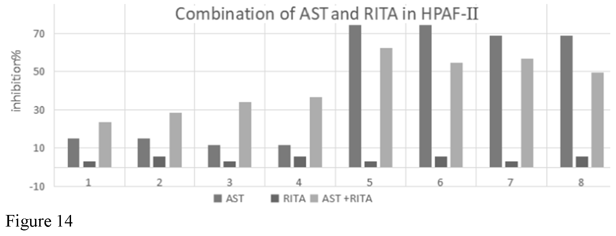

The dosing groups and experimental results of AST alone, RITA alone, and their combination in the HPAF-II cell proliferation inhibition experiment are shown in Table 15 below, and Figure 14 was plotted according to the inhibition rate data in Table 15.

**Table 15: The dosing groups and inhibition rate results of AST alone, RITA alone and their combination in the HPAF-II cell proliferation inhibition experiment**

| Experimental group | 1 | 2 | 3 | 4 | 5 | 6 | 7 | 8 |
|---|---|---|---|---|---|---|---|---|
| AST (nM) | 60 | 60 | 200 | 200 | 1667 | 1667 | 5000 | 5000 |
| RITA (µM) | 2.5 | 5 | 2.5 | 5 | 2.5 | 5 | 2.5 | 5 |
| AST + RITA | 60+ 2.5 | 60+ 5 | 200+ 2.5 | 200+ 5 | 1667+ 2.5 | 1667+ 5 | 5000+ 2.5 | 5000+ 5 |
| Experimental group and inhibition rate | Inhibition rate/% | | | | | | | |
| | 1 | 2 | 3 | 4 | 5 | 6 | 7 | 8 |
| AST | 14.9 | 14.9 | 11.75 | 11.75 | 74.32 | 74.32 | 68.75 | 68.75 |
| RITA | 2.86 | 5.56 | 2.86 | 5.56 | 2.86 | 5.56 | 2.86 | 5.56 |
| AST + RITA | 23.91 | 28.34 | 34.21 | 36.57 | 62.61 | 54.91 | 56.82 | 49.75 |

Clearly, compared with each singe drug, the combination of Nutlin-3 or RITA with AST significantly enhances the proliferation inhibitory effect on p53 wild-type H460 cells, whereas the combination regimen exhibits almost no enhancing effect on p53 mutant HPAF-II cells. In other words, the aforementioned experiments further confirm that p53 protein has a determinant effect on the efficacy of the drug AST-3424/AST. DNA alkylating agents such as AST-3424/AST exhibit a stronger proliferation inhibitory effect on the cells negative for p53 gene mutation and with normal p53 protein.

In particular, taking single drug AST as an example, to achieve an inhibition rate of 11-12%, the concentration required for p53 wild-type H460 cells is only 1.5 nM, while 60 nM is used for p53 mutant HPAF-II cells. This indicates that DNA alkylating agents such as AST/AST-3424 have a stronger proliferation inhibitory effect on p53 wild-type cancer cells.

The expression levels of AKR1C3 protein in H460 and HPAF-II cells are very close, indicating that the activation degree of AST-3424/AST is similar in the two cells. However, taking single drug AST or single drug AST-3424 as an example, the applicant's previous cancer cell proliferation inhibition test data are as follows:
The IC₅₀ values of AST in H460 and HPAF-II cells are 6.87 nM and 329.1 nM respectively.

The IC₅₀ values of AST-3424 in H460 and HPAF-II cells are 0.47 nM and 107.3 nM respectively.

The above data further verify that DNA alkylating agents such as AST/AST-3424 have a stronger proliferation inhibitory effect on p53 wild-type cancer cells.

According to Examples 18, 19 and 20, p53 protein has a determinant effect on the efficacy of AST-3424/AST. DNA alkylating agents such as AST-3424/AST have a stronger proliferation inhibitory effect on the cells negative for p53 gene mutation and with normal p53 protein. Their action mechanisms include cell cycle G2/M arrest and cell colony inhibition.

To further explain the above experimental phenomena, the effects of single drug or combination drugs on the p53 protein pathway were detected.

### Example 21: Experiment about the effects of AST-3424 ± Nutlin-3 on p53 protein pathway

The first experiment about the effect of AST-3424 alone, Nutlin-3 alone, and their combination on Ser15-p53, Ser20-p53, Total p53, MDM2, and p21 proteins in H460 cells

### Experimental Procedure Overview:

1) H460 cell suspension was added to a 24-well plate, with a cell density of 80000 cells/well and 995 µL of medium per well.
2) The cells were cultured overnight in a 37°C, 5% CO₂ incubator.
3) Compound Treatment

24 hours after cell plating, according to the protocol, the experimental groups for each cell line were as follows:
Monotherapy: 24 hours after cell plating, 5 µL of test compounds at different concentrations was added: 0.5% DMSO, 0.1 nM AST-3424, and 5 µM Nutlin-3. The plate was gently shaken to ensure even mixing, and then placed in a 37°C, 5% CO₂ incubator.
Combination therapy: 24 hours after cell plating, 5 µL of test compounds at different concentrations was added. After pre-treatment with 5 µM Nutlin-3 for 2 hours, 0.1 nM AST-3424 was added. The plate was gently shaken to ensure even mixing, and then placed in a 37°C, 5% CO₂ incubator.

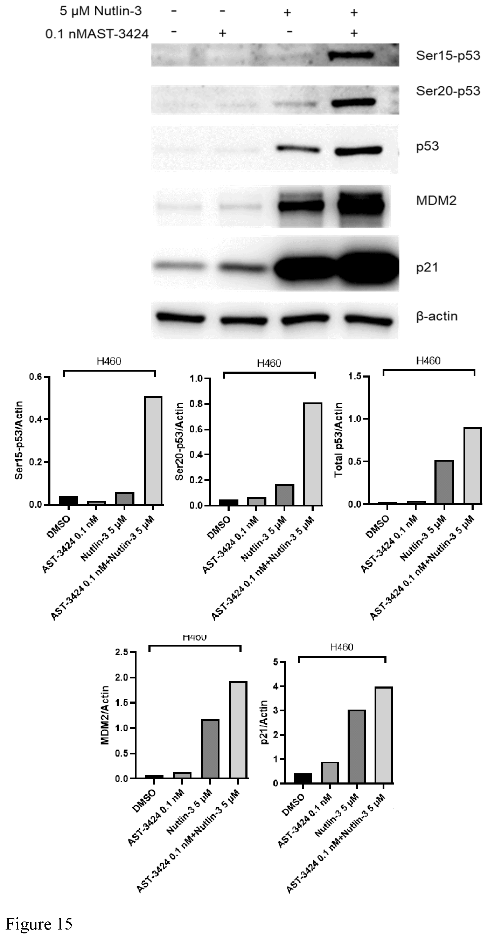

4) Cell protein lysates were collected for WB detection. The results of the WB detection bands are shown in Figure 15.

WB detection refers to the Western Blot (WB) experiment.

Compared with AST-3424 alone or Nutlin-3 alone, the combination of AST-3424 and Nutlin-3 significantly promoted the phosphorylation of p53 (Ser15-p53 and Ser20-p53) and the protein expression of total-p53, and upregulated the expression of p53 downstream genes MDM2 and p21.

To further explore the effects of the combination of AST-3424 at different concentrations and Nutlin-3, a second experiment was conducted.

The second experiment about the effect of AST-3424 alone, Nutlin-3 alone, and their combination on Ser15-p53, Ser20-p53, Total p53, MDM2, and p21 proteins in H460 cells

The remaining operations were similar to the first experiment above, except that the compound treatment groups were as follows:
Monotherapy groups: 1% DMSO, adding 5 µM Nutlin-3 for treatment for 24 hours, adding 0.1 nM AST-3424 for treatment for 24 hours, adding 0.3 nM AST-3424 for treatment for 24 hours, and adding 1 nM AST-3424 for treatment for 24 hours;

### Combination therapy groups:

Adding 0.1 nM AST-3424 for pre-treatment for 2 hours, and then adding 5 µM Nutlin-3 for co-treatment for 22 hours;
Adding 0.3 nM AST-3424 for pre-treatment for 2 hours, and then adding 5 µM Nutlin-3 for co-treatment for 22 hours;
Adding 1 nM AST-3424 for pre-treatment for 2 hours, and then adding 5 µM Nutlin-3 for co-treatment for 22 hours.

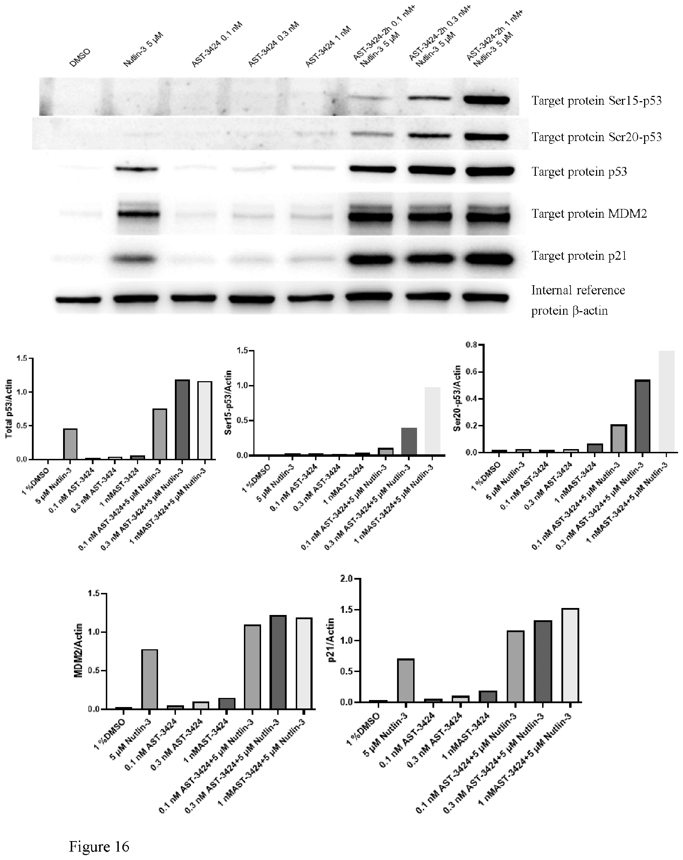

The results are shown in Figure 16.

Experimental results show that Nutlin-3 upregulates the expression levels of p53 and its downstream proteins p21 and MDM2 in H460 cells (p53 wild-type, normal protein expression), which is consistent with the data reported in the literature (Meijer A, Kruyt FA, van der Zee AG, et al. Nutlin-3 preferentially sensitises wild-type p53-expressing cancer cells to DR5-selective TRAIL over rhTRAIL. Br J Cancer. 2013;109(10):2685-2695. doi:10.1038/bjc.2013.636). Compared with each single drug, the combination of AST-3424 at increasing concentrations and Nutlin-3 significantly promotes the phosphorylation of p53 (Ser15-p53 and Ser20-p53) and the protein expression of total-p53, and upregulates the expression of P53 downstream genes MDM2 and p21. Meanwhile, the effect on the expression of the aforementioned proteins is AST-3424 dose-dependent.

To further investigate the effect of administration sequence on the efficacy of the combination of AST-3424 and Nutlin-3, a third experiment was conducted.

### The third experiment about the effect of AST-3424 alone, Nutlin-3 alone, and their combination on Ser15-p53, Ser20-p53, total p53, MDM2, and p21 proteins in H460 cells

The remaining operations were similar to the first experiment above, except that the compound treatment group were as follows:
Monotherapy groups: 0.5% DMSO, adding 5 µM Nutlin-3 for treatment for 24 hours, and adding 0.1 nM AST-3424 for treatment for 24 hours;

### Combination therapy groups:

Adding 0.1 nM AST-3424 for pre-treatment for 2 hours, and then adding 5 µM Nutlin-3 for co-treatment for 22 hours;
Adding 0.1 nM AST-3424 and 5 µM Nutlin-3 for co-treatment for 24 hours;
Adding 5 µM Nutlin-3 for pre-treatment for 2 hours, and then adding 0.1 nM AST-3424 for co-treatment for 22 hours.

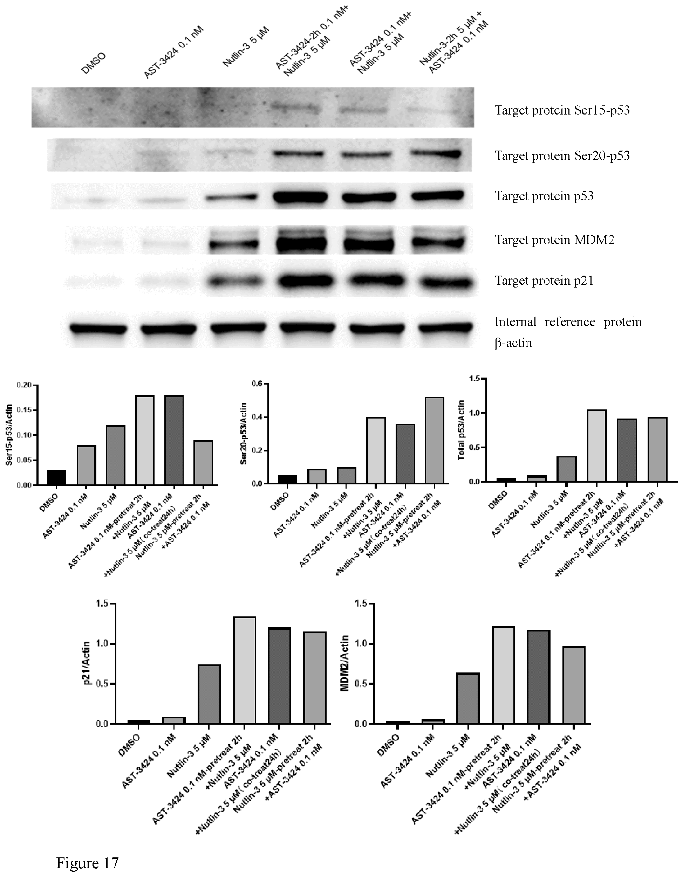

The results are shown in Figure 17.

The experimental results show that compared with each single drug, the combination of AST-3424 and Nutlin-3 significantly promotes the phosphorylation of p53 (Ser15-p53 and Ser20-p53) and the protein expression of total-p53, and upregulates the expression of P53 downstream genes MDM2 and p21. However, there was no significant difference among the effects of different administration sequences on the expression of the aforementioned proteins.

### Example 22: The effects of Nutlin-3 alone, AST-3424 alone, and their combination on RAD51 protein level

### The combination of AST-3424 and Nutlin-3 significantly inhibits the protein expression of RAD51

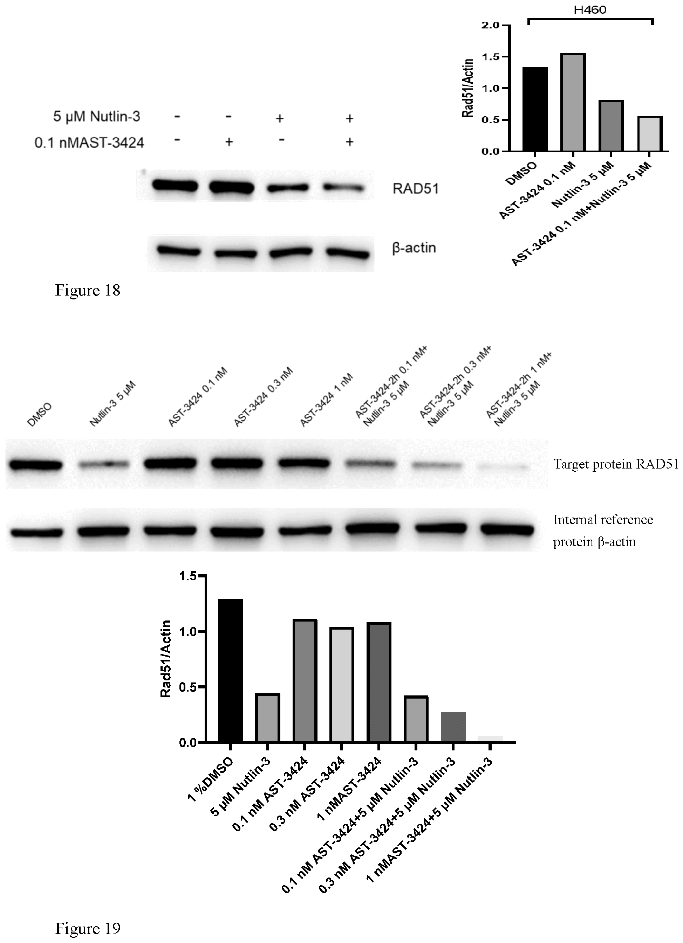

In the first experiment, the second experiment and the third experiment about the effect of the combination of AST-3424 and Nutlin-3 on Ser15-p53, Ser20-p53, Total p53, MDM2, and p21 proteins in H460 cells in Example 21, the applicant also performed WB detection of RAD51 protein. The results are shown in Figures 18, 19 and 20.

The results of all the above three experiments indicate that compared with single drug AST-3424 and single drug Nutlin-3, their combination significantly reduces the RAD51 protein level and the effect is AST-3424 dose-dependent.

### The combination of AST-3424 and Nutlin-3 increases RAD51 protein degradation

### Experimental Procedure Overview:

1) H460 cell suspension was added to a 24-well plate, with a cell density of 100000 cells/well and 995 µL of medium per well.
2) The cells were cultured overnight in a 37°C, 5% CO₂ incubator.
3) Compound Treatment

Monotherapy: 24 hours after cell plating, 5 µL of test compounds at different concentrations was added: 1% DMSO, 0.1 nM AST-3424, and 5 µM Nutlin-3. The plate was gently shaken to ensure even mixing, and then placed in a 37°C, 5% CO₂ incubator. After culturing for another 24 hours, the cells were collected.

Combination therapy: 24 hours after cell plating,
Adding 0.1 nM AST-3424 for pre-treatment for 2 hours, and then adding 5 µM Nutlin-3 for co-treatment for 22 hours;
Adding 1% DMSO and 5 µM MG-132 for co-treatment for 24 hours;
Adding 0.1 nM AST-3424 and 5 µM MG-132 for co-treatment for 24 hours;
Adding 5 µM Nutlin-3 and 5 µM MG-132 for co-treatment for 24 hours;
Adding 0.1 nM AST-3424 for pre-treatment for 2 hours, and then adding 5 µM Nutlin-3 for treatment for 16 hours, and then adding 5 µM MG-132 for co-treatment for 6 hours.

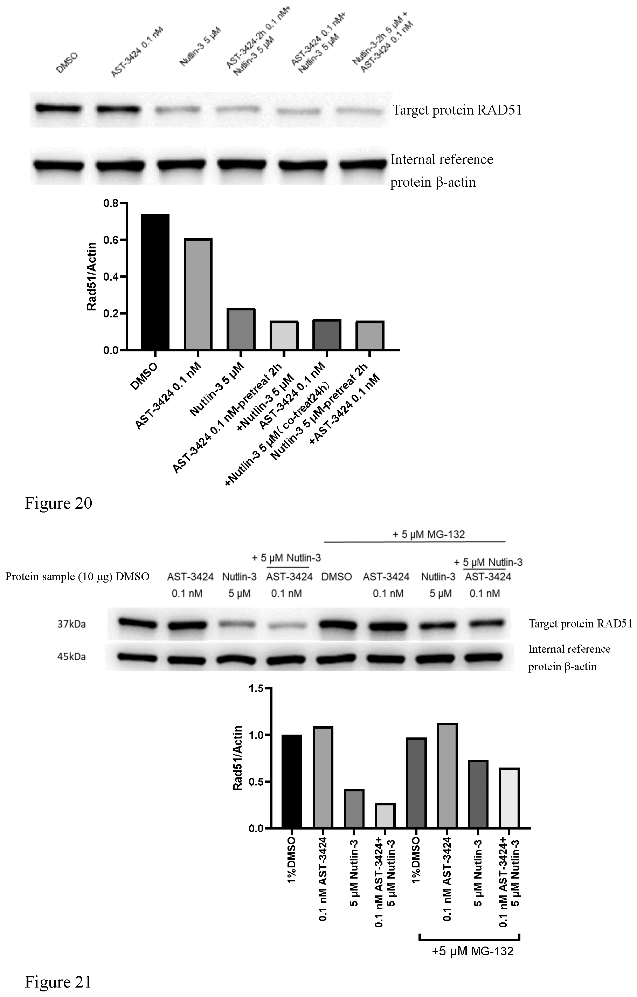

4) Cell protein lysates were collected for WB detection. The detection results are shown in Figure 21.

MG-132 is a proteasome (26Sproteasome) inhibitor that inhibits protein degradation upon addition.

The addition of MG-132 reverses the downregulation of RAD51 protein induced by the combination of AST-3424 and Nutlin-3, thereby suggesting that the reduced level of RAD51 protein caused by the combination is associated with the increased ubiquitination degradation of RAD51 protein.

### The combination of AST-3424 and Nutlin-3 reduces the half-life of RAD51 protein

### Experimental Procedure Overview:

1) H460 cell suspension was added to a 24-well plate, with a cell density of 100000 cells/well and 995 µL of medium per well.
2) The cells were cultured overnight in a 37°C, 5% CO₂ incubator.
3) Compound Treatment

24 hours after cell plating, according to the protocol, the experimental groups for each cell line were as follows:
Monotherapy groups: 1% DMSO treatment group, 5 µM Nutlin-3 treatment group, and 0.1 nM AST-3424 treatment group.

### Combination therapy groups:

1% DMSO + Cycloheximide, 0.5-hour treatment group,
1% DMSO + Cycloheximide, 1-hour treatment group,
1% DMSO + Cycloheximide, 2-hour treatment group,
1% DMSO + Cycloheximide, 4-hour treatment group,
0.1 nM AST-3424 + Cycloheximide, 0.5-hour treatment group,
0.1 nM AST-3424 + Cycloheximide, 1-hour treatment group,
0.1 nM AST-3424 + Cycloheximide, 2-hour treatment group,
0.1 nM AST-3424 + Cycloheximide, 4-hour treatment group,
5 µM Nutlin-3 + Cycloheximide, 0.5-hour treatment group,
5 µM Nutlin-3 + Cycloheximide, 1-hour treatment group,
5 µM Nutlin-3 + Cycloheximide, 2-hour treatment group,
5 µM Nutlin-3 + Cycloheximide, 4-hour treatment group,
0.1 nM AST-3424 + 5 µM Nutlin-3, treatment group,
0.1 nM AST-3424 + 5 µM Nutlin-3 + Cycloheximide, 0.5-hour treatment group,
0.1 nM AST-3424 + 5 µM Nutlin-3 + Cycloheximide, 1-hour treatment group,
0.1 nM AST-3424 + 5 µM Nutlin-3 + Cycloheximide, 2-hour treatment group, and
0.1 nM AST-3424 + 5 µM Nutlin-3 + Cycloheximide, 4-hour treatment group,

Monotherapy: 24 hours after cell plating, 5 µL of test compounds at different concentrations was added. The plate was gently shaken to ensure even mixing, and then placed in a 37°C, 5% CO₂ incubator. After culturing for anoter 24 hours, the cells were collected.

### Combination therapy:

24 hours after cell plating, 5 µL of 1% DMSO was added, and the plate was placed in a 37°C, 5% CO₂ incubator and cultured for another 24 hours. Then, 5 µL of 4 µM Cycloheximide was added, and the plate was placed in a 37°C, 5% CO₂ incubator and respectively cultured for another 0.5, 1, 2,, and 4 hours. Then, the cells were collected.

24 hours after cell plating, 5 µL of 0.1 nM AST-3424 was added, and the plate was placed in a 37°C, 5% CO₂ incubator and cultured for another 24 hours. Then, 5 µL of 4 µM Cycloheximide was added, and the plate was placed in a 37°C, 5% CO₂ incubator and respectively cultured for another 0.5, 1, 2, and 4 hours. Then, the cells were collected.

24 hours after cell plating, 5 µL of 5 µM Nutlin-3 was added, and the plate was placed in a 37°C, 5% CO₂ incubator and cultured for another 24 hours. Then, 5 µL of 4 µM Cycloheximide was added, and the plate was in a 37°C, 5% CO₂ incubator and respectively cultured for another 0.5, 1, 2, and 4 hours. Then, the cells were collected.

24 hours after cell plating, 5 µL of 0.1 nM AST-3424 was added, and the plate was placed in a 37°C, 5% CO₂ incubator and cultured for 2 hours. Then, 5 µL of 5 µM Nutlin-3 was added, and the plate was in a 37°C, 5% CO₂ incubator and cultured for another 24 hours respectively cultured for another the cells were collected.

24 hours after cell plating, 5 µL of 0.1 nM AST-3424 was added, and the plate was placed in a 37°C, 5% CO₂ incubator and cultured for 2 hours. Then, 5 µL of 5 µM Nutlin-3 was added. The plate was gently shaken to ensure even mixing, and then placed in a 37°C, 5% CO₂ incubator and cultured for another 24 hours. Then, 5 µL of 4 µM Cycloheximide was added, and the plate was placed in a 37°C, 5% CO₂ incubator and respectively cultured for another 0.5, 1, 2, and 4 hours. Then, the cells were collected.

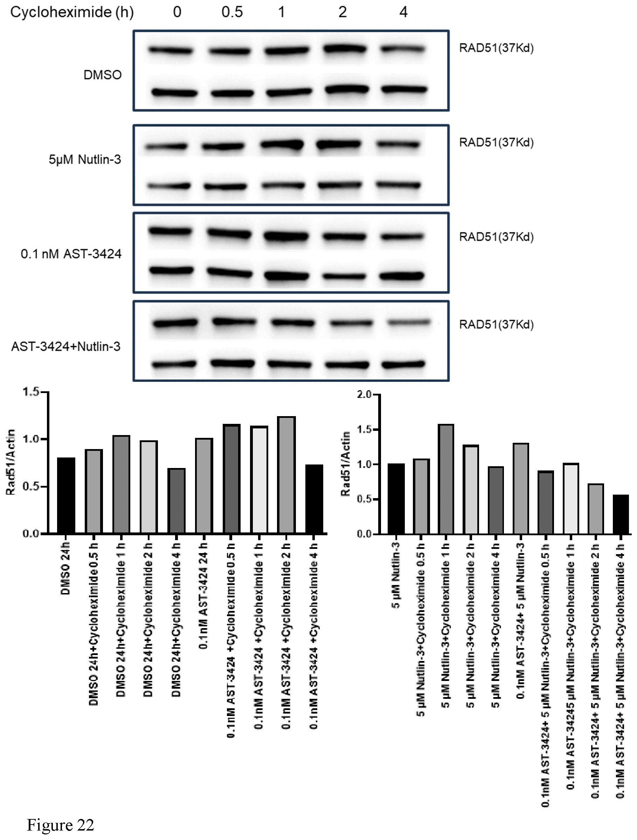

4) Cell protein lysates were collected for WB detection. The detection results are shown in Figure 22.

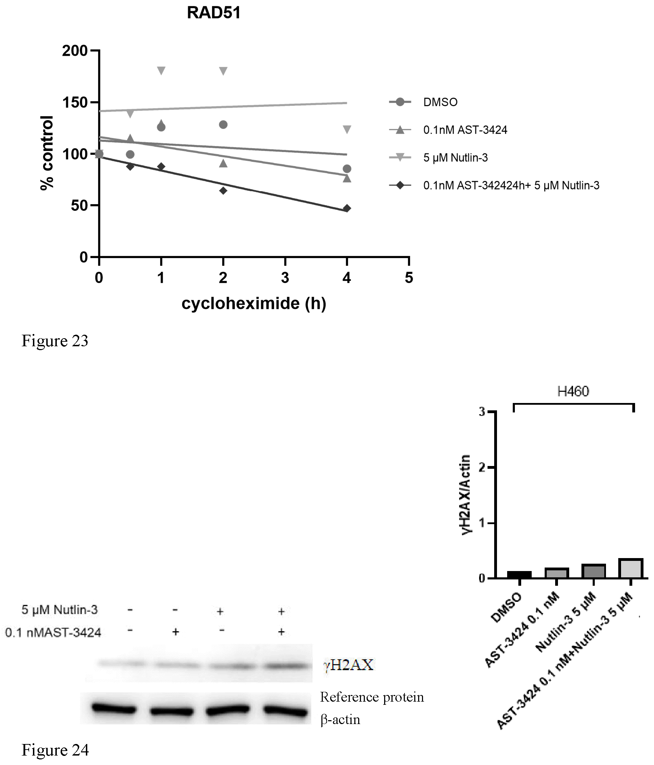

Furthermore, according to the ratio data of the corresponding protein relative to the internal reference protein β-actin in Figure 22, the curves of changes in the relative levels of RAD51 at different time points were calculated and plotted. The results are shown in Figure 23.

Cycloheximide (cycloheximide) is an inhibitor for eukaryotic protein synthesis which terminates protein synthesis upon addition.

After treating cells with AST-3424 alone, Nutlin-3 alone, and their combination for 24 hours, no new protein was synthesized after cycloheximide was added. As shown in Figure 23, the RAD51 protein level is reduced in each group. However, compared with each single drug, the combination of Nutlin-3 and AST-3424 significantly decreases the expression level of RAD51 protein and its half-life, further confirming that the combination therapy could promote RAD51 degradation.

### Example 23: Effects of the combination of AST-3424 and Nutlin-3 on DNA damage process in H460 cells

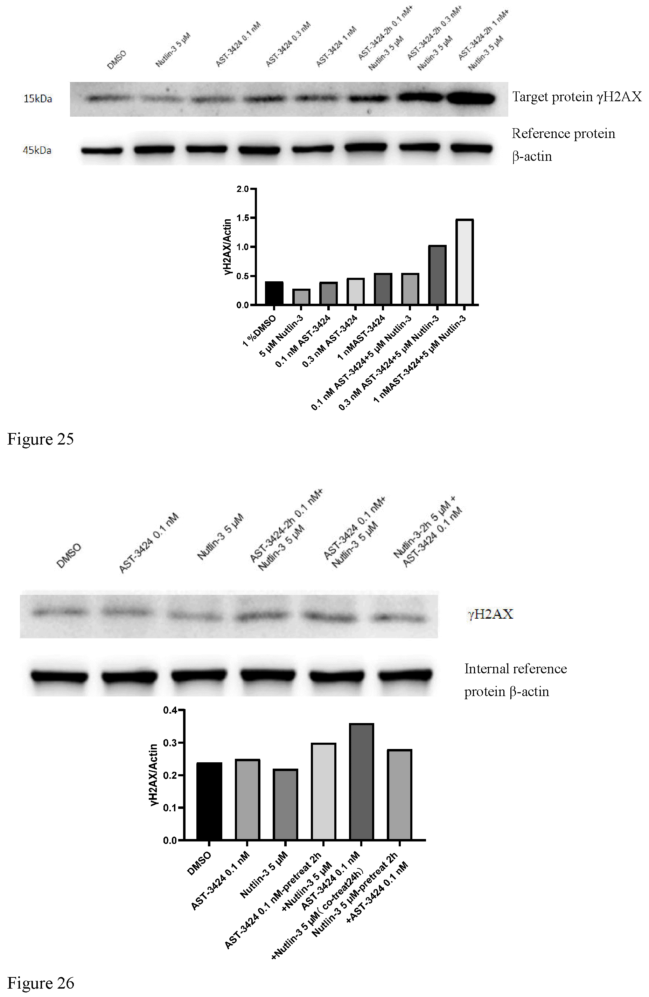

In the first experiment, the second experiment and the third experiment about the effect of the combination of AST-3424 and Nutlin-3 on Ser15-p53, Ser20-p53, Total p53, MDM2, and p21 proteins in H460 cells in Example 21, the applicant also performed WB detection of γH2AX protein. The results are shown in Figures 24, 25 and 26.

The results show that AST-3424 could upregulate the level of γH2AX protein (a biomarker of DNA double-strand damage) in a dose-dependent manner.

Compared with each single drug, the combination of AST-3424 and Nutlin-3 further increases the γH2AX protein level and the effect is AST-3424 dose-dependent. However, the combination of AST-3424 and Nutlin-3 upregulates γH2AX protein more significantly.

Example 24: Effects of 24-hour Nutlin-3/AST treatment on the expression of p53, Rad51, MDM2, and p21 proteins in HPAF-II cells

### Experimental Procedure Overview:

1) HPAF-II cell suspension was added to a 24-well plate, with a cell density of 300000 cells/well and 995 µL of medium per well.
2) The cells were cultured overnight in a 37°C, 5% CO₂ incubator.
3) Compound treatment. 24 hours after cell plating, the cells were grouped and treated according to different cell types.

HPAF-II groups were as follows: 1% DMSO treatment group, 60 nM AST treatment group, 8 µM Nutlin-3 treatment group, and AST 60 nM + Nutlin-38 µM treatment group.

Monotherapy: 24 hours after cell plating, 5 µL of test compounds at different concentrations was added: 1% DMSO, 1.5 nM AST, 1 µM Nutlin-3, 60 nM AST, and 8 µM Nutlin-3. The plate was gently shaken to ensure even mixing, and then placed in a 37°C, 5% CO₂ incubator.

Combination therapy: 24 hours after cell plating, 5 µL of 1 µM Nutlin-3 and 5 µL of 8 µM Nutlin-3 were separately added for pretreatment for 2 hours. Then 5 µL of 1.5 nM AST and 5 µL of 60 nM AST were added correspondingly. The plate was gently shaken to ensure even mixing, and then placed in a 37°C, 5% CO₂ incubator.

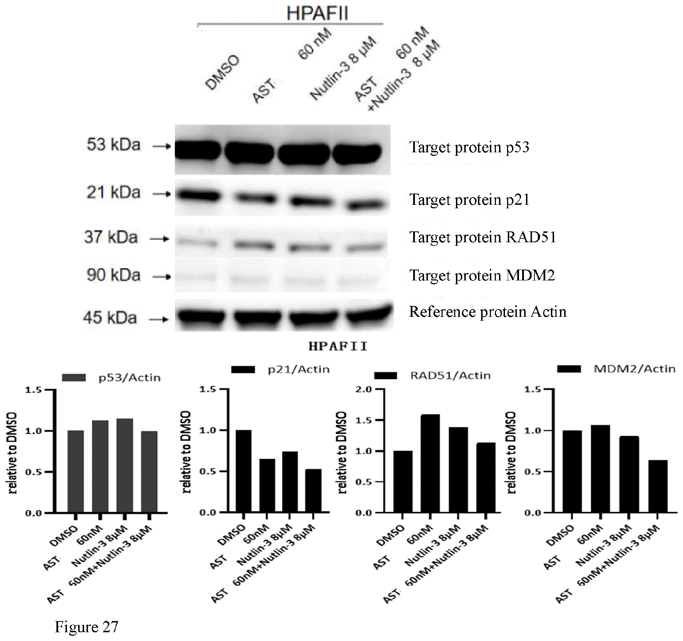

4) After incubating the 24-well plates in a 37°C, 5% CO₂ incubator for 24 hours, cell protein lysates were collected for WB detection. The WB detection results are shown in Figure 27.

Nutlin-3 is an inhibitor of the binding of p53 and MDM2 proteins, activating the function of p53.

The experimental results indicate that 60 nM AST has no effect on the expression of p53, p53 downstream proteins p21 and MDM2 in HPAF-II cells (cells with p53 pathogenic mutation).

The combination of AST and Nutlin-3 has no effect on the expression of p53, p53 downstream proteins p21 and MDM2 in HPAF-II cells (with p53 mutation and abnormal protein expression).

AST alone promotes the expression of RAD51 protein in HPAF-II cells (with p53 mutation and abnormal protein expression), but the combination of AST and Nutlin-3 has no synergistic effect.

Example 25: Effects of the treatment with Nutlin-3/AST-3424 at high concentration on Ser15-p53, Ser20-p53, p53, Rad51, MDM2, p21, γH2AX, and apoptosis-related proteins Caspase 3 and Cleaved Caspase 3 in HPAF-II cells

The aforementioned experiments reveal that for HPAF-II cells with p53 gene mutation, lower concentration of Nutlin-3/AST-3424 has little effect on related proteins. To further verify this, the following experiments were conducted with the drug combination of Nutlin-3/AST-3424 at high concentration: 5 µM Nutlin-3 + 1000 nM AST-3424, to exclude the impact of concentration.

### Experimental Procedure Overview:

1) HPAF-II cell suspension was added to a 24-well plate, with a cell density of 300000 cells/well and 995 µL of medium per well.
2) The cells were cultured overnight in a 37°C, 5% CO₂ incubator.
3) Compound Treatment

24 hours after cell plating, according to the protocol, the experimental groups for each cell line were as follows:
Monotherapy: 24 hours after cell plating, 5 µL of test compounds at different concentrations were added: 0.5% DMSO, 1000 nM AST-3424, and 15 µM Nutlin-3. The plate was gently shaken to ensure even mixing, and then placed in a 37°C, 5% CO₂ incubator.
Combination therapy: 24 hours after cell plating, 5 µL of test compounds at different concentrations was added respectively. After pre-treated with 15 µM Nutlin-3 for 2 hours, 1000 nM AST-3424 was added. The plate was gently shaken to ensure even mixing, and then placed in a 37°C, 5% CO incubator.

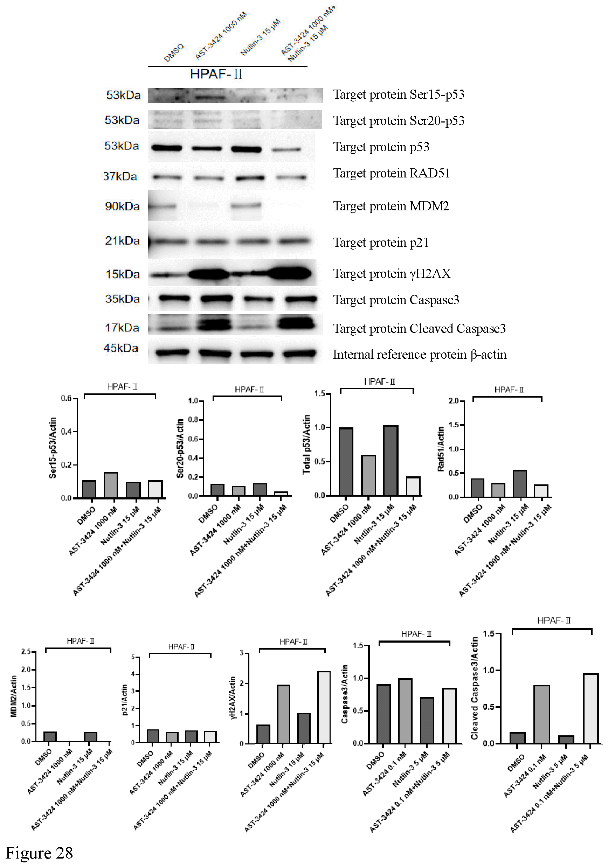

4) Cell protein lysates were collected for WB analysis. The results of the WB detection bands are shown in Figure 28.

Clearly, in the case of high concentration administration, no increased protein expression induced by the relevant combination was observed, or the increase induced by the combination was non-obvious in HPAF-II cells of p53 mutant cell line.

Based on the above Examples 21 to 25, the following conclusions can be drawn:
1. The drugs Nutlin-3/RITA, which upregulate p53 protein expression or activate p53 function, can enhance the *in vitro* toxicity of AST-3424/AST to cancer cells. Moreover, the enhancement is more significant for p53 wild-type cancer cells.
2. The drugs which upregulate p53 protein expression or activate p53 function can enhance the cell apoptosis caused by AST-3424/AST. Especially for p53 wild-type cancer cells, the cell apoptosis caused by the combination is more significant.
3. The drugs Nutlin-3/RITA, which upregulate p53 protein expression or activate p53 function, can significantly increase the DNA double-strand damage caused by AST-3424/AST. For p53 wild-type cancer cells, the DNA double-strand damage caused by the combination is more significant.
4. The drugs Nutlin-3/RITA, which upregulate p53 protein expression or activate p53 function can significantly increase the cell cycle G2/M arrest caused by AST-3424/AST. For p53 wild-type cancer cells, the cell cycle G2/M arrest caused by the combination is more significant.
   In summary, the above enhancements reveal that DNA alkylating agents such as AST/AST-3424 have a stronger proliferation inhibitory effect on p53 wild-type cells.
5. Compared with single drug, the combination of the drugs Nutlin-3/RITAand AST-3424/AST (which upregulate p53 protein expression or activate p53 function) would significantly upregulate p53 phosphorylation, total-p53 expression and the expression of p53 downstream proteins MDM2 and p21, and activate the p53 protein pathway, which in turn downregulates the protein expression of RAD51. For p53 wild-type cancer cells, the above phenomena caused by the combination are more significant.
6. Compared with single drug, the combination of the drugs Nutlin-3/RITA and AST-3424/AST (which upregulate p53 protein expression or activate p53 function) would significantly promote the degradation of RAD51 protein and reduce the half-life of RAD51 protein.

In view of the above, the p53 protein promotes the degradation of homologous recombination repair protein RAD51, leading to downregulation of RAD51 and reduced ability to repair DNA double-strand damage, thereby improving the pharmacological activity of DNA alkylating agents such as AST-3424/AST. In other words, the patients with normal p53 protein and p53 gene are more sensitive to the treatment with DNA alkylating agents such as AST-3424/AST due to the above process and pathway in which p53 protein is involved. The patients may obtain more significant benefit from the treatment (compared with the patients with low p53 protein expression or p53 gene mutation). Such phenomenon has been preliminarily observed in the clinical trial of AST-3424.

Example 26: Effects of compounds on the *in vitro* proliferation of H460 cells and p53 knockout cell lines H460 P53 KO #1, H460 P53 KO #7, and H460 P53 KO #12 under normoxia

The p53 CRISPR/Cas9 KO plasmid (human) and p53 HDR plasmid (human) were purchased from Santa Cruz Company to construct H460 P53 KO cell lines, namely p53 knockout H460 cell lines. Three groups of H460 P53 KO cell lines were constructed and designated as H460 P53 KO # 1, H460 P53 KO # 7, and H460 P53 KO # 12.

The process for constructing p53 gene knockout cells is briefly described as follows:
H460 cells were plated in 6-well plates with 1 × 10⁶ cells/well.
H460 cells were co-transfected with p53 CRISPR/Cas9 KO plasmid (h) (sc-416469) and p53 HDR plasmid (h) (sc-416469-HDR): 125 µL Opti-MEM + 1.25 µg each of the two plasmids + 5 µL P3000; 125 µL Opti-MEM + 15 µL liposome 3000 (increase volume, expected to increase transfection efficiency); they were co-incubated for 15 minutes, and then were added dropwise to the culture medium and mixed well.

After 48 h, 2 µg/mL puromycin was added for screening.

Two days later, the medium was replaced with fresh 1640 medium containing 1 µg/mL puromycin, and the medium was changed every two days.

When the cell colonies grew to a sufficient size, single colonies were picked and cultured.

When the cell number is sufficient, samples were collected for WB identification of the colonies. Colonies with no p53 expression were the p53 knockout H460 cell clones.

For the experimental procedures of this Example, please refer to the Experimental Procedure Overview in the section "Effects of compounds on *in vitro* H460 cell proliferation under normoxia" in Example 18.

### Compound Treatment

Monotherapy: Compounds AST-3424/AST were separately administered in H460 and H460 P53 KO cell lines, respectively.

Combination therapy: Compounds AST-3424/AST were respectively used in combination with AST-3021, and respectively administeredin H460 and H460 P53 KO cell lines.

H460 P53 KO cell lines, i.e. H460 P53 KO # 1, H460 P53 KO # 7, H460 P53 KO # 12 cell lines, were used in experiments, respectively.

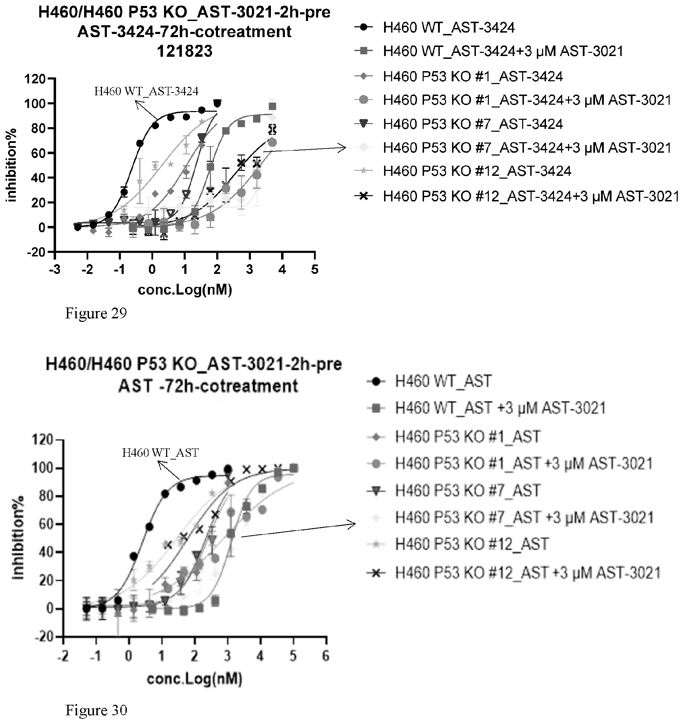

The experimental results of AST-3424 are shown in Table 21. The corresponding *in vitro* proliferation inhibition rate curves of AST-3424 alone and the combination of AST-3424 and AST-3021 in H460 cells and H460 P53 KO cell lines are shown in Figure 29.

The experimental results of AST are shown in Table 22. The corresponding *in vitro* proliferation inhibition rate curves of AST alone and the combination of AST and AST-3021 in H460 cells and H460 P53KO cell lines are shown in Figure 30.

**Table 21: Inhibition rate results of AST-3424 on the in vitro proliferation of H460 WT (H460 wild-type cells) and H460 P53 KO #1, H460 P53 KO #7 and H460 P53 KO #12**

| Cell line/compound combination | IC₅₀(nM) | Ratio, combination therapy/monotherapy | Ratio (monotherapy), knockout/wild |
|---|---|---|---|
| H460 WT_AST-3424 | 0.23 | | |
| H460 WT_AST-3424 + 3 µM AST-3021 | 57.72 | 250.96 | |
| H460 P53 KO # 1_AST-3424 | 11.3 | | 49.13 |
| H460 P53 KO # 1_AST-3424 + 3 µM AST-3021 | 2107 | 186.46 | |
| H460 P53 KO # 7_AST-3424 | 23.1 | | 100.43 |
| H460 P53 KO # 7_AST-3424 + 3 µM AST-3021 | 2709 | 117.27 | |
| H460 P53 KO # 12_AST-3424 | 1.99 | | 8.65 |
| H460 P53 KO # 12_AST-3424 + 3 µM AST-3021 | 344.7 | 173.22 | |

The experimental data shown in Table 21 and Figure 29 indicate that the sensitivity of H460 P53 KO #1 and #12 to AST-3424 is respectively decreased by 49.13-fold and 8.65-fold as compared with H460 wild-type cells, which indicates that p53 deficiency results in reduced sensitivity to the drug AST-3424 and p53 deficiency has no effect on the AKR1C3 protein selectivity of AST-3424. This experiment further proves the experimental results of Examples 18 and 19: p53 protein has a determinant effect on the efficacy of AST-3424, and DNA alkylating agents such as AST-3424 have a stronger proliferation inhibitory effect on the cells negative for p53 gene mutation and with normal p53 protein.

**Table 22: Inhibition rate results of AST on the in vitro proliferation of H460 and H460 P53 KO #1, H460 P53 KO #7, and H460 P53 KO #12.**

| Cell line/compound combination | IC₅₀(nM) | Ratio, combination therapy/monotherapy | Ratio (monotherapy), knockout/wild |
|---|---|---|---|
| H460 WT_AST | 2.55 | | |
| H460 WT_AST + 3 µM AST-3021 | 1283 | 503.14 | |
| H460 P53 KO # 1_AST | 264 | | 103.53 |
| H460 P53 KO # 1_AST + 3 µM AST-3021 | 745.8 | 2.83 | |
| H460 P53 KO # 7_AST | 219.5 | | 86.08 |
| H460 P53 KO # 7_AST + 3 µM AST-3021 | 2166 | 9.87 | |
| H460 P53 KO # 12_AST | 27.96 | | 10.96 |
| H460 P53 KO # 12_AST + 3 µM AST-3021 | 60.53 | 2.16 | |

The experimental data shown in Table 22 and Figure 30 indicate that the sensitivity of H460 P53KO KO #1 and #12 to AST-001 is respectively decreased by 103.53-fold and 10.96-fold as compared with H460 wild-type cells, which indicates that p53 deficiency results in reduced sensitivity to AST. Nevertheless, p53 deficiency causes AST to almost lose the AKR1C3 protein selectivity. This experiment further proves the experimental results of Example 20: p53 protein has a determinant effect on the efficacy of AST, and DNA alkylating agents such as AST has a stronger proliferation inhibitory effect on the cells negative for p53 gene mutation and with normal p53 protein.

### Example 27: Effects of compounds on the in vitro proliferation of H460 cells, H460 P53 KO #1, H460 P53 KO #7, and H460 P53 KO #12 cells under hypoxia conditions

### Experimental Procedure Overview:

1) H460, H460 P53 KO # 1, H460 P53 KO # 7, and H460 P53 KO # 12 cell suspensions were added to two types of 24-well plates (495 µL per well), with a cell density of 1 × 10⁴ cells/well. A 24-well plate fitted with glass insert was used for hypoxia experiments, while a normal plastic 24-well plate was used for normoxia experiments.
2) The cells were cultured overnight in a 37°C, 5% CO₂ incubator.
3) Compound Treatment

### Hypoxia Conditions:

The hypoxia workstation was adjusted to a hypoxia environment (O₂ < 0.01%), and the hypoxia conditions in the workstation were verified using an oxygen indicator. 24 hours after cell plating, the 24-well plate fitted with glass insert was transferred into the hypoxia workstation.

The 24-well plate was placed on a shaker and shaken with the plate lids open for gas exchange for 5 minutes.

5 µL of the compound at 100 x the corresponding concentration was added to each well, with 3 replicate wells for each experimental group.

The plate was gently shaken to ensure even mixing of the compound. The plate lid of the 24-well plate was partially opened, and the plate was incubated in the hypoxia workstation for 3 hours.

### Normoxia Conditions:

24 hours after cell plating, 5 µL of the compound at 100 x the corresponding concentration was added to each well, with 3 replicate wells for each experimental group.

The plate was gently shaken to ensure even mixing of the compouns. The 24-well plate was incubated in a 37°C, 5% CO₂ standard incubator for 3 hours.
4) All 24-well plates were washed twice with complete medium (500 µL per well per wash).
5) 1000 µL of medium was added to each well.
6) The plates were placed in a 37°C, 5% CO ₂ incubator for 72 hours.
7) From each well, 800 µL of medium was discarded. Then 50 µL CTG was added to each well. The plates were shaken for even mixing for 2 min and placed at room temperature for 15 minutes (protected from light).
8) 100 µL of medium was transferred from each well of the 24-well plates to 96-well white plates.
9) The chemiluminescence signal value was read using a multifunctional microplate reader with a reading time of 1000 ms.
10) The IC₅₀ (half maximal inhibitory concentration) values of the compounds were calculated using GraphPad Prism 5 software, with the IC₅₀ values derived from the following non-linear fitting equation.

In this Example, the above experiments were carried out with the specific compound A of Formula (1) and the specific compound B of Formula (2), and the structures of the compounds A and B are as follows:

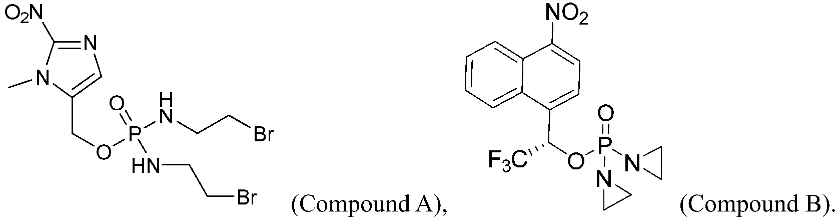

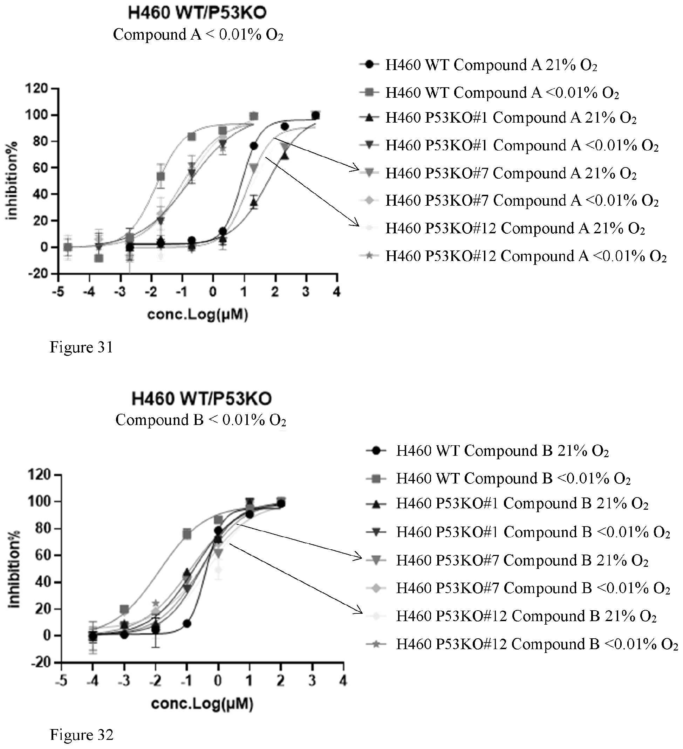

The experimental results of Compound A are shown in Table 23. The corresponding *in vitro* proliferation inhibition rate curves of Compound A in H460 cells and H460 P53 KO cells are shown in Figure 31.

The experimental results of Compound B are shown in Table 24. The corresponding *in vitro* proliferation inhibition rate curves of Compound B in H460 cells and H460 P53 KO cells are shown in Figure 32.

**Table 23: Inhibition rate results of Compound A on the in vitro proliferation of H460 and H460 P53 KO #1 H460 P53 KO #7, and H460 P53 KO #12.**

| Cell line/compound combination | IC₅₀ (uM) | Ratio, 21% O₂/ <0.01% O₂ | Ratio, knockout / wild 21% O₂ | Ratio, knockout / wild<0.01% O₂ |
|---|---|---|---|---|
| H460 WT A 21% O₂ | 8.26 | | | |
| H460 WT A<0.01% O₂ | 0.0144 | 573.61 | | |
| H460 P53KO # 1 A 21% O₂ | 61.52 | | 7.45 | |
| H460 P53KO # 1 A < 0.01% O₂ | 0.1746 | 352.35 | | 12.13 |
| H460 P53KO # 7 A 21% O₂ | 12.9 | | 1.56 | |
| H460 P53KO # 7 A < 0.01% O₂ | 0.1478 | 87.28 | | 10.26 |
| H460 P53KO # 12 A 21% O₂ | 17.37 | | 2.1 | |
| H460 P53KO # 12 A< 0.01% O₂ | 0.0951 | 182.65 | | 6.6 |

The experimental data shown in Table 23 and Figure 31 indicate that under hypoxia conditions, the sensitivity of H460 P53 KO #1, #7, and #12 to Compound A is decreased by 12.13-fold, 10.26-fold and 6.60-fold as compared with H460 P53 WT cells, while these cells still have good hypoxia selectivity. In other words, p53 protein has a determinant effect on the efficacy of Compound A (DNA alkylating agent), which further proves the experimental results of the above Examples that the DNA alkylating agents have a stronger proliferation inhibitory effect on the cells negative for p53 gene mutation and with normal p53 protein.

**Table 24: Inhibition rate results of Compound B on the in vitro proliferation of H460 and H460 P53 KO #1, H460 P53 KO #7 and H460 P53 KO #12.**

| Cell line/compound combination | IC₅₀ (uM) | Ratio, 21% O₂/ <0.01% O₂ | Ratio, 0.01% O₂/ <21% O2 | Ratio, knockout / wild-type 21% O₂ | Ratio, knockout / wild-type <0.01% O₂ |
|---|---|---|---|---|---|
| H460 WT B 21% O₂ | 0.4 | | | | |
| H460 WT B < 0.01% O₂ | 0.013 | 30.77 | | | |
| H460 P53KO # 1 B 21% O₂ | 0.17 | | | 0.43 | |
| H460 P53KO # 1 B < 0.01% O₂ | 0.29 | | 1.71 | | 22.31 |
| H460 P53KO # 7 B 21% O₂ | 0.27 | | | 0.68 | |
| H460 P53KO # 7 B < 0.01% O₂ | 0.36 | | 1.33 | | 27.69 |
| H460 P53KO # 12 B 21% O₂ | 0.47 | | | 1.18 | |
| H460 P53KO # 12 B < 0.01% O₂ | 0.13 | 3.62 | | | 10 |

The experimental data shown in Table 24 and Figure 32 indicate that under hypoxia conditions, the sensitivity of P53 KO #1, #7, and #12 to Compound B is decreased by 22.31-fold, 27.69-fold and 10.00-fold as compared with H460 P53 WT cells, while these cells have reduced hypoxia selectivity. In other words, p53 protein has a determinant effect on the efficacy of Compound B (DNA alkylating agent), which further proves the experimental results of the above Examples that the DNA alkylating agents have a stronger proliferation inhibitory effect on the cells negative for p53 gene mutation and with normal p53 protein.

### Example 28: Effects of presence and absence of Nutlin-3 on Total P53, MDM2, P21, AKR1C3, and Actin proteins in H460 and H460 P53 KO cells

A P53 gene knockout cell line (designated as H460 P53 KO # 8) was constructed using the same method as described in Example 26.

### Experimental Procedure Overview:

1) H460 and H460 P53 KO cell suspensions were added to 24-well plates, with a cell density of 100000 cells/well and 995 µL of medium per well.
2) The cells were cultured overnight in a 37°C, 5% CO₂ incubator.
3) Compound Treatment

24 hours after cell plating, according to the protocol, the experimental groups for each cell line were as follows:
H460 WT non-treatment group, H460 P53 KO # 1 non-treatment group, H460 P53 KO # 7 non-treatment group, H460 P53 KO # 8 non-treatment group, H460 P53 KO # 12 non-treatment group, H460 WT 0.1% DMSO treatment group, H460 WT 5 µM Nutlin-3 treatment group, H460 P53 KO # 1 5 µM Nutlin-3 treatment group, H460 P53 KO # 7 5 µM Nutlin-3 treatment group, H460 P53 KO # 8 5 µM Nutlin-3 treatment group, and H460 P53 KO # 12 5 µM Nutlin-3 treatment group.

Monotherapy: 24 hours after cell plating, 5 µL of test compounds at different concentrations was added. The plates are gently shaken to ensure even mixing, and then placed in a 37°C, 5% CO₂ incubator.

4) After incubating the 24-well plates in the 37°C, 5% CO₂ incubator for 24h, cell protein lysates were collected for WB detection.

### WB detection method:

1) The cell plates were taken out and the medium supernatant was discarded. The plates were gently rinsed with PBS. 30 µL of RIPA protein lysates (RIPA: phosphatase inhibitor = 10:1) was added to each well. The cells were scraped off with a cell scraper, lysed on ice for 30 min, and centrifuged at 14000 rpm at 4°C for 10 min.
2) Protein quantification was performed using the BCA assay. Then, sample loading and gel electrophoresis were carried out: protein samples were separated using 4-12% precast SDS-PAGE gels. The protein samples loaded were as follows (from left to right):
   H460 WT non-treatment group, H460 P53 KO # 1 non-treatment group, H460 P53 KO # 7 non-treatment group, H460 P53 KO # 8 non-treatment group, H460 P53 KO # 12 non-treatment group, H460 WT 0.1% DMSO treatment group, H460 WT 5 µM Nutlin-3 treatment group, H460 P53 KO # 1 5 µM Nutlin-3 treatment group, H460 P53 KO # 7 5 µM Nutlin-3 treatment group, H460 P53 KO # 8 5 µM Nutlin-3 treatment group, and H460 P53 KO # 12 5 µM Nutlin-3 treatment group. The protein loading volume for each sample was 10 µg/12.5 µL. After gel electrophoresis, the proteins were transferred to membranes under the conditions of 100 V for 1 h.
3) Antibody incubation and chemiluminescence detection: After membrane transfer, the membrane was blocked with 5% non-fat milk on a horizontal shaker for 1 h at room temperature. The respective antibodies were added at an antibody ratio of 1: 1000. The membranes were incubated overnight at 4°C in an antibody incubation box. The next day, the membranes were re-warmed at room temperature for 1 h and then washed three times with TBST (10 min per wash). After washing, the membranes were incubated with the corresponding secondary antibodies (1:4000 dilution) according to the type of primary antibodies. The incubation time was 2 h. After the incubation, the membranes were washed three times with TBST (10 min per wash).The ECL luminescence solutions A and B (SuperSignal West Femto Maximum Sensitivity) were mixed and added to the membranes blotting dry. Chemiluminescence detection was performed using a gel imager.

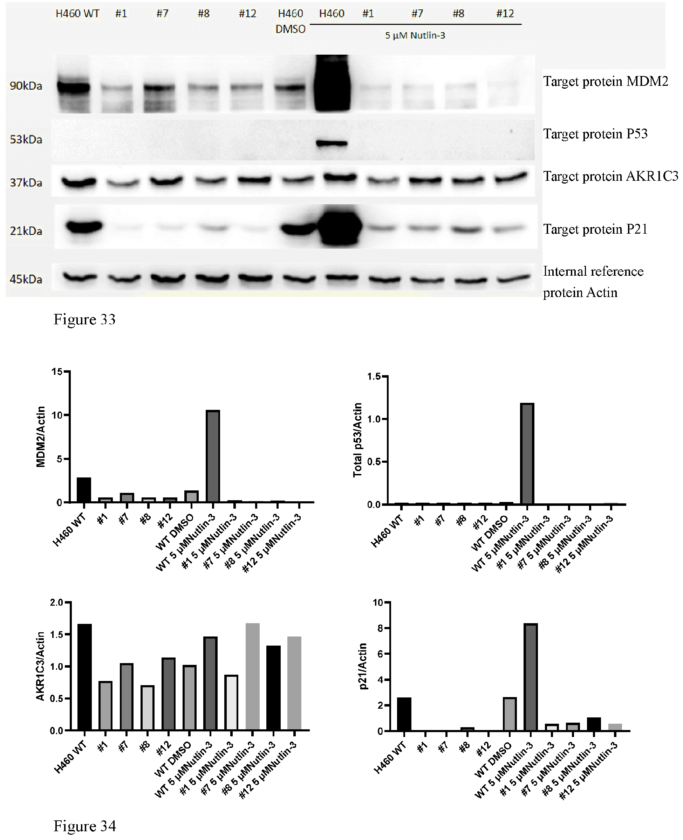

The protein band images of the WB detection results of cell protein lysates are shown in Figure 33. The ratios of the corresponding proteins to the internal reference protein β-actin are shown in Figure 34.

The internal reference protein actin remains unchanged cross all cell lines and various treatments in the above experiments.

The experimental data shown in Figure 33 and Figure 34 indicate that as compared with wild-type H460 cells, the p53 knockout cells have decreased protein expression of AKR1C3 (more significantly in #1) and simultaneously reduced expression of MDM2 and P21. Compared with the H460 wild-type DMSO group, the expressions of MDM2, P53, P21, and AKR1C3 proteins are all upregulated after treating wild-type H460 cells with the positive drug Nutlin-3. However, after treating wild-type H460 and H460 P53 KO cell lines with Nutlin-3, the expressions of MDM2, P53, P21, and AKR1C3 proteins are upregulated in H460 P53 KO cells compared with the H460 wild-type DMSO group, especially in H460 P53 KO # 1 cells. These results suggest that H460 P53 KO may downregulate the protein expression of AKR1C3, which is consistent with the experimental results of IC₅₀ values showing that Compound AST loses AKR1C3 selectivity in H460 P53 KO cells in Table 22 of Example 26 and Figure 30.

### Example 29: Effects of Compound C and AST-3424 on the in vitro proliferation of NCI-H460 cells and NCI-H460 P53KO #1 cells under normoxia conditions

This Example further verifies the effects of the specific Compound C of Formula (9) on the *in vitro* proliferation of NCI-H460 cells and NCI-H460 P53KO #1 cells under normoxia conditions. The structure of Compound C is as follows:

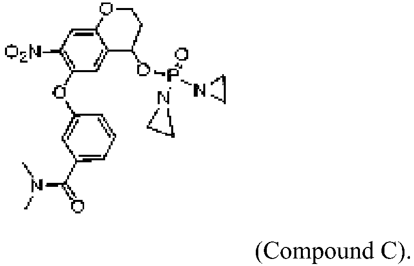

For the synthesis method of compound C, please refer to the corresponding method in WO2021068952A1.

### Experimental Procedure Overview:

1) NCI-H460 and NCI-H460 P53KO # 1 cell suspensions were added to 96-well plate (100 µL per well), with a cell density of 2000 cells/well.
2) The cells were cultured overnight in a 37°C, 5% CO₂ incubator.
3) Compound Treatment

Monotherapy: 24 hours after cell plating, each well was supplemented with 99.5 µL of growth medium. Then, 0.5 µL of test compounds at different concentrations was added. The plate was gently shaken to ensure even mixing, and then placed in a 37°C, 5% CO₂ incubator.

Combination therapy: 24 hours after cell plating, each well was supplemented with 99 µL of growth medium. Then, 0.5 µL of the combination compound was added. The plate was gently shaken to ensure even mixing, and then placed in an incubator for 2 hours. Then, 0.5µL of test compounds at different concentrations was added. The plate was gently shaken to ensure even mixing, and then placed in a 37°C, 5% CO₂ incubator.
4) The cell plate was placed in the incubator for 72 hours.
5) The cell plate to be tested was placed at room temperature and equilibrated for 30 minutes, and 100 µL of medium was discarded from each well.
6) 25 µL of CTG reagent was added to each well, placed at a fast shaker to shake for 2 minutes, and placed at room temperature for 30 minutes (protected from light).
7) The chemiluminescence signal value was read using a multifunctional microplate reader with a reading time of 1000 ms.
8) IC₅₀ (half maximal inhibitory concentration) values were calculated using GraphPad Prism 5 software.

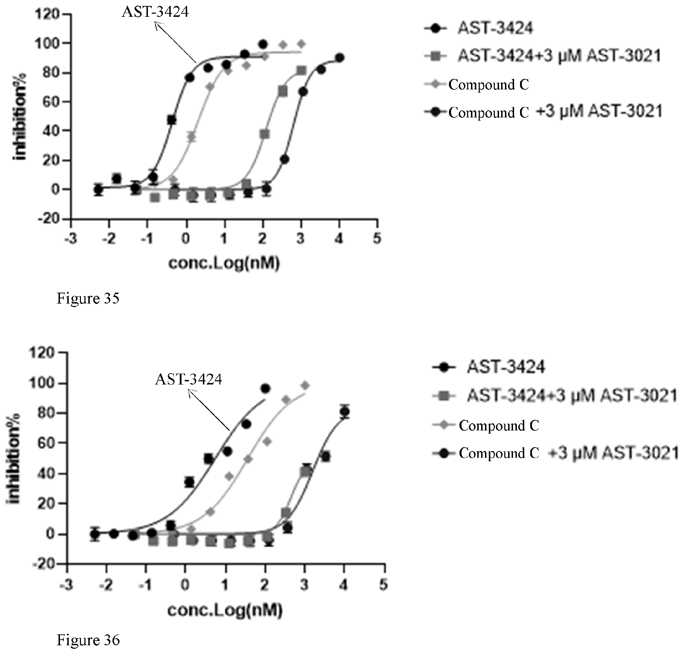

The IC₅₀ results detected in the experiment are shown in Table 25. The *in vitro* proliferation inhibition rate curves of NCI-H460 and NCI-H460 P53 KO #1 cells are shown in Figures 35 and 36.

**Table 25: Inhibition rate results of Compound C and AST-3424 on the in vitro proliferation of NCI-H460 and NCI-H460 P53 KO #1 cells.**

| Cell line | Compounds | IC₅₀ (nM) | Ratio, combination therapy/ monotherapy | Ratio, Compound C/AST-3424 | Ratio, knockout / wild-type |
|---|---|---|---|---|---|
| NCI-H460 | AST-3424 | 0.43 | | | |
| | AST-3424 + 3 µM AST-3021 | 121.00 | 281.40 | | |
| | Compound C | 2.05 | | 4.77 | |
| | Compound C+3 µM AST-3021 | 626.00 | 305.37 | | |
| NCI-H460 P53KO # 1 | AST-3424 | 5.45 | | | 12.67 |
| | AST-3424 + 3 µM AST-3021 | > 1000 | > 183.49 | | |
| | Compound C | 36.08 | | 6.62 | 17.60 |
| | Compound C + 3 µM AST-3021 | 1560.00 | 43.24 | | |

The above experimental data show that:
The IC₅₀ of compound C in NCI-H460 cells is 2.05 nM, which is 4.77 times that of AST-3424. Its AKR1C3 selectivity shows no significant difference as compared with AST-3424.

After p53 knockout, the IC₅₀ of Compound C increases by 17.60-fold, and the IC₅₀ of AST-3424 increases by 12.67-fold, indicating that P53 protein can increase the cytotoxicity of both Compound C and AST-3424.

After p53 knockout, the ratio of the IC₅₀ of the combination of Compound C and the AKR1C3 enzyme inhibitor AST-3021 to the IC₅₀ of Compound C alone decreases from 305.37-fold to 43.24-fold, indicating that the AKR1C3 selectivity of Compound C is significantly reduced upon p53 knockout.

In other words, p53 protein has a determinant effect on the efficacy of Compound C (DNA alkylating agent), which further corroborates the experimental results of the above Examples that the DNA alkylating agents have a stronger proliferation inhibitory effect on the cells negative for p53 gene mutation and with normal p53 protein.

## Claims

1. A treatment method, comprising using a drug containing a DNA alkylating agent prodrug compound, or a salt, ester, solvate, or isotopic isomer thereof, alone or in combination, for treating a cancer or tumor patient, wherein the cancer or tumor patient is selected from the group consisting of the patients that meet one of the following conditions:
cancer patients negative for P53 gene mutation and positive for KRAS gene mutation;
cancer patients negative for P53 gene mutation and positive for BRCA gene mutation;
cancer patients positive for both KRAS gene mutation and BRCA gene mutation; and
cancer patients that meet all three conditions of being negative for P53 gene mutation and positive for both KRAS gene mutation and BRCA gene mutation.

2. A treatment method, comprising using a drug containing a DNA alkylating agent prodrug compound, or a salt, ester, solvate, or isotopic isomer thereof, alone or in combination, for treating a cancer or tumor patient, wherein the DNA alkylating agent prodrug compound is activated by AKR1C3 enzyme, β-D-Glucosidase, Carboxylesterase, Esterase and Caspase-3, Cathepsin B, γ-Glutamyltranspeptidase, β-galactosidase, or hypoxia, and wherein the cancer or tumor patient is selected from the group consisting of the patients that meet one of the following conditions:
cancer patients negative for P53 gene mutation and positive for KRAS gene mutation;
cancer patients negative for P53 gene mutation and positive for BRCA gene mutation;
cancer patients positive for both KRAS gene mutation and BRCA gene mutation; and
cancer patients that meet all three conditions of being negative for P53 gene mutation and positive for both KRAS gene mutation and BRCA gene mutation.

3. A treatment method, comprising using a drug containing a DNA alkylating agent prodrug compound, or a salt, ester, solvate, or isotopic isomer thereof, alone or in combination with other drugs, for treating a cancer patient negative for P53 gene mutation and positive for BRCA gene mutation, wherein the DNA alkylating agent prodrug compound is selected from the group consisting of the DNA alkylating agent prodrug compounds that are activated by AKR1C3 enzyme, β-D-Glucosidase, Carboxylesterase, Esterase and Caspase-3, Cathepsin B, γ-Glutamyltranspeptidase, β-galactosidase, or hypoxia.

4. A treatment method, comprising using a drug containing an anti-cancer prodrug compound that is activated by AKR1C3 enzyme, or a salt, ester, solvate, or isotopic isomer thereof, alone or in combination with other drugs, for treating a cancer patient negative for P53 gene mutation and positive for KRAS gene mutation.

5. A treatment method, comprising using a drug containing an anti-cancer prodrug compound that is activated by AKR1C3 enzyme, or a salt, ester, solvate, or isotopic isomer thereof, alone or in combination with other drugs, for treating a cancer patient positive for both KRAS gene mutation and BRCA gene mutation.

6. The treatment method according to any one of claim 1, 2 or 3, wherein the DNA alkylating agent prodrug compound is selected from the group consisting of the DNA alkylating agent prodrug compounds that are activated by AKR1C3 enzyme, β-D-Glucosidase, Carboxylesterase, Esterase and Caspase-3, Cathepsin B, γ-Glutamyltranspeptidase, β-galactosidase, or hypoxia, preferably activated by AKR1C3 enzyme, β-D-Glucosidase, or hypoxia;
the DNA alkylating agent prodrug compound activated by hypoxia is selected from the group consisting of the compounds of Formulae (1)-(3); the DNA alkylating agent prodrug compound activated by AKR1C3 enzyme is selected from the group consisting of the compounds of Formulae (4)-(11); and the DNA alkylating agent prodrug compound activated by β-D-Glucosidase or β-galactosidase is selected from the group consisting of the compound of Formula (15): 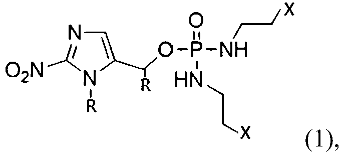
wherein R is each independently selected from the group consisting of H, -CH₃, - CH₂CH₃, and -CF₃, and X is each independently selected from the group consisting of leaving groups Cl, Br, MsO, TsO, and the like; 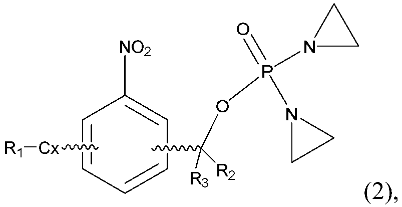
wherein the definitions of R₁, R₂, R₃, and Cx are as described in the claims of the patent application PCT/CN2020/114519 with publication number WO2021120717A1 (corresponding to the Chinese application No. 2020800673113 with publication number CN114466853A); 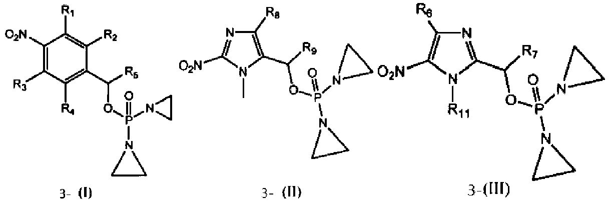 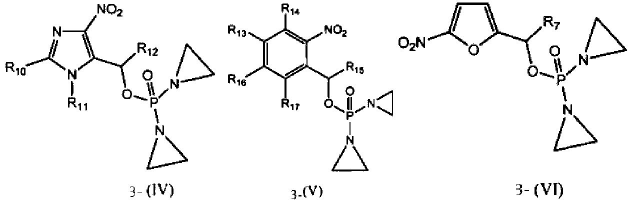
wherein the definitions of R₁, R₂, R₃, R₄, R₅, R₆, R₇, R₈, R₉, R₁₀, R₁₁, R₁₂, R₁₃, R₁₄, R₁₅, R₁₆, and R₁₇ are as described in the claims of the patent application PCT/US2016/039092 with publication number WO2016210175A1 (corresponding to the Chinese application No. 2016800368985 with publication number CN108024974A); 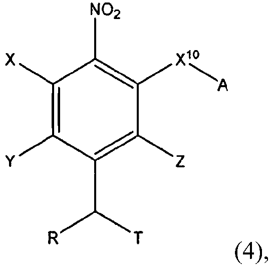
wherein the definitions of X, Y, Z, R, T, A, and X¹⁰ are as described in the claims of the patent application PCT/US2016/021581 with publication number WO2016145092A1 (corresponding to the Chinese application No. 2016800150788 with publication number CN107530556A); 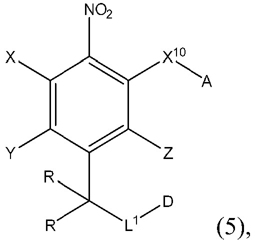
wherein the definitions of X, Y, Z, R, D, L¹, A, and X¹⁰ are as described in the claims of the patent application PCT/US2016/025665 with publication number WO2016161342A3 (corresponding to the Chinese application No. 2016800200132 with publication number CN108136214A); 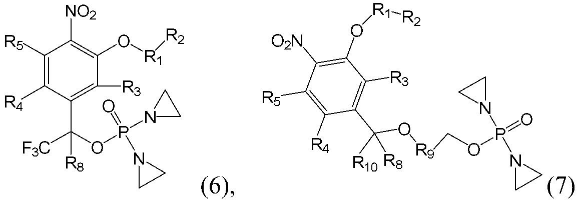
wherein the definitions of R₁, R₂, R₃, R₄, R₅, R₈, R₉, and R₁₀ are as described in the claims of the patent application PCT/CN2020/089692 with publication number WO2020228685A9 (corresponding to the Chinese application No. 2020800358890 with publication number CN113853379A); 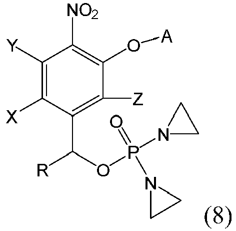
wherein:
A is substituted or unsubstituted C₆-C₁₀ aryl, biaryl or substituted biaryl, 5- to 15-membered heteroaryl, or -N=CR¹R²; wherein the substituent is selected from the group consisting of halogeno, -CN, -NO₂, -O-(CH₂)-O-, -CO₂H and salt thereof, -OR¹⁰⁰, - CO₂R¹⁰⁰, -CONR¹⁰¹R¹⁰², -NR¹⁰¹R¹⁰², -NR¹⁰⁰SO₂R¹⁰⁰, -SO₂R¹⁰⁰, -SO₂NR¹⁰¹R¹⁰², C₁-C₆ alkyl, and C₃-C₁₀ heterocyclyl;
wherein R¹⁰⁰, R¹⁰¹, and R¹⁰² are each independently hydrogen, C₁-C₈ alkyl, or C₆-C₁₂ aryl; or R¹⁰¹ and R¹⁰² together with the nitrogen atom to which they are attached form a 5- to 7-membered heterocycle;
wherein the alkyl group and the aryl group are each substituted by 1-3 halogeno groups or 1-3 C₁-C₆ alkyl groups;
R¹ and R² are each independently phenyl or methyl;
X, Y, and Z are each independently hydrogen or halogeno; and
R is a hydrogen or a C₁-C₆ alkyl or a halogen-substituted alkyl; 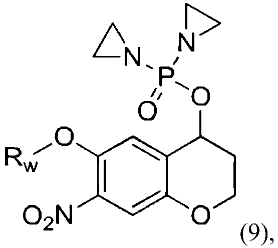
wherein the definition of R_{w} is as described in the claims of the patent application PCT/CN2020/120281 with publication number WO2021068952A1 (corresponding to the Chinese application No. 202080071652.8 with publication number CN114555574A); 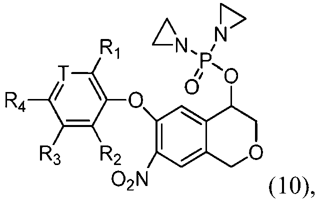
wherein the definitions of R₁, R₂, R₃, R₄, and T are as described in the claims of the patent application PCT/CN2021/118597 with publication number WO2022057838A1; 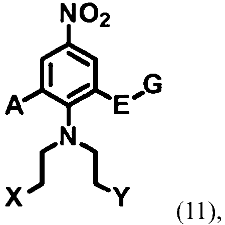
wherein the definitions of A, E, G, X, and Y are as described in the claims of the patent application PCT/NZ2019/050030 with publication number WO2019190331A1 (corresponding to the Chinese application No. 2019800234236 with publication number CN111918864A); 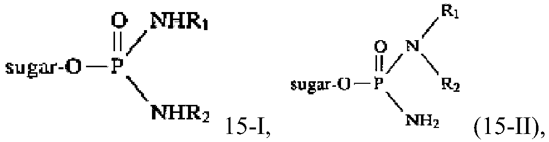
wherein the sugar moiety is attached to a phosphamide mustard residue (15-I) or an ifosfamide mustard residue (15-II), R₁ and R₂ may be the same or different and are selected from the group consisting of hydrogen, C₁-C₄ alkyl, and C₁-C₆ haloalkyl,
and the sugar moiety is an isomer or enantiomer form of any existing monosaccharide, disaccharide or polysaccharide.

7. The treatment method according to claim 4 or 5, wherein the anti-cancer prodrug compound activated by AKR1C3 enzyme comprises a DNA alkylating agent prodrug compound activated by AKR1C3 enzyme, and a non-DNA alkylating agent prodrug compound activated by AKR1C3 enzyme;
the DNA alkylating agent prodrug compound activated by AKR1C3 enzyme is selected from the group consisting of the compounds of Formulae (4)-(11) and (15); and the non-DNA alkylating agent prodrug compound activated by AKR1C3 enzyme is selected from the group consisting of the compounds of Formulae (12)-(14):
the definitions of Formulae (4)-(11) are the same as those defined in claim 6; 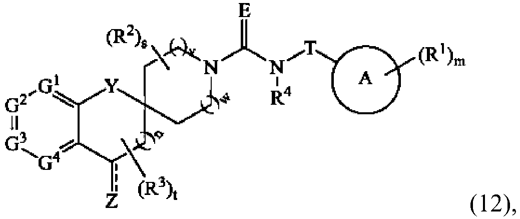
wherein the definitions of R₁, R₂, R₃, R₄, G₁, G₂, G₃, G₄, E, T, Y, Z, m, n, s, t, v, w, and ring A are as described in the claims of the patent application CN202210585771.6 with publication number CN115403579A; 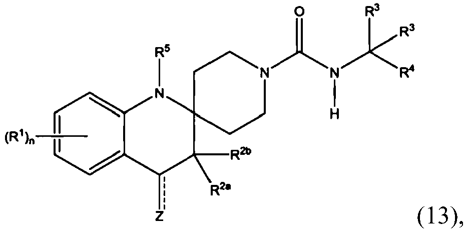
wherein the definitions of R¹, R^{2a}, R^{2b}, R³, R⁴, R⁵, n, and Z are as described in the claims of the patent application PCT/IB2020/057285 with publication number WO2021005586A1 (corresponding to the Chinese application No. CN202080053804.1 with publication number CN114206870A); 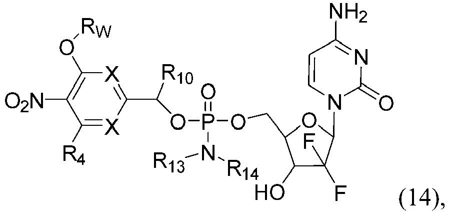
wherein the definitions of R_{w}, X, R₄, R₁₀, R₁₃, and R₁₄ are as described in the claims of the patent application PCT/CN2022/098082 with publication number WO2022258043A1.

8. The treatment method according to claim 6 or 7, wherein the compound of Formula (1) is selected from the group consisting of the compounds of the following Formulae: 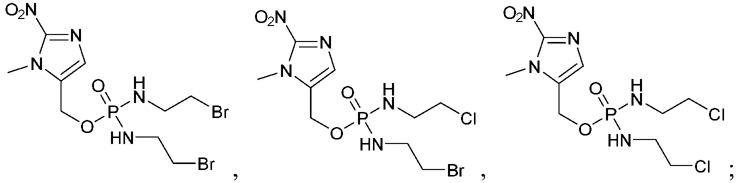 the compound of Formula (2) is selected from the group consisting of the compounds of the following Formulae: 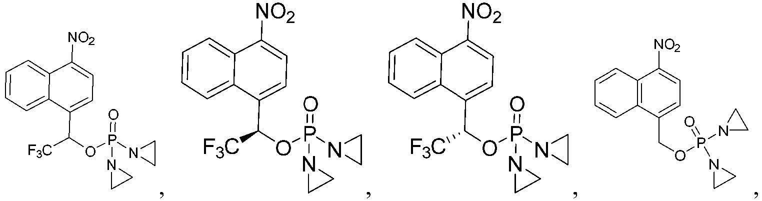 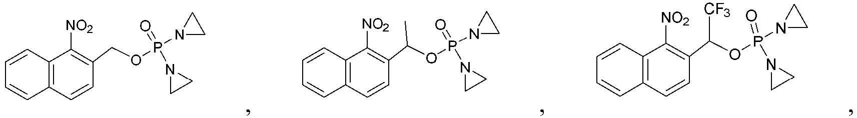 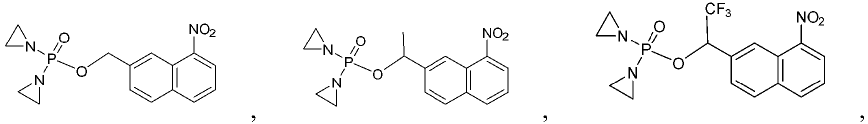 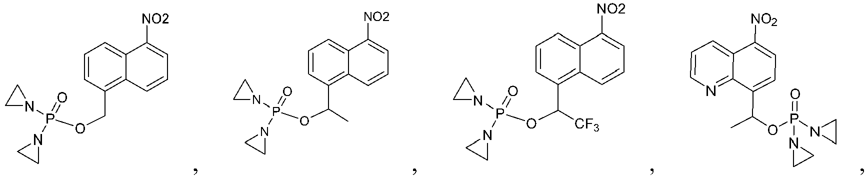 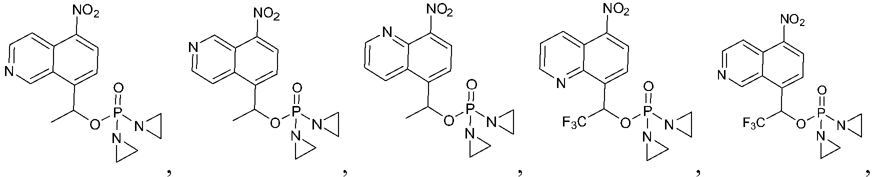 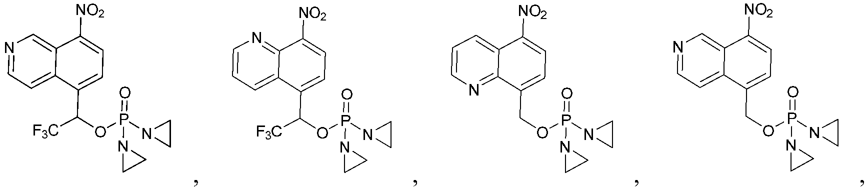 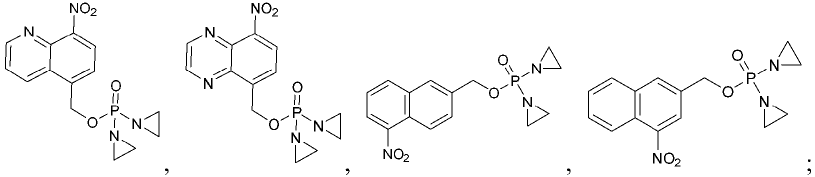 the compound of Formula (3) is selected from the group consisting of the compounds of the following Formulae: 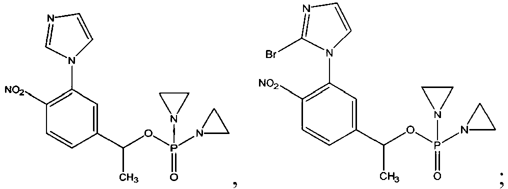 the compound of Formula (4) is selected from the group consisting of the compounds of the following Formulae: 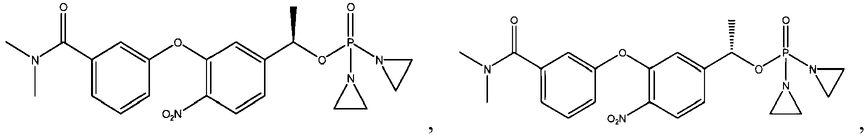 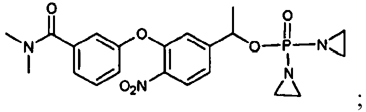 the compound of Formula (5) is selected from the group consisting of the compound of the following Formula: 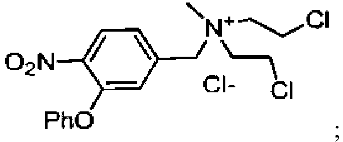 the compounds of Formulae (6) and (7) are selected from the group consisting of the compounds of the following Formulae: 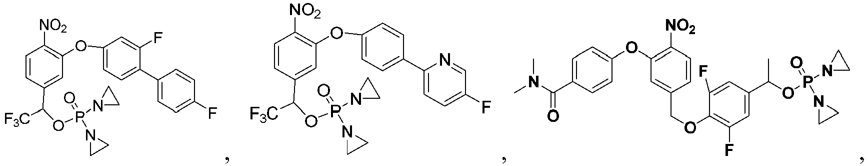 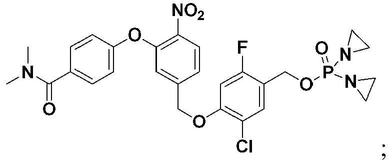 the compound of Formula (8) is selected from the group consisting of the compounds of the following Formulae: 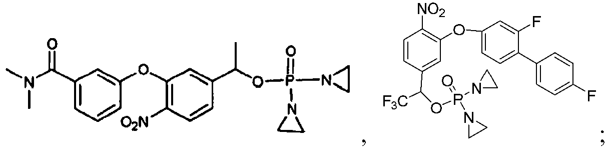 the compound of Formula (9) is selected from the group consisting of the compounds of the following Formulae: 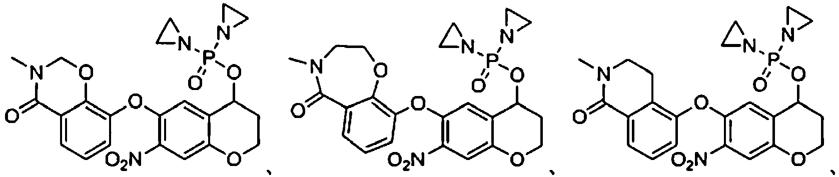 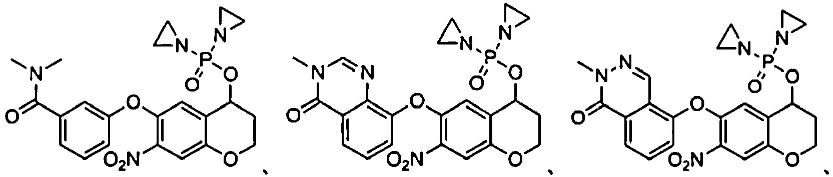 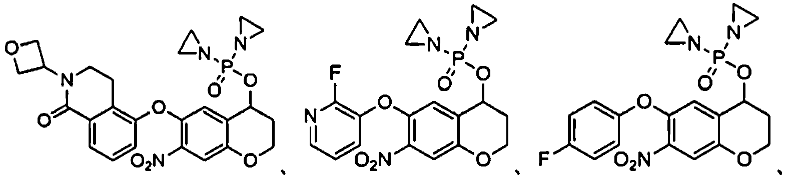 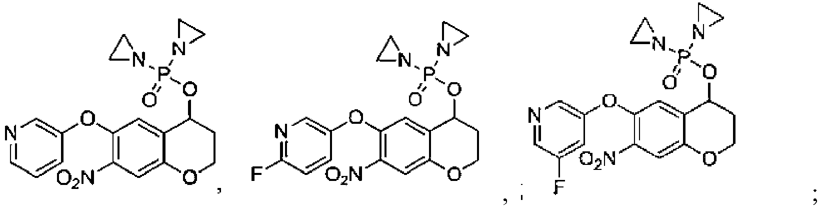 the compound of Formula (10) is selected from the group consisting of the compounds of the following Formulae: 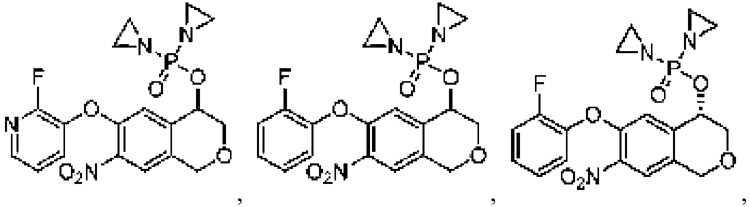 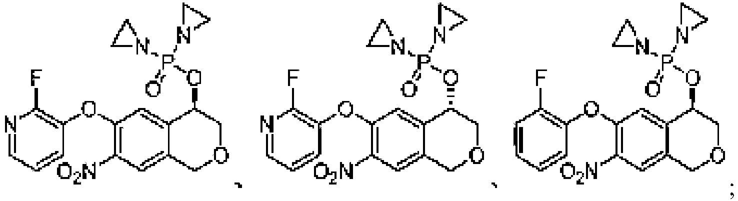 the compound of Formula (11) is selected from the group consisting of the compounds of the following Formulae: 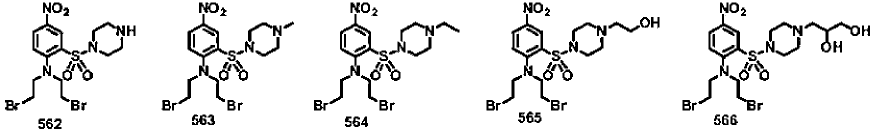 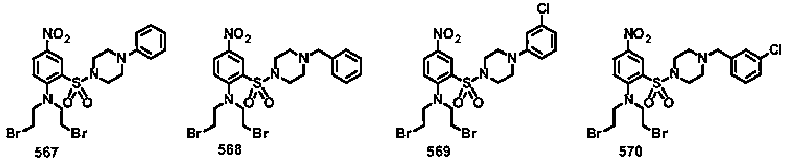 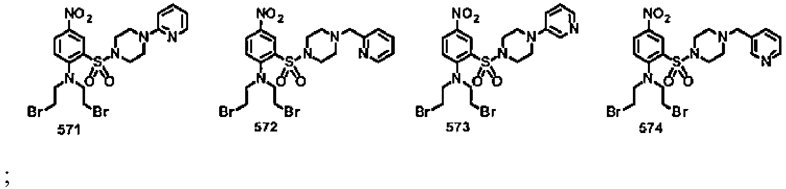 the compound of Formula (12) is selected from the group consisting of the compounds of the following Formulae: 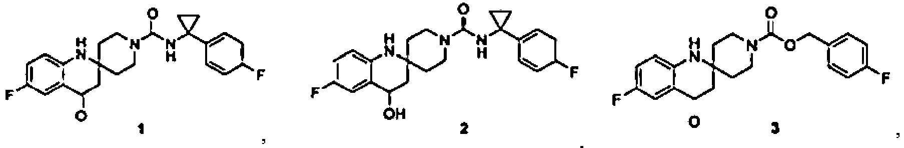 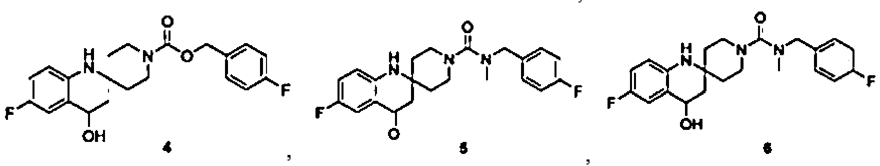 the compound of Formula (13) is selected from the group consisting of the compounds of the following Formulae: 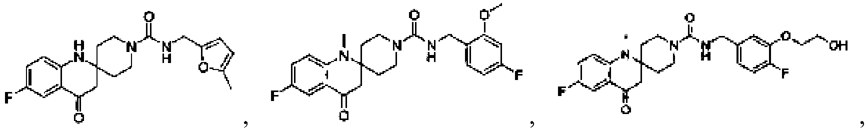 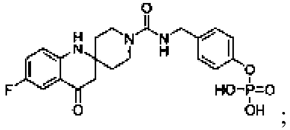 the compound of Formula (14) is selected from the group consisting of the compounds of the following Formulae: 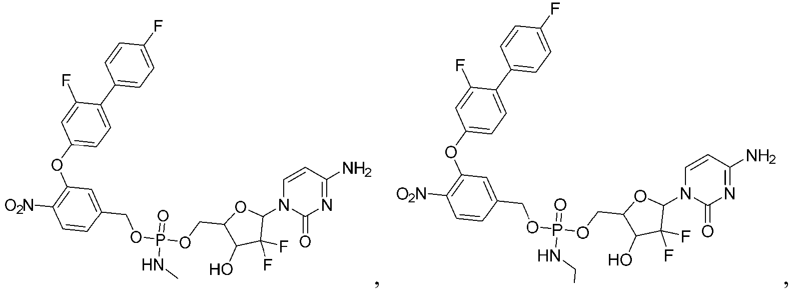 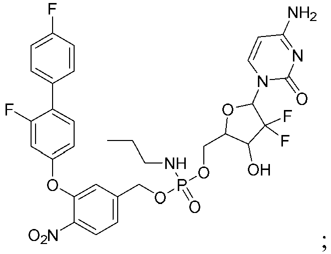 the compound of Formula (15) is selected from the group consisting of the compounds of the following Formulae:

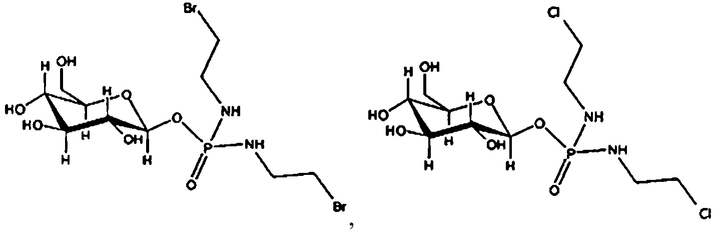

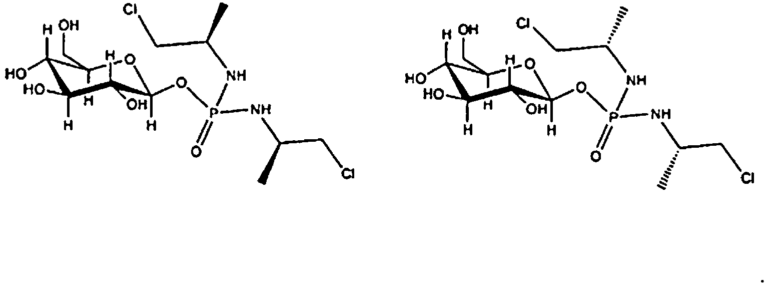

9. The treatment method according to any one of claims 1 to 5, wherein the other drugs used in combination comprise immune checkpoint inhibitors, CDK inhibitors, ATR inhibitors, CHK inhibitors, and WEE1 inhibitors.
